(19) Europäisches Patentamt / European Patent Office / Office européen des brevets

(11) **EP 4 596 720 A2**

(12) **EUROPEAN PATENT APPLICATION**

(43) Date of publication:
**06.08.2025 Bulletin 2025/32**

(21) Application number: 25182512.1

(22) Date of filing: **17.10.2018**

(51) International Patent Classification (IPC):
*C12R 1/39* *(2006.01)*

(52) Cooperative Patent Classification (CPC):
**C07K 14/21; C12N 1/205; C12N 9/80; C12N 9/82;
C12N 15/52; C12N 15/62; C12N 15/625;
C12N 15/67; C12P 21/02; C12Y 305/01001;**
C07K 2319/02; C12R 2001/39

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.10.2017 US 201762578304 P**

(62) Document number(s) of the earlier application(s) in
accordance with Art. 76 EPC:
**18870110.6 / 3 700 915**

(71) Applicant: **Pelican Technology Holdings, Inc.
San Diego, CA 92121 (US)**

(72) Inventor: **Coleman, Russell J.
San Diego 92121 (US)**

(74) Representative: **Wichmann, Hendrik
Wuesthoff & Wuesthoff
Patentanwälte und Rechtsanwalt PartG mbB
Schweigerstraße 2
81541 München (DE)**

Remarks:
• The complete document including Reference Table(s) and the Sequence Listing(s) can be downloaded from the EPO website
• This application was filed on 12-06-2025 as a divisional application to the application mentioned under INID code 62.

(54) **BACTERIAL LEADER SEQUENCES FOR PERIPLASMIC PROTEIN EXPRESSION**

(57) Provided herein are polypeptides comprising a bacterial leader peptide for periplasmic protein expression, and protein expression methods using such a polypeptide.

EP 4 596 720 A2

**Description**

**CROSS-REFERENCE TO RELATED APPLICATIONS**

This application claims benefit of U.S. Provisional Application No. 62/578,304, filed October 27, 2017, which is incorporated herein by reference.

**SEQUENCE LISTING**

This application contains an electronic Sequence Listing which has been submitted in XML file format with this application, the entire content of which is incorporated by reference herein in its entirety. The Sequence Listing XML file submitted with this application is entitled "EPA-166934 seq list.xml", was created on June 3, 2025, and is 16,904 bytes in size.

**BACKGROUND OF THE INVENTION**

More than 150 recombinantly produced proteins and polypeptides have been approved by the U.S. Food and Drug Administration (FDA) for use as biotechnology drugs and vaccines, with another 370 in clinical trials. Unlike small molecule therapeutics that are produced through chemical synthesis, proteins and polypeptides are most efficiently produced in living cells. However, current methods of production of recombinant proteins in bacteria often produce improperly folded, aggregated or inactive proteins, and many types of proteins require secondary modifications that are inefficiently achieved using known methods.

One primary problem with known methods lies in the formation of inclusion bodies made of aggregated proteins in the cytoplasm, which can occur when an excess amount of protein accumulates in the cell. Another problem in recombinant protein production is establishing the proper secondary and tertiary conformation for the expressed proteins. One barrier is that bacterial cytoplasm actively resists disulfide bond formation, which often underlies proper protein folding (Derman et al., 1993, Science, 1744-7). As a result, many recombinant proteins, particularly those of eukaryotic origin, are improperly folded and inactive when produced in bacteria.

Numerous attempts have been developed to increase production of properly folded, soluble, and/or active proteins in recombinant systems. For example, investigators have changed fermentation conditions, varied promoter strength, or used overexpressed chaperone proteins, which often help prevent the formation of inclusion bodies.

An alternative approach to increase the harvest of properly folded, soluble, and/or active proteins is to secrete the protein from the intracellular environment. The most common form of secretion of polypeptides with a signal sequence involves the Sec system. The Sec system is responsible for export of proteins with a Sec system N-terminal secretion leader across the cytoplasmic membrane.

Strategies have been developed to excrete proteins from the cell into the supernatant. Other strategies for increased expression are directed to targeting the protein to the periplasm. Some investigations focus on non-Sec type secretion. However, the majority of research has focused on the secretion of exogenous proteins with a Sec-type secretion system. A number of secretion signals have been described for use in expressing recombinant polypeptides or proteins.

Strategies that rely on signal sequences for targeting proteins out of the cytoplasm often produce improperly processed protein. This is particularly true for amino-terminal secretion signals such as those that lead to secretion through the Sec system. Proteins that are processed through this system often either retain a portion of the secretion signal, require a linking element which is often improperly cleaved, or are truncated at the terminus.

As is apparent from the above-described art, many strategies have been developed to target proteins to the periplasm of a host cell. However, known strategies have not resulted in consistently high yield of properly processed, active recombinant protein, which are often purified for therapeutic use. One major limitation in previous strategies has been the expression of proteins with poor secretion signal sequences in inadequate cell systems.

There remains a need for improved large-scale expression systems capable of secreting and properly processing recombinant polypeptides to produce them in properly processed form.

**SUMMARY OF THE INVENTION**

Provided herein are polypeptides having a first portion and a second portion, wherein the first portion has an amino acid sequence homologous to one or more of SEQ ID NOS: 1-3 and the second portion has an amino acid sequence of a protein or polypeptide of interest, and wherein the first portion and the second portion are operably linked. In some embodiments, the first portion of the polypeptide is not native to the protein or polypeptide of interest. In some embodiments, the protein of interest is selected from an antibody or antibody derivative, an enzyme, a cytokine, a chemokine, a growth factor, and a vaccine antigen. In some embodiments, the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody. In some embodiments, the polypeptide further comprises a linker. In some embodiments, the polypeptide further comprises a cleavage domain. In some embodiments, the first portion is a leader peptide directing expression of the protein of interest to the periplasm of a prokaryotic host cell. In some embodiments, the host cell is selected from a *Pseudomonad* cell or an *E. coli* cell.

Also provided herein are polypeptides comprising a leader peptide and a protein or polypeptide of interest, wherein the leader peptide has an amino acid sequence selected from one or more of SEQ ID NOS: 1-3. In some embodiments, the leader peptide is not native to the protein or polypeptide of interest. In some embodiments, the protein or polypeptide of interest is selected from an antibody or antibody derivative, an enzyme, a cytokine, a chemokine, a growth factor, and a vaccine antigen. In some embodiments, the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody. In some embodiments, the polypeptide further comprises a linker. In some embodiments, the polypeptide further comprises a cleavage domain. In some embodiments, the first portion is a leader peptide directing expression of the protein or polypeptide of interest to the periplasm of a prokaryotic host cell. In some embodiments, the host cell is selected from a *Pseudomonad* cell or an *E. coli* cell.

Further provided herein are polynucleotides encoding a polypeptide having a first portion and a second portion, wherein the first portion is encoded by a nucleic acid sequence selected from one or more of SEQ ID NOS: 4-6 and the second portion is encoded by a nucleic acid sequence of a protein or polypeptide of interest, and wherein the first portion and the second portion are operably linked. In some embodiments, the first portion of the polypeptide is not native to the protein or polypeptide of interest. In some embodiments, the protein or polypeptide of interest is selected from an antibody or antibody derivative, an enzyme, a cytokine, a chemokine, a growth factor, and a vaccine antigen. In some embodiments, the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody. In some embodiments, the polynucleotide further comprises a nucleic acid encoding linker. In some embodiments, the polynucleotide further comprises further comprising a nucleic acid encoding a cleavage domain. In some embodiments, the first portion is a leader peptide directing expression of the protein or polypeptide of interest to the periplasm of a prokaryotic host cell. In some embodiments, the host cell is selected from a *Pseudomonad* cell or an *E. coli* cell.

Also provided herein are vectors for protein expression comprising a nucleic acid encoding a leader peptide, wherein the nucleic acid has a nucleic acid sequence selected from one or more of SEQ ID NOS: 4-6. In some embodiments, the vector further comprises a linker. In some embodiments, the vector further comprises a cleavage domain. In some embodiments, the leader peptide directs expression of a protein or polypeptide of interest to the periplasm of a prokaryotic host cell. In some embodiments, the host cell is selected from a *Pseudomonad* cell or an *E. coli* cell.

Also provided herein are methods of producing a protein or polypeptide of interest in a prokaryotic cell culture. In some embodiments, the method comprises: (a) culturing the prokaryotic cells comprising a nucleic acid encoding the protein or polypeptide of interest and a leader peptide in a cell culture growth medium; and (b) isolating the protein or polypeptide of interest from the periplasm of the prokaryotic cells, wherein the leader peptide comprises an amino acid sequence selected from one or more of SEQ ID NOS: 1-3. In some embodiments, the protein or polypeptide of interest is selected from an antibody or antibody derivative, an enzyme, a cytokine, a chemokine, a growth factor, and a vaccine antigen. In some embodiments, the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody. In some embodiments, the nucleic acid encodes a linker. In some embodiments, the nucleic acid encodes a cleavage domain. In some embodiments, the prokaryotic cells are selected from a *Pseudomonad* cell or an *E. coli* cell. In some embodiments, expression of the protein or polypeptide of interest is induced with addition of IPTG to the culture media. In some embodiments, the prokaryotic cells are cultured at a pH of about 5.0 to about 8.0. In some embodiments, the prokaryotic cells are cultured at a temperature of about 22°C to about 33°C.

In some embodiments, methods herein comprise a method of producing a protein or polypeptide of interest in a prokaryotic cell culture, the method comprising: (a) culturing the prokaryotic cells comprising a nucleic acid encoding the protein or polypeptide of interest and a leader peptide in a cell culture growth medium; and (b) isolating the protein or polypeptide of interest from the periplasm of the prokaryotic cells, wherein the leader peptide is encoded by a nucleic acid sequence selected from one or more of SEQ ID NOS: 4-6. In some embodiments, the protein or polypeptide of interest is selected from an antibody or antibody derivative, an enzyme, a cytokine, a chemokine, a growth factor, and a vaccine antigen. In some embodiments, the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody. In some embodiments, the nucleic acid encodes a linker. In some embodiments, the nucleic acid encodes a cleavage domain. In some embodiments, the prokaryotic cells are selected from a *Pseudomonad* cell or an *E. coli* cell. In some embodiments, expression of the protein or polypeptide of interest is induced with addition of IPTG to the culture media. In some embodiments, the prokaryotic cells are cultured at a pH of about 5.0 to about 8.0. In some embodiments, the prokaryotic cells are cultured at a temperature of about 22°C to about 33°C.

In some embodiments, methods herein comprise a method of expressing a protein or polypeptide of interest in the periplasm of a prokaryotic cell, the method comprising culturing the prokarotic cell comprising a nucleic acid encoding the protein or polypeptide of interest and a leader peptide in a cell culture growth medium, wherein the leader peptide comprises an amino acid sequence selected from one or more of SEQ ID NOS: 1-3. In some embodiments, the protein or polypeptide of interest is selected from an antibody or antibody derivative, an enzyme, a cytokine, a chemokine, a growth factor, and a vaccine antigen. In some embodiments, the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody. In some embodiments, the nucleic acid encodes a linker. In some embodiments, the nucleic acid encodes a cleavage domain. In some embodiments, the prokaryotic cells are selected from a *Pseudomonad* cell or an *E. coli* cell. In some embodiments, expression of the protein or polypeptide of interest is induced with addition of IPTG to the culture media. In some embodiments, the prokaryotic cells are cultured at a pH of about 5.0 to about 8.0. In some embodiments, the prokaryotic cells are cultured at a temperature of about 22°C to about 33°C.

In some embodiments, methods herein comprise a method of expressing a protein or polypeptide of interest in the periplasm of a prokaryotic cell, the method comprising culturing the prokaryotic cells comprising a nucleic acid encoding the protein or polypeptide of interest and a leader peptide in a cell culture growth medium, wherein the leader peptide comprises is encoded by a nucleic acid sequence selected from one or more of SEQ ID NOS: 4-6. In some embodiments, the protein or polypeptide of interest is selected from an antibody or antibody derivative, an enzyme, a cytokine, a chemokine, a growth factor, and a vaccine antigen. In some embodiments, the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody. In some embodiments, the nucleic acid encodes a linker. In some embodiments, the nucleic acid encodes a cleavage domain. In some embodiments, the prokaryotic cells are selected from a *Pseudomonad* cell or an *E. coli* cell. In some embodiments, expression of the protein or polypeptide of interest is induced with addition of IPTG to the culture media. In some embodiments, the prokaryotic cells are cultured at a pH of about 5.0 to about 8.0. In some embodiments, the prokaryotic cells are cultured at a temperature of about 22°C to about 33°C.

## INCORPORATION BY REFERENCE

All publications, patents, and patent applications mentioned in this specification are herein incorporated by reference to the same extent as if each individual publication, patent, or patent application was specifically and individually indicated to be incorporated by reference.

## BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

　　**FIG. 1** shows SDS-CGE Gel-like images for TrxA expression.
　　**FIG. 2** shows SDS-CGE Gel-like images for Gal2 expression.
　　**FIG. 3** shows SDS-CGE Gel-like images for mrPA expression.
　　**FIG. 4. Crisantaspase Example Sequences.** An exemplary nucleic acid sequence encoding crisantaspase (SEQ ID NO: 8) is shown with the corresponding amino acid sequence (SEQ ID NO: 7). FIG. 4 also discloses a full-length nucleotide sequence including cloning sites as SEQ ID NO: 20.

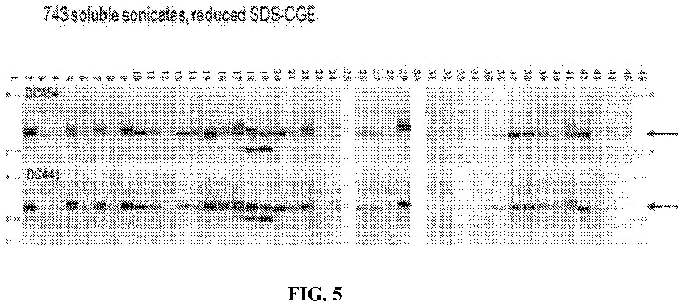

　　**FIG. 5. SDS-CGE Gel-like Images - Crisantaspase Expression Plasmid Screen.** Crisantaspase small scale growth whole broth sonicate soluble samples from DC454 (upper panel) and DC441 (lower panel) were analyzed by reduced SDS-CGE. The lane at the far left shows molecular weight marker ladder 1 (upper panel MW ladder 48 KD, 29 KD; lower panel MW ladder 48 KD, 29 KD, 21 KD), and the lane at the far right shows the same ladders. From left to right (lanes 1 to 46), beginning immediately to the right of ladder 1 are lanes showing the expression patterns observed when the following secretion leader peptides were fused to the N-terminus of crisantaspase protein (high RBS except as otherwise indicated): no leader; DsbD; Leader A; DsbA; DsbA-Medium RBS; Azu; Azu-Medium RBS; Lao; Ibp-S31A; TolB; DC432 null (wild type host strain carrying vector only plasmid); Tpr; Ttg2C; FlgI; CupC2; CupB2; Pbp; PbpA20V; DsbC; Leader B; Leader C; DC432 null; Leader D; Leader E; Leader F; Leader G; Leader H; PorE; Leader I; Leader J; Leader K; Leader L; DC432 null; Leader M; Leader N; Leader O; 5193; Leader P; Leader Q; Leader R; 8484; Leader S; Leader T; DC432 null. The arrows to the right of the gel image indicate migration of the crisantaspase target protein (35 kDa).

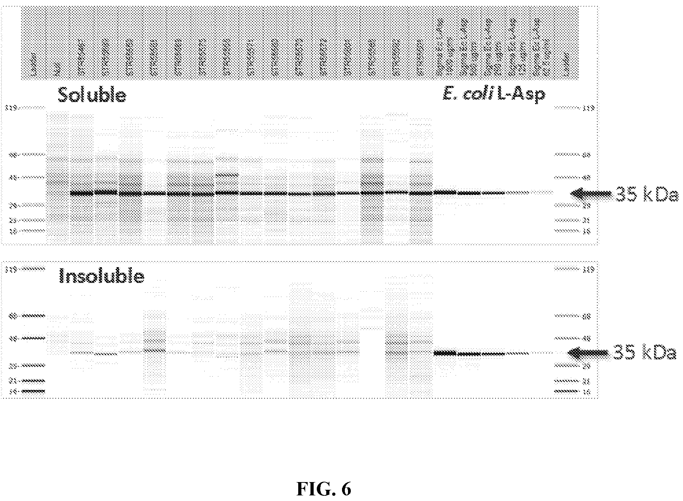

**FIG. 6. SDS-CGE Gel-like Images - *E. coli* Asparaginase Expression Plasmid Screen.** Asparaginase small scale (0.5 ml) growth whole broth sonicate soluble (upper panel) and insoluble (lower panel) samples were analyzed by reduced SDS-CGE. The lane at the far left shows molecular weight marker ladder (upper panel MW ladder 119 kDa, 68 KDa, 48 kDa, 29 kDa, 21 kDa, 16 kDa; lower panel MW ladder 119 kDa, 68 KDa, 48 kDa, 29 kDa, 21 kDa, 16 kDa) and the lane at the far right shows the same ladders. From left to right beginning immediately to the right of ladder 1 are lanes showing the expression patterns observed in Null, STR55467, STR55689, STR55559, STR55561, STR55569, STR55575, STR55555, STR55571, STR55560, STR55570, STR55572, STR55601, STR55585, STR55592, STR55501, and controls: Sigma *E. coli* L-Asparaginase 1000 $\mu$g/ml, Sigma *E. coli* L-Asparaginase 500 $\mu$g/ml, Sigma *E. coli* L-Asparaginase 250 $\mu$g/ml, Sigma *E. coli* L-Asparaginase 125 $\mu$g/ml, and Sigma *E. coli* L-Asparaginase 62.5 $\mu$g/ml. Arrows to the right of the gel images indicate migration of the asparaginase target protein (35 kDa).

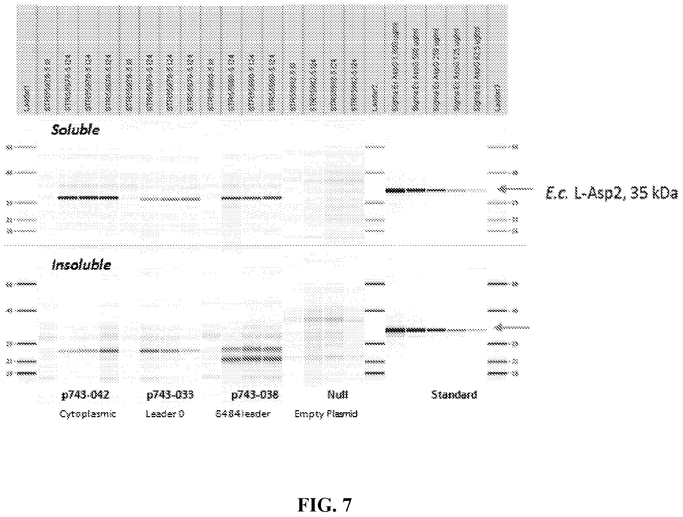

**FIG. 7. SDS-CGE Gel-like Images - Crisantaspase Shake Flask Expression Analysis.** Expression under different growth conditions as measured by soluble, reduced capillary gel electrophoresis (SDS-CGE) is shown. From left to right are lanes showing the expression patterns observed in the following samples: Ladder 1 (molecular weight markers 68, 48, 29, 21, and 16 KD); STR55987 at I0 (cytoplasmic expression with no leader); STR55987 at I24 (cytoplasmic expression with no leader); STR55987 at I24 (cytoplasmic expression with no leader); STR55987 at I24 (cytoplasmic expression with no leader); STR55979 at I0 (Leader O); STR55979 at I24 (Leader O); STR55979 at I24 (Leader O); STR55979 at I24 (Leader O); STR55980 at I0 (8484 Leader); STR55980 at I24 (8484 Leader); STR55980 at I24 (8484 Leader); STR55980 at I24 (8484 Leader); STR55982 at I0 (Null plasmid); STR55982 at I24 (Null plasmid); STR55982 at I24 (Null plasmid); STR55982 at I24 (Null plasmid); Ladder 2 (same markers as in Ladder 1); Sigma *E. coli* AspG 1,000 ug/ml (standard *E. coli* Asp2); Sigma *E. coli* AspG 500 ug/ml; Sigma *E. coli* AspG 250 ug/ml; Sigma *E. coli* AspG 125 ug/ml; Sigma *E. coli* AspG 62.5 ug/ml; and Ladder 3 (same markers as in Ladder 1), where I0 samples are taken at the time of induction and I24 samples are taken 24 hours post induction. The arrows at the right indicate migration of *E. coli* L-Asp2 (35 KD).

**FIG. 8** shows LC-MS output for analysis of protein expression samples.

## DETAILED DESCRIPTION OF THE INVENTION

### Overview

Compositions and methods for producing high levels of properly processed recombinant proteins or polypeptides in a host cell are provided. In particular, novel secretion signals are provided which promote the targeting of the recombinant protein or polypeptide of interest to the periplasm of Gram-negative bacteria or into the extracellular environment. Periplasmic secretion leaders, disclosed herein, enable transport of proteins across the inner membrane to the periplasmic space in gram negative bacteria. For recombinant expression, periplasmic expression allows for formation of disulfide bonds in the periplasm and in some cases enables high level recombinant protein expression. Expression to the periplasmic space may also enable more efficient recovery / purification of the recombinant protein. For the purposes of the present disclosure, a "secretion signal," "secretion leader," "secretion signal polypeptide," "signal peptide," or "leader sequence" is intended to refer to a peptide sequence (or the polynucleotide encoding the peptide sequence) that is useful for targeting a protein or polypeptide of interest to the periplasm of Gram-negative bacteria or into the extracellular space. The secretion signal sequences of the present invention include the secretion leaders selected from AnsB, 8484, and 5193 secretion signals, and fragments and variants thereof. The amino acid sequences for the secretion signals are set forth in SEQ ID NOS: 1-3. Examples of nucleotide sequences encoding SEQ ID NOS: 1-3 and useful in the present methods are provided in SEQ ID NOS: 4-6, respectively (Table 1). As known to those of skill in the art, an amino acid sequence can be encoded by different nucleotide sequences due to the redundancy in the genetic code. The present invention thus includes the use of peptides or proteins that have the same amino acid sequences but are encoded by different nucleotide sequences. Also provided herein are fragments and variants of these secretion signal sequences that can direct periplasmic expression of an operably linked recombinant protein or polypeptide of interest.

| Table 1: Amino Acid and Nucleic Acid Sequences | | |
| --- | --- | --- |
| Leader name | Amino Acid Sequence | DNA coding sequence |
| AnsB | MKSALKNVIPGALALLLLFP VAAQA (SEQ ID NO: 1) | ATGAAATCTGCATTGAAGAACGTTATTCCGGG CGCCCTGGCCCTTCTGCTGCTATTCCCCGTCGC CGCCCAGGCC (SEQ ID NO: 4) |
| 8484 | MRQLFFCLMLMVSLTAHA (SEQ ID NO: 2) | ATGCGACAACTATTTTTCTGTTTGATGCTGATG GTGTCGCTCACGGCGCACGCC (SEQ ID NO: 5) |
| 5193 | MQSLPFSALRLLGVLAVMV CVLLTTPARA (SEQ ID NO: 3) | ATGCAAAGCCTGCCGTTCTCTGCGTTACGCCT GCTCGGTGTGCTGGCAGTCATGGTCTGCGTGC TGTTGACGACGCCAGCCCGTGCC (SEQ ID NO: 6) |

The methods disclosed herein provide improvements of current methods of production of recombinant proteins in bacteria that often produce improperly folded, aggregated or inactive proteins. Additionally, many types of proteins require secondary modifications that are inefficiently achieved using known methods. The methods herein increase the harvest of properly folded, soluble, and/or active proteins by secreting the protein from the intracellular environment. In Gram-negative bacteria, a protein secreted from the cytoplasm often ends up in the periplasmic space, attached to the outer membrane, or in the extracellular broth. The methods also avoid formation of inclusion bodies, which are made of aggregated proteins. Secretion into the periplasmic space also has the effect of facilitating proper disulfide bond formation (Bardwell et al., 1994, "Pathways of Disulfide Bond Formation in Proteins In Vivo," in Phosphate in microorganisms : cellular and molecular biology, eds. Torriani-Gorini et al., pp. 270-5, and Manoil, 2000, Methods in Enzymol. 326:35-47, both incorporated by reference herein). Benefits of secretion of a recombinant protein include more efficient isolation of the protein; proper folding and disulfide bond formation of the transgenic protein, leading to an increase in the percentage of the protein in soluble and/or active form; reduced formation of inclusion bodies and reduced toxicity to the host cell. The potential for secretion of the protein of interest into the culture medium, in some cases, promotes continuous, rather than batch culture for protein production.

Gram-negative bacteria have evolved numerous systems for the active export of proteins across their dual membranes. These routes of secretion include, e.g.: the ABC (Type I) pathway, the Path/Fla (Type III) pathway, and the Path/Vir (Type IV) pathway for one-step translocation across both the plasma and outer membrane; the Sec (Type II), Tat, MscL, and Holins pathways for translocation across the plasma membrane; and the Sec-plus-fimbrial usher porin (FUP), Sec-plus-autotransporter (AT), Sec-plus-two partner secretion (TPS), Sec-plus-main terminal branch (MTB), and Tat-plus-MTB pathways for two-step translocation across the plasma and outer membranes. Not all bacteria have all of these secretion pathways.

Three protein systems (types I, III and IV) secrete proteins across both membranes in a single energy-coupled step. Four systems (Sec, Tat, MscL and Holins) secrete only across the inner membrane, and four other systems (MTB, FUP, AT and TPS) secrete only across the outer membrane.

In some cases, the signal sequences herein utilize the Sec secretion system. The Sec system is responsible for export of proteins with the N-terminal secretion leader across the cytoplasmic membranes (see, Agarraberes and Dice, 2001, Biochim Biophys Acta. 1513:1-24; Muller et al., 2001, Prog Nucleic Acid Res Mol. Biol. 66:107-157). Protein complexes of the Sec family are found universally in prokaryotes and eukaryotes. The bacterial Sec system consists of transport proteins, a chaperone protein (SecB) or signal recognition particle (SRP) and signal peptidases (SPase I and SPase II). The Sec transport complex in E. coli consists of three integral inner membrane proteins, SecY, SecE and SecG, and the cytoplasmic ATPase, SecA. SecA recruits SecY/E/G complexes to form the active translocation channel. The chaperone protein SecB binds to the nascent polypeptide chain to prevent it from folding and targets it to SecA. The linear polypeptide chain is subsequently transported through the SecYEG channel and, following cleavage of the signal peptide, the protein is folded in the periplasm. Three auxiliary proteins (SecD, SecF and YajC) form a complex that is not essential for secretion but stimulates secretion up to ten-fold under many conditions, particularly at low temperatures.

Proteins that are transported into the periplasm, i.e., through a type II secretion system, are also often exported into the extracellular media in a further step. The mechanisms are generally through an autotransporter, a two partner secretion system, a main terminal branch system or a fimbrial usher porin.

Of the twelve known secretion systems in Gram-negative bacteria, eight are known to utilize targeting signal peptides found as part of the expressed protein. These signal peptides interact with the proteins of the secretion systems so that the cell properly directs the protein to its appropriate destination. Five of these eight signal-peptide-based secretion systems are those that involve the Sec system. These five are referred to as involved in Sec-dependent cytoplasmic membrane translocation and their signal peptides operative therein, in some cases, are referred to as Sec-dependent secretion signals. One of the issues in developing an appropriate secretion signal is to ensure that the signal is appropriately expressed and cleaved from the expressed protein.

Signal peptides for the sec pathway generally have the following three domains: (i) a positively charged n-region, (ii) a hydrophobic-region and (iii) an uncharged but polar c-region. The cleavage site for the signal peptidase is located in the c-region. However, the degree of signal sequence conservation and length, as well as the cleavage site position, often varies between different proteins.

A signature of Sec-dependent protein export is the presence of a short (about 30 amino acids), mainly hydrophobic amino-terminal signal sequence in the exported protein. The signal sequence aids protein export and is cleaved off by a periplasmic signal peptidase when the exported protein reaches the periplasm. A typical N-terminal Sec signal peptide contains an N-domain with at least one arginine or lysine residue, followed by a domain that contains a stretch of hydrophobic residues, and a C-domain containing the cleavage site for signal peptidases.

Bacterial protein production systems have been developed in which transgenic protein constructs are engineered as fusion proteins containing both a protein of interest and a secretion signal in an attempt to target the protein out of the cytoplasm.

*P. fluorescens* has been demonstrated to be an improved platform for production of a variety of proteins and several efficient secretion signals have been identified from this organism (see, U.S. Pat. No. 7,985,564, "Expression Systems with Sec-system Secretion," herein incorporated by reference in its entirety). *P. fluorescens* produces exogenous proteins in a correctly processed form to a higher level than typically seen in other bacterial expression systems, and transports these proteins at a higher level to the periplasm of the cell, leading to increased recovery of fully processed recombinant protein. Therefore, in one embodiment, there is provided a method for producing exogenous protein in a *P. fluorescens* cell by expressing the target protein operably linked to a secretion signal.

The secretion signal sequences herein, in some cases, are useful in *Pseudomonas*. The *Pseudomonas* system offers advantages for commercial expression of polypeptides and enzymes, in comparison with other bacterial expression systems. In particular, *P. fluorescens* has been identified as an advantageous expression system. *P. fluorescens* encompasses a group of common, nonpathogenic saprophytes that colonize soil, water and plant surface environments. Commercial enzymes derived from *P. fluorescens* have been used to reduce environmental contamination, as detergent additives, and for stereoselective hydrolysis. *P. fluorescens* is also used agriculturally to control pathogens. U.S. Patent Number 4,695,462, "Cellular Encapsulation of Biological Pesticides," describes the expression of recombinant bacterial proteins in *P. fluorescens*.

## Compositions

### Secretion Leaders

In one embodiment herein, a peptide is provided, wherein the peptide is a novel secretion leader or signal useful for targeting a protein or polypeptide of interest to the periplasm of Gram-negative bacteria or into the extracellular space. In one embodiment, the peptide has an amino acid sequence that is, or is substantially homologous to, an AnsB, 8484, or 5193 secretion signal, or a fragment or variant thereof. The invention also provides a polypeptide comprising a secretion signal peptide of the invention fused to a target protein or polypeptide of interest, and expression constructs that produce a fusion protein comprising a secretion signal peptide and a polypeptide of interest. In embodiments, the secretion signal peptide is operably linked to the polypeptide of interest.

In embodiments, the secretion signal sequence is homologous to or substantially homologous to a secretion signal peptide set forth in any of SEQ ID NOS: 1-3, or is encoded by a polynucleotide sequence set forth in any of SEQ ID NOS: 4-6. In another embodiment, the secretion signal sequence comprises at least amino acids 2-25 of SEQ ID NO: 1, at least amino acids 2-18 of SEQ ID NO: 2, or at least amino acids 2-29 of SEQ ID NO: 3. In yet another embodiment, the secretion signal sequence comprises a fragment of one of SEQ ID NOS: 1-3, which is truncated by 1, 2, 3, 4, 5, 6, 7, 8, 9, or 10 amino acids from the amino terminus but retains biological activity, i.e., secretion signal activity.

In one embodiment the amino acid sequence of the peptide is a variant of a given original peptide, wherein the sequence of the variant is obtainable by replacing up to or about 30% of the original peptide's amino acid residues with other amino acid residue(s), including up to about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%, provided that the variant retains the desired function of the original peptide. A variant amino acid with substantial homology will be at least about 70%, at least about 75%, at least about 80%, about 85%, about 90%, about 95%, about 96%, about 97%, about 98%, or at least about 99% homologous to the original peptide. A variant amino acid sequence may be obtained in various ways including amino acid substitutions, deletions, truncations, and insertions of one or more amino acids of any of SEQ ID NOS:

1-3, including 1 or more, 1-5, 1-10, 1, 2, 3, 4, 5, 6, 7, 8, 9, 10, 15, 20, 25, or more amino acid substitutions, deletions, insertions, or combinations thereof.

By "substantially homologous," "substantially identical," or "substantially similar" is intended an amino acid or nucleotide sequence that has about or at least about 60%, about or at least about 65%, about or at least about 70%, about or at least about 75%, about or at least about 80%, about or at least about 85%, about or at least about 81%, about or at least about 82%, about or at least about 83%, about or at least about 84%, about or at least about 85%, about or at least about 86%, about or at least about 87%, about or at least about 88%, about or at least about 89%, about or at least about 90%, about or at least about 91%, about or at least about 92%, about or at least about 93%, about or at least about 94%, about or at least about 95%, about or at least about 96%, about or at least about 97%, about or at least about 98% or about or at least about 99%, or greater sequence identity as compared to a reference sequence using a suitable alignment program described herein or known in the art using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

In embodiments, a peptide, protein, or polypeptide used in the present invention may include one or more modifications of a "non-essential" amino acid residue. In this context, a "non-essential" amino acid residue is a residue that can be altered, e.g., deleted, substituted, or derivatized, in the novel amino acid sequence without abolishing or substantially reducing the activity (e.g., the agonist activity) of the original peptide, protein, or polypeptide (also referred to as the "analog" or "reference" peptide, protein, or polypeptide). In some embodiments, a peptide, protein, or polypeptide may include one or more modifications of an "essential" amino acid residue. In this context, an "essential" amino acid residue is a residue that when altered, e.g., deleted, substituted, or derivatized, in the novel amino acid sequence the activity of the reference peptide, protein, or polypeptide is substantially reduced or abolished. In such embodiments where an essential amino acid residue is altered, the modified peptide, protein, or polypeptide may possess an activity of the original peptide, protein, or polypeptide. The substitutions, insertions and deletions may be at the N-terminal or C-terminal end, or may be at internal portions of the peptide, protein, or polypeptide. By way of example, the peptide, protein, or polypeptide may include 1, 2, 3, 4, 5, 6, 7, 8, 9, 10, or more substitutions, both in a consecutive manner or spaced throughout the peptide, protein, or polypeptide molecule. Alone or in combination with the substitutions, the peptide, protein, or polypeptide may include 1, 2, 3, 4, 5, 6, 7, 8, 9, 10, or more insertions, again either in consecutive manner or spaced throughout the peptide, protein, or polypeptide molecule. The peptide, protein, or polypeptide, alone or in combination with the substitutions and/or insertions, may also include 1, 2, 3, 4, 5, 6, 7, 8, 9, 10, or more deletions, again either in consecutive manner or spaced throughout the peptide, protein, or polypeptide molecule. The peptide, protein, or polypeptide, alone or in combination with the substitutions, insertions and/or deletions, may also include 1, 2, 3, 4, 5, 6, 7, 8, 9, 10, or more amino acid additions.

Substitutions include conservative amino acid substitutions. A "conservative amino acid substitution" is one in which the amino acid residue is replaced with an amino acid residue having a similar side chain, or physicochemical characteristics (e.g., electrostatic, hydrogen bonding, isosteric, hydrophobic features). The amino acids may be naturally occurring or unnatural. Families of amino acid residues having similar side chains are known in the art. These families include amino acids with basic side chains (e.g. lysine, arginine, histidine), acidic side chains (e.g., aspartic acid, glutamic acid), uncharged polar side chains (e.g., glycine, asparagine, glutamine, serine, threonine, tyrosine, methionine, cysteine), nonpolar side chains (e.g., alanine, valine, leucine, isoleucine, proline, phenylalanine, tryptophan), β-branched side chains (e.g., threonine, valine, isoleucine) and aromatic side chains (e.g., tyrosine, phenylalanine, tryptophan, histidine). Substitutions may also include non-conservative changes.

Variant peptide, protein, or polypeptides encompassed herein are biologically active, that is they continue to possess the desired biological activity of the original peptide, protein, or polypeptide; for example, a variant secretion leader peptide retains secretion signal activity. By "retains activity" is intended that the variant will have about or at least about 30%, about or at least about 35%, about or at least about 40%, about or at least about 45%, about or at least about 50%, about or at least about 55%, about or at least about 60%, about or at least about 65%, about or at least about 70%, about or at least about 75%, about or at least about 80%, about or at least about 85%, about or at least about 81%, about or at least about 82%, about or at least about 83%, about or at least about 84%, about or at least about 85%, about or at least about 86%, about or at least about 87%, about or at least about 88%, about or at least about 89%, about or at least about 90%, about or at least about 91%, about or at least about 92%, about or at least about 93%, about or at least about 94%, about or at least about 95%, about or at least about 96%, about or at least about 97%, about or at least about 98% or about or at least about 99%, about or at least about 100%, about or at least about 110%, about or at least about 125%, about or at least about 150%, about or at least about 200% or greater activity, e.g., secretion signal activity, of the original peptide, protein, or polypeptide.

### Polynucleotides

The disclosure also includes a nucleic acid with a sequence that encodes a novel secretion signal useful for targeting a protein or polypeptide of interest to the periplasm of Gram-negative bacteria or into the extracellular space. In one embodiment, the isolated polynucleotide encodes a peptide sequence substantially homologous to an AnsB, 8484, or 5193 secretion signal peptide. In another embodiment, the present disclosure provides a nucleic acid that encodes a peptide sequence substantially homologous to at least amino acids 2-25 of SEQ ID NO: 1, at least amino acids 2-18 of SEQ ID NO: 2, or at least amino acids 2-29 of SEQ ID NO: 2, or provides a nucleic acid substantially homologous to any one of the nucleotide sequences set forth as SEQ ID NOS: 4-6, including biologically active variants and fragments thereof. In another embodiment, the nucleic acid sequence is about or at least about 60%, about or at least about 65%, about or at least about 70%, about or at least about 75%, about or at least about 80%, about or at least about 85%, about or at least about 81%, about or at least about 82%, about or at least about 83%, about or at least about 84%, about or at least about 85%, about or at least about 86%, about or at least about 87%, about or at least about 88%, about or at least about 89%, about or at least about 90%, about or at least about 91%, about or at least about 92%, about or at least about 93%, about or at least about 94%, about or at least about 95%, about or at least about 96%, about or at least about 97%, about or at least about 98% or about or at least about 99%, or greater identical to any one of the nucleic acid sequences set forth as SEQ ID NOS: 4-6.

In embodiments, secretion signal peptides herein are encoded by a nucleotide sequence substantially homologous to any one of the nucleotide sequences set forth as SEQ ID NOS: 4-6. Corresponding secretion signal peptide sequences having substantial identity to the secretion signal sequences of the present invention can be identified using any appropriate method known in the art, e.g., PCR, hybridization methods, or as described in the literature. See, for example, Sambrook J., and Russell, D.W., 2001, Molecular Cloning: A Laboratory Manual. Cold Spring Harbor Laboratory Press, Cold Spring Harbor, NY; and Innis, et al., 1990, PCR Protocols: A Guide to Methods and Applications; Academic Press, NY. A variant nucleotide sequence can include a synthetically derived nucleotide sequence that has been generated, for example, by using site-directed mutagenesis. In embodiments, a mutagenized sequence still encodes the secretion signal peptides disclosed herein. Variant secretion signal peptides are biologically active, that is, they continue to possess the desired biological activity of the native protein, that is, they retain secretion signaling activity. By "retains activity" is meant that the variant will have about 30%, about or at least about 35%, about or at least about 40%, about or at least about 45%, about or at least about 50%, about or at least about 55%, about or at least about 60%, about or at least about 65%, about or at least about 70%, about or at least about 75%, about or at least about 80%, about or at least about 85%, about or at least about 81%, about or at least about 82%, about or at least about 83%, about or at least about 84%, about or at least about 85%, about or at least about 86%, about or at least about 87%, about or at least about 88%, about or at least about 89%, about or at least about 90%, about or at least about 91%, about or at least about 92%, about or at least about 93%, about or at least about 94%, about or at least about 95%, about or at least about 96%, about or at least about 97%, about or at least about 98% or about or at least about 99%, about or at least about 100%, about or at least about 110%, about or at least about 125%, about or at least about 150%, about or at least about 200% or greater of the activity of the original secretion signal peptide. Any appropriate method for measuring peptide, protein, or polypeptide activity, e.g., secretion signal activity. Such methods are well known in the art, with examples discussed herein.

The skilled artisan will further appreciate that changes, in some cases, are introduced by mutation into the nucleotide sequences provided herein thereby leading to changes in the amino acid sequence of the encoded secretion signal peptides, without altering the biological activity of the secretion signal peptides. Thus, variant isolated nucleic acid molecules are often created by introducing one or more nucleotide substitutions, additions, or deletions into the corresponding nucleotide sequence disclosed herein, such that one or more amino acid substitutions, additions or deletions are introduced into the encoded protein. Mutations can be introduced by any standard technique, e.g., site-directed mutagenesis and PCR-mediated mutagenesis.

Nucleic Acid and Amino Acid Homology

Nucleic acid and amino acid sequence homology is determined according to any suitable method known in the art, including but not limited to those described herein.

For example, alignments and searches for similar sequences can be performed using the U.S. National Center for Biotechnology Information (NCBI, Bethesda, MD) program, MegaBLAST. Use of this program with options for percent identity set at, for example, 70% for amino acid sequences, or set at, for example, 90% for nucleotide sequences, will identify those sequences with 70%, or 90%, or greater sequence identity to the query sequence. Other software known in the art is also available for aligning and/or searching for similar sequences, e.g., sequences at least 70% or 90% identical to an information string containing a secretion signal sequence herein. For example, sequence alignments for comparison to identify sequences at least 70% or 90% identical to a query sequence is often performed by use of, e.g., the GAP, BESTFIT, BLAST, FASTA, and TFASTA programs available in the GCG Sequence Analysis Software Package (available from the Genetics Computer Group, University of Wisconsin Biotechnology Center, 1710 University Avenue, Madison, Wis. 53705), with the default parameters as specified therein, plus a parameter for the extent of sequence identity set at the desired percentage. Also, for example, the CLUSTAL program (available in the PC/Gene software package from Intelligenetics, Mountain View, Cal.) may be used.

These and other sequence alignment methods are well known in the art and may be conducted by manual alignment, by visual inspection, or by manual or automatic application of a sequence alignment algorithm, such as any of those embodied by the above-described programs. Various useful algorithms include, e.g.: the similarity search method described in W. R. Pearson & D. J. Lipman, Proc. Natl. Acad. Sci. USA 85:2444-48 (April 1988); the local homology method described in T. F. Smith & M. S. Waterman, in Adv. Appl. Math. 2:482-89 (1981) and in J. Molec. Biol. 147:195-97 (1981); the homology alignment method described in S. B. Needleman & C. D. Wunsch, J. Molec. Biol. 48(3):443-53 (March 1970); and the various methods described, e.g., by W. R. Pearson, in Genomics 11(3):635-50 (November 1991); by W. R. Pearson, in Methods Molec. Biol. 24:307-31 and 25:365-89 (1994); and by D. G. Higgins & P. M. Sharp, in Comp. Appl'ns in Biosci. 5:151-53 (1989) and in Gene 73(1):237-44 (15 Dec. 1988).

GAP Version 10, which uses the algorithm of Needleman and Wunsch (1970) supra, can be used to determine sequence identity or similarity using the following parameters: % identity and % similarity for a nucleotide sequence using GAP Weight of 50 and Length Weight of 3, and the nwsgapdna.cmp scoring matrix; % identity or % similarity for an amino acid sequence using GAP weight of 8 and length weight of 2, and the BLOSUM62 scoring program. Equivalent or similar programs may also be used as will be understood by one of skill in the art. For example, a sequence comparison program can be used that, for any two sequences in question, generates an alignment having identical nucleotide residue matches and an identical percent sequence identity when compared to the corresponding alignment generated by GAP Version 10. In embodiments, the sequence comparison is performed across the entirety of the query or the subject sequence, or both.

Hybridization Conditions

In another aspect herein, a nucleic acid that hybridizes to an isolated nucleic acid with a sequence that encodes a peptide with a sequence substantially similar to an AnsB, 8484, or 5193 secretion signal peptide is provided. In certain embodiments, the hybridizing nucleic acid will bind under high stringency conditions. In various embodiments, the hybridization occurs across substantially the entire length of the nucleotide sequence encoding the secretion signal peptide, for example, across substantially the entire length of one or more of SEQ ID NOS: 4-6. A nucleic acid molecule hybridizes to "substantially the entire length" of a secretion signal-encoding nucleotide sequence disclosed herein when the nucleic acid molecule hybridizes over at least 80% of the entire length of one or more of SEQ ID NOS: 4-6, at least 85%, at least 90%, or at least 95% of the entire length. Unless otherwise specified, "substantially the entire length" refers to at least 80% of the entire length of the secretion signal-encoding nucleotide sequence where the length is measured in contiguous nucleotides (e.g., hybridizes to at least 60 contiguous nucleotides of SEQ ID NO: 4, at least 43 contiguous nucleotides of SEQ ID NO: 5, or at least 43 contiguous nucleotides of SEQ ID NO: 6, etc.).

In a hybridization method, all or part of the nucleotide sequence encoding the secretion signal peptide, in some cases, is used to screen cDNA or genomic libraries. Methods for construction of such cDNA and genomic libraries are generally known in the art and are disclosed in Sambrook and Russell, 2001. The so-called hybridization probes may be genomic DNA fragments, cDNA fragments, RNA fragments, or other oligonucleotides, and may be labeled with a detectable group such as 32P, or any other detectable marker, such as other radioisotopes, a fluorescent compound, an enzyme, or an enzyme co-factor. Probes for hybridization are often be made by labeling synthetic oligonucleotides based on the known secretion signal peptide-encoding nucleotide sequence disclosed herein. Degenerate primers designed on the basis of conserved nucleotides or amino acid residues in the nucleotide sequence or encoded amino acid sequence are sometimes additionally be used. The probe typically comprises a region of nucleotide sequence that hybridizes under stringent conditions to at least about 10, at least about 11, at least about 12, at least about 13, at least about 14, at least about 15, at least about 16, at least about 17, at least about 18, at least about 19, at least about 20, or more consecutive nucleotides of a secretion signal peptide-encoding nucleotide sequence herein or a fragment or variant thereof. Methods for the preparation of probes for hybridization are generally known in the art and are disclosed in Sambrook and Russell, 2001, herein incorporated by reference.

In hybridization techniques, all or part of a known nucleotide sequence is used as a probe that selectively hybridizes to other corresponding nucleotide sequences present in a population of cloned genomic DNA fragments or cDNA fragments (i.e., genomic or cDNA libraries) from a chosen organism. The hybridization probes may be genomic DNA fragments, cDNA fragments, RNA fragments, or other oligonucleotides, and may be labeled with a detectable group such as 32P, or any other detectable marker. Thus, for example, probes for hybridization are often made by labeling synthetic oligonucleotides based on the secretion signal peptide-encoding nucleotide sequence herein. Methods for the preparation of probes for hybridization and for construction of cDNA and genomic libraries are generally known in the art and are disclosed in Sambrook et al., 1989, Molecular Cloning: A Laboratory Manual, 2nd ed., Cold Spring Harbor Laboratory Press, Plainview, New York.

The entire secretion signal peptide-encoding nucleotide sequence disclosed herein, or one or more portions thereof, may be used as a probe capable of specifically hybridizing to corresponding nucleotide sequences and messenger RNAs encoding secretion signal peptides. These probes can include sequences that are unique and are preferably at least about 10 nucleotides in length, or at least about 15 nucleotides in length. Such probes may be used to amplify corresponding secretion signal peptide-encoding nucleotide sequences from a chosen organism by PCR. This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al., 1989.

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe is often identified (homologous probing). Alternatively, stringency conditions are sometimes adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 60 °C. In embodiments, the temperature is about 68 °C. Stringent conditions may also be achieved with the addition of destabilizing agents such as formamide. Low stringency conditions can include e.g., hybridization with a buffer solution of 30 to 35% formamide, 1 M NaCl, 1% SDS (sodium dodecyl sulfate) at 37 °C, and a wash in 1X to 2X SSC (20X SSC = 3.0 M NaCl/0.3 M trisodium citrate) at 50 to 55 °C. Exemplary moderate stringency conditions include hybridization in 40 to 45% formamide, 1.0 M NaCl, 1% SDS at 37 °C, and a wash in 0.5X to 1X SSC at 55 to 60 °C. Exemplary high stringency conditions include hybridization in 50% formamide, 1 M NaCl, 1% SDS at 37 °C, and a wash in 0.1X SSC at 60 to 68 °C. Optionally, wash buffers may comprise about 0.1% to about 1% SDS. Duration of hybridization is generally less than about 24 hours, and typically about 4 to about 12 hours.

Specificity is typically the function of post-hybridization washes, the critical factors being the ionic strength and temperature of the final wash solution. For DNA-DNA hybrids, the Tm is often approximated from the equation of Meinkoth and Wahl (1984) Anal. Biochem. 138:267-284: $Tm = 81.5\,°C + 16.6\,(\log M) + 0.41\,(\%GC) - 0.61\,(\%\,form) - 500/L$; where M is the molarity of monovalent cations, %GC is the percentage of guanosine and cytosine nucleotides in the DNA, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1 °C for each 1% of mismatching; thus, Tm, hybridization, and/or wash conditions are sometimes adjusted to hybridize to sequences of the desired identity. For example, if sequences with >90% identity are sought, the Tm is often decreased 10 °C. Generally, stringent conditions are selected to be about 5 °C lower than the thermal melting point (Tm) for the specific sequence and its complement at a defined ionic strength and pH. However, severely stringent conditions often utilize a hybridization and/or wash at 1, 2, 3, or 4 °C lower than the thermal melting point (Tm); moderately stringent conditions often utilize a hybridization and/or wash at 6, 7, 8, 9, or 10 °C lower than the thermal melting point (Tm); low stringency conditions often utilize a hybridization and/or wash at 11, 12, 13, 14, 15, or 20 °C lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45 °C (aqueous solution) or 32 °C (formamide solution), it is preferred to increase the SSC concentration so that a higher temperature is used. A guide to the hybridization of nucleic acids is found in Tijssen, 1993, Laboratory Techniques in Biochemistry and Molecular Biology-Hybridization with Nucleic Acid Probes, Part I, Chapter 2, Elsevier, New York; and Ausubel et al., eds., 1995, Current Protocols in Molecular Biology, Chapter 2, Greene Publishing and Wiley-Interscience, New York. See Sambrook et al., 1989.

Codon Optimization

In one embodiment, the compositions and methods herein comprise expression of a recombinant protein or polypeptide of interest from a construct that has been optimized for codon usage in a strain of interest. In embodiments, the strain is a *Pseudomonas* host cell, e.g., *Pseudomonas fluorescens.* Methods for optimizing codons to improve expression in bacterial hosts are known in the art and described in the literature. For example, optimization of codons for expression in a *Pseudomonas* host strain is described, e.g., in U.S. Pat. App. Pub. No.2007/0292918, "Codon Optimization Method," incorporated herein by reference in its entirety.

In heterologous expression systems, optimization steps may improve the ability of the host to produce the foreign protein. Protein expression is governed by a host of factors including those that affect transcription, mRNA processing, and stability and initiation of translation. The polynucleotide optimization steps may include steps to improve the ability of the host to produce the foreign protein as well as steps to assist the researcher in efficiently designing expression constructs. Optimization strategies may include, for example, the modification of translation initiation regions, alteration of mRNA structural elements, and the use of different codon biases. Methods for optimizing the nucleic acid sequence of to improve expression of a heterologous protein in a bacterial host are known in the art and described in the literature. For example, optimization of codons for expression in a *Pseudomonas* host strain is described, e.g., in U.S. Pat. App. Pub. No.2007/0292918, "Codon Optimization Method," incorporated herein by reference in its entirety.

Optimization addresses any of a number of sequence features of the heterologous gene. As a specific example, a rare codon-induced translational pause often results in reduced heterologous protein expression. A rare codon-induced translational pause includes the presence of codons in the polynucleotide of interest that are rarely used in the host organism may have a negative effect on protein translation due to their scarcity in the available tRNA pool. One method of improving optimal translation in the host organism includes performing codon optimization which sometimes results in rare host codons being removed from the synthetic polynucleotide sequence.

Alternate translational initiation also sometimes results in reduced heterologous protein expression. Alternate translational initiation includes a synthetic polynucleotide sequence inadvertently containing motifs capable of functioning as a ribosome binding site (RBS). These sites, in some cases, result in initiating translation of a truncated protein from a gene-internal site. One method of reducing the possibility of producing a truncated protein, which are often difficult to remove during purification, includes eliminating putative internal RBS sequences from an optimized polynucleotide sequence.

Repeat-induced polymerase slippage often results in reduced heterologous protein expression. Repeat-induced polymerase slippage involves nucleotide sequence repeats that have been shown to cause slippage or stuttering of DNA polymerase which sometimes results in frameshift mutations. Such repeats also often cause slippage of RNA polymerase. In an organism with a high G+C content bias, there is sometimes a higher degree of repeats composed of G or C nucleotide repeats. Therefore, one method of reducing the possibility of inducing RNA polymerase slippage, includes altering extended repeats of G or C nucleotides.

Interfering secondary structures also sometimes result in reduced heterologous protein expression. Secondary structures often sequester the RBS sequence or initiation codon and have been correlated to a reduction in protein expression. Stem loop structures are also often involved in transcriptional pausing and attenuation. An optimized polynucleotide sequence usually contains minimal secondary structures in the RBS and gene coding regions of the nucleotide sequence to allow for improved transcription and translation.

Another feature that sometimes effect heterologous protein expression is the presence of restriction sites. By removing restriction sites that could interfere with subsequent sub-cloning of transcription units into host expression vectors a polynucleotide sequence is optimized.

For example, the optimization process often begins by identifying the desired amino acid sequence to be heterologously expressed by the host. From the amino acid sequence a candidate polynucleotide or DNA is designed. During the design of the synthetic DNA sequence, the frequency of codon usage is often compared to the codon usage of the host expression organism and rare host codons are removed from the synthetic sequence. Additionally, the synthetic candidate DNA sequence is sometimes modified in order to remove undesirable enzyme restriction sites and add or remove any desired signal sequences, linkers or untranslated regions. The synthetic DNA sequence is often analyzed for the presence of secondary structure that may interfere with the translation process, such as G/C repeats and stem-loop structures. Before the candidate DNA sequence is synthesized, the optimized sequence design is often be checked to verify that the sequence correctly encodes the desired amino acid sequence. Finally, the candidate DNA sequence is synthesized using DNA synthesis techniques, such as those known in the art.

In another embodiment herein, the general codon usage in a host organism, such as *P. fluorescens,* is often utilized to optimize the expression of the heterologous polynucleotide sequence. The percentage and distribution of codons that rarely would be considered as preferred for a particular amino acid in the host expression system is evaluated. Values of 5% and 10% usage is often used as cutoff values for the determination of rare codons. For example, the codons listed in Table 2 have a calculated occurrence of less than 5% in the *P. fluorescens* MB214 genome and would be generally avoided in an optimized gene expressed in a *P. fluorescens* host.

**Table 2.** Codons occurring at less than 5% in *P. fluorescens* MB214

| Amino Acid(s) | Codon(s) Used | % Occurrence |
|---|---|---|
| G Gly | GGA | 3.26 |
| I Ile | ATA | 3.05 |
| L Leu | CTA | 1.78 |
| | CTT | 4.57 |
| | TTA | 1.89 |
| R Arg | AGA | 1.39 |
| | AGG | 2.72 |
| | CGA | 4.99 |

(continued)

| Amino Acid(s) | Codon(s) Used | % Occurrence |
|---|---|---|
| S Ser | TCT | 4.28 |

The present disclosure contemplates the use of any polypeptide or protein of interest coding sequence, including any sequence that has been optimized for expression in the *Pseudomonas* host cell being used. Sequences contemplated for use are often optimized to any degree as desired, including, but not limited to, optimization to eliminate: codons occurring at less than 5% in the *Pseudomonas* host cell, codons occurring at less than 10% in the *Pseudomonas* host cell, a rare codon-induced translational pause, a putative internal RBS sequence, an extended repeat of G or C nucleotides, an interfering secondary structure, a restriction site, or combinations thereof.

Furthermore, the amino acid sequence of any secretion leader useful in practicing the methods provided herein is encoded by any appropriate nucleic acid sequence. Codon optimization for expression in *E. coli* is described, e.g., by Welch, et al., 2009, PLoS One, "Design Parameters to Control Synthetic Gene Expression in Escherichia coli," 4(9): e7002, Ghane, et al., 2008, Krishna R. et al., (2008) Mol Biotechnology "Optimization of the AT-content of Codons Immediately Downstream of the Initiation Codon and Evaluation of Culture Conditions for High-level Expression of Recombinant Human G-CSF in Escherichia coli," 38:221-232.

## Expression Systems

Methods herein, in some cases, comprise expressing polypeptides comprising a protein or polypeptide of interest operably linked to a secretion signal peptide selected from the group consisting of an AnsB, 8484, or 5193 secretion signal sequence, or a sequence that is substantially homologous to the secretion signal peptide sequence disclosed herein as SEQ ID NOS: 1-3. In embodiments, the secretion signal peptide sequence is encoded by a nucleotide sequence set forth as SEQ ID NOS: 4-6, from an expression construct in a *Pseudomonas* host cell. The expression construct, in some cases, is a plasmid. In some embodiments, a plasmid encoding the polypeptide or protein of interest sequence comprises a selection marker, and host cells maintaining the plasmid are grown under selective conditions. In some embodiments, the plasmid does not comprise a selection marker. In some embodiments, the expression construct is integrated into the host cell genome.

Methods for expressing heterologous proteins, including regulatory sequences (e.g., promoters, secretion leaders, and ribosome binding sites) useful in the methods of the invention in host strains, including *Pseudomonas* host strains, are described, e.g., in U.S. Patent No. 7, 618,799, "Bacterial leader sequences for increased expression," in U.S. Pat. No. 7,985,564, "Expression systems with Sec-system secretion," in U.S. Pat. Nos. 9,394,571 and 9,580,719, both titled "Method for Rapidly Screening Microbial Hosts to Identify Certain Strains with Improved Yield and/or Quality in the Expression of Heterologous Proteins," U.S. Pat. No. 9,453,251, "Expression of Mammalian Proteins in *Pseudomonas fluorescens,"* U.S. Pat. No. 8,603,824, "Process for Improved Protein Expression by Strain Engineering," and U.S. Pat. No. 8,530,171, "High Level Expression of Recombinant Toxin Proteins," each incorporated herein by reference in its entirety. In embodiments, a secretion leader used in the context of the present invention is a secretion leader as disclosed in any of U.S. Pat. Nos. 7, 618,799, 7,985,564, 9,394,571, 9,580,719, 9,453,251, 8,603,824, and 8,530,171. These patents also describe bacterial host strains useful in practicing the methods herein, that have been engineered to overexpress folding modulators or wherein protease mutations have been introduced, in order to increase heterologous protein expression.

In embodiments, an expression strain used in the methods of the invention is any expression strain described in Example 4, as listed in Table 13. In embodiments, an expression strain used in the methods of the invention is a microbial expression strain having a background phenotype of an expression strain described in Example 4, as listed in Table 13. In embodiments, an expression strain used in the methods of the invention is a microbial expression strain having a background phenotype of an expression strain described in Example 4, as listed in Table 13, and wherein the strain expresses the recombinant asparaginase in a fusion with the respective secretion leader as listed in Table 13. In embodiments, an expression strain used in the methods of the invention is a microbial expression strain having a background phenotype of expression strain STR57864, STR57865, STR57866, STR57860, STR57861, STR57862, STR57863 described in Example 4, as listed in Table 13, except that the expression strain is not a folding modulator overexpressor. In embodiments, an expression strain used in the methods of the invention is a microbial expression strain having a background phenotype of expression strain STR57864, STR57865, STR57866, STR57860, STR57861, STR57862, STR57863 described in Example 4, as listed in Table 13, cultured without mannitol.

### Promoters

The promoters used in accordance with the methods herein may be constitutive promoters or regulated promoters. Common examples of useful regulated promoters include those of the family derived from the lac promoter (i.e. the lacZ promoter), especially the tac and trc promoters described in U.S. Pat. No. 4,551,433 to DeBoer, as well Ptac16, Ptac17, PtacII, PlacUV5, and the T7lac promoter. In one embodiment, the promoter is not derived from the host cell organism. In certain embodiments, the promoter is derived from an *E. coli* organism.

Inducible promoter sequences are used to regulate expression of polypeptides or proteins of interest in accordance with the methods herein. In embodiments, inducible promoters useful in the methods herein include those of the family derived from the lac promoter (i.e. the lacZ promoter), especially the tac and trc promoters described in U.S. Pat. No. 4,551,433 to DeBoer, as well as Ptac16, Ptac17, PtacII, PlacUV5, and the T7lac promoter. In one embodiment, the promoter is not derived from the host cell organism. In certain embodiments, the promoter is derived from an *E. coli* organism. In some embodiments, a lac promoter is used to regulate expression of the polypeptide or protein of interest from a plasmid. In the case of the lac promoter derivatives or family members, e.g., the tac promoter, an inducer is IPTG (isopropyl-$\beta$-D-1-thiogalactopyranoside, also called "isopropylthiogalactoside"). In certain embodiments, IPTG is added to culture to induce expression of the polypeptide or protein of interest from a lac promoter in a *Pseudomonas* host cell.

Common examples of non-lac-type promoters useful in expression systems according to the methods herein include, e.g., those listed in **Table 3**.

| Table 3. Examples of non-*lac* Promoters | |
| --- | --- |
| **Promoter** | **Inducer** |
| $P_R$ | High temperature |
| $P_L$ | High temperature |
| Pm | Alkyl- or halo-benzoates |
| Pu | Alkyl- or halo-toluenes |
| Psal | Salicylates |
| $P_{BAD}$ | arabinose |

See, e.g.: J. Sanchez-Romero & V. De Lorenzo (1999) Manual of Industrial Microbiology and Biotechnology (A. Demain & J. Davies, eds.) pp. 460-74 (ASM Press, Washington, D.C.); H. Schweizer (2001) Current Opinion in Biotechnology, 12:439-445; R. Slater & R. Williams (2000 Molecular Biology and Biotechnology (J. Walker & R. Rapley, eds.) pp. 125-54 (The Royal Society of Chemistry, Cambridge, UK); and L.-M. Guzman, et al., 1995, J. Bacteriol. 177(14): 4121-4130, all incorporated by reference herein. A promoter having the nucleotide sequence of a promoter native to the selected bacterial host cell also may be used to control expression of the transgene encoding the recombinant protein or polypeptide of interest, e.g, a *Pseudomonas* anthranilate or benzoate operon promoter (Pant, Pben). Tandem promoters may also be used in which more than one promoter is covalently attached to another, whether the same or different in sequence, e.g., a Pant-Pben tandem promoter (interpromoter hybrid) or a Plac-Plac tandem promoter, or whether derived from the same or different organisms.

Regulated promoters utilize promoter regulatory proteins in order to control transcription of the gene of which the promoter is a part. Where a regulated promoter is used herein, a corresponding promoter regulatory protein will also be part of an expression system according to methods herein. Examples of promoter regulatory proteins include: activator proteins, e.g., *E. coli* catabolite activator protein, MalT protein; AraC family transcriptional activators; repressor proteins, e.g., *E. coli LacI* proteins; and dual-function regulatory proteins, e.g., *E. coli* NagC protein. Many regulated-promoter/-promoter-regulatory-protein pairs are known in the art. In one embodiment, the expression construct for the target protein(s) and the heterologous protein of interest are under the control of the same regulatory element.

Promoter regulatory proteins interact with an effector compound, i.e., a compound that reversibly or irreversibly associates with the regulatory protein so as to enable the protein to either release or bind to at least one DNA transcription regulatory region of the gene that is under the control of the promoter, thereby permitting or blocking the action of a transcriptase enzyme in initiating transcription of the gene. Effector compounds are classified as either inducers or co-repressors, and these compounds include native effector compounds and gratuitous inducer compounds. Many regulated-promoter/promoter-regulatory-protein/effector-compound trios are known in the art. Although, in some cases, an effector compound is used throughout the cell culture or fermentation, in one embodiment in which a regulated promoter is used, after growth of a desired quantity or density of host cell biomass, an appropriate effector compound is added to the culture to directly or indirectly result in expression of the desired gene(s) encoding the protein or polypeptide of interest.

In embodiments wherein a lac family promoter is utilized, a *lacI* gene is sometimes present in the system. The *lacI* gene, which is normally a constitutively expressed gene, encodes the Lac repressor protein *LacI* protein, which binds to the lac operator of lac family promoters. Thus, where a lac family promoter is utilized, the *lacI* gene is sometimes also included and expressed in the expression system.

Promoter systems useful in *Pseudomonas* are described in the literature, e.g., in U.S. Pat. App. Pub. No. 2008/0269070, also referenced above.

Other Regulatory Elements

In embodiments, the expression vector contains an optimal ribosome binding sequence. Modulating translation strength by altering the translation initiation region of a protein of interest can be used to improve the production of heterologous cytoplasmic proteins that accumulate mainly as inclusion bodies due to a translation rate that is too rapid. Secretion of heterologous proteins into the periplasmic space of bacterial cells can also be enhanced by optimizing rather than maximizing protein translation levels such that the translation rate is in sync with the protein secretion rate.

The translation initiation region has been defined as the sequence extending immediately upstream of the ribosomal binding site (RBS) to approximately 20 nucleotides downstream of the initiation codon (McCarthy et al. (1990) Trends in Genetics 6:78-85, herein incorporated by reference in its entirety). In prokaryotes, alternative RBS sequences can be utilized to optimize translation levels of heterologous proteins by providing translation rates that are decreased with respect to the translation levels using the canonical, or consensus, RBS sequence (AGGAGG; SEQ ID NO: 11) described by Shine and Dalgarno (Proc. Natl. Acad. Sci. USA 71: 1342-1346, 1974). By "translation rate" or "translation efficiency" is intended the rate of mRNA translation into proteins within cells. In most prokaryotes, the Shine-Dalgarno sequence assists with the binding and positioning of the 30S ribosome component relative to the start codon on the mRNA through interaction with a pyrimidine-rich region of the 16S ribosomal RNA. The RBS (also referred to herein as the Shine-Dalgarno sequence) is located on the mRNA downstream from the start of transcription and upstream from the start of translation, typically from 4 to 14 nucleotides upstream of the start codon, and more typically from 8 to 10 nucleotides upstream of the start codon. Because of the role of the RBS sequence in translation, there is a direct relationship between the efficiency of translation and the efficiency (or strength) of the RBS sequence.

In some embodiments, modification of the RBS sequence results in a decrease in the translation rate of the heterologous protein. This decrease in translation rate may correspond to an increase in the level of properly processed protein or polypeptide per gram of protein produced, or per gram of host protein. The decreased translation rate can also correlate with an increased level of recoverable protein or polypeptide produced per gram of recombinant or per gram of host cell protein. The decreased translation rate can also correspond to any combination of an increased expression, increased activity, increased solubility, or increased translocation (e.g., to a periplasmic compartment or secreted into the extracellular space). In this embodiment, the term "increased" is relative to the level of protein or polypeptide that is produced, properly processed, soluble, and/or recoverable when the protein or polypeptide of interest is expressed under the same conditions, and wherein the nucleotide sequence encoding the polypeptide comprises the canonical RBS sequence. Similarly, the term "decreased" is relative to the translation rate of the protein or polypeptide of interest wherein the gene encoding the protein or polypeptide comprises the canonical RBS sequence. The translation rate can be decreased by at least about 5%, at least about 10%, at least about 15%, at least about 20%, about 25%, about 30%, about 35%, about 40%, about 45%, about 50%, about 55%, about 60%, about 65%, about 70, at least about 75% or more, or at least about 2-fold, about 3-fold, about 4-fold, about 5-fold, about 6-fold, about 7-fold, or greater.

In some embodiments, the RBS sequence variants described herein can be classified as resulting in high, medium, or low translation efficiency. In one embodiment, the sequences are ranked according to the level of translational activity compared to translational activity of the canonical RBS sequence. A high RBS sequence has about 60% to about 100% of the activity of the canonical sequence. A medium RBS sequence has about 40% to about 60% of the activity of the canonical sequence. A low RBS sequence has less than about 40% of the activity of the canonical sequence.

Examples of RBS sequences are shown in **Table 4.** The sequences were screened for translational strength using COP-GFP as a reporter gene and ranked according to percentage of consensus RBS fluorescence. Each RBS variant was placed into one of three general fluorescence ranks: High ("Hi" - 100% Consensus RBS fluorescence), Medium ("Med" - 46-51% of Consensus RBS fluorescence), and Low ("Lo" - 16-29% Consensus RBS fluorescence).

| Table 4. RBS Sequences | | | |
|---|---|---|---|
| **RBS** | **Sequence** | **Binding Strength** | **SEQ ID NO:** |
| Consensus | AGGAGG | High | 11 |
| RBS2 | GGAGCG | Med | 12 |
| RBS34 | GGAGCG | Med | 13 |
| RBS41 | AGGAGT | Med | 14 |
| RBS43 | GGAGTG | Med | 15 |

(continued)

| Table 4. RBS Sequences | | | |
|---|---|---|---|
| **RBS** | **Sequence** | **Binding Strength** | **SEQ ID NO:** |
| RBS48 | GAGTAA | Low | 16 |
| RBS1 | AGAGAG | Low | 17 |
| RBS35 | AAGGCA | Low | 18 |
| RBS49 | CCGAAC | Low | 19 |

An expression construct useful in practicing the methods herein include, in addition to the protein coding sequence, the following regulatory elements operably linked thereto: a promoter, a ribosome binding site (RBS), a transcription tenninator, and translational start and stop signals. Useful RBSs are obtained from any of the species useful as host cells in expression systems according to, e.g., U.S. Pat. App. Pub. No. 2008/0269070 and U.S. Pat. App. Ser. No. 12/610,207. Many specific and a variety of consensus RBSs are known, e.g., those described in and referenced by D. Frishman et al., Gene 234(2):257-65 (8 Jul. 1999); and B. E. Suzek et al., Bioinformatics 17(12):1123-30 (December 2001). In addition, either native or synthetic RBSs may be used, e.g., those described in: EP 0207459 (synthetic RBSs); O. Ikehata et al., Eur. J. Biochem. 181(3):563-70 (1989). Further examples of methods, vectors, and translation and transcription elements, and other elements useful in the methods herein are described in, e.g.: U.S. Pat. No. 5,055,294 to Gilroy and U.S. Pat. No. 5,128,130 to Gilroy et al.; U.S. Pat. No. 5,281,532 to Rammler et al.; U.S. Pat. Nos. 4,695,455 and 4,861,595 to Barnes et al.; U.S. Pat. No. 4,755,465 to Gray et al.; and U.S. Pat. No. 5,169,760 to Wilcox.

Host Strains

Bacterial hosts, including *Pseudomonads,* and closely related bacterial organisms are contemplated for use in practicing the methods herein. In certain embodiments, the *Pseudomonad* host cell is *Pseudomonas fluorescens.* In some cases, the host cell is an *E. coli* cell.

Host cells and constructs useful in practicing the methods herein are identified or made using reagents and methods known in the art and described in the literature, e.g., in U.S. Pat. App. Pub. No. 2009/0325230, "Protein Expression Systems," incorporated herein by reference in its entirety. This publication describes production of a recombinant polypeptide by introduction of a nucleic acid construct into an auxotrophic *Pseudomonas fluorescens* host cell comprising a chromosomal *lacI* gene insert. The nucleic acid construct comprises a nucleotide sequence encoding the recombinant polypeptide operably linked to a promoter capable of directing expression of the nucleic acid in the host cell, and also comprises a nucleotide sequence encoding an auxotrophic selection marker. The auxotrophic selection marker is a polypeptide that restores prototrophy to the auxotrophic host cell. In embodiments, the cell is auxotrophic for proline, uracil, or combinations thereof. In embodiments, the host cell is derived from MB101 (ATCC deposit PTA-7841). U. S. Pat. App. Pub. No. 2009/0325230, "Protein Expression Systems," and in Schneider, et al., 2005, "Auxotrophic markers pyrF and proC, in some cases, replace antibiotic markers on protein production plasmids in high-cell-density Pseudomonas fluorescens fermentation," Biotechnol. Progress 21(2): 343-8, both incorporated herein by reference in their entirety, describe a production host strain auxotrophic for uracil that was constructed by deleting the *pyrF* gene in strain MB101. The *pyrF* gene was cloned from strain MB214 (ATCC deposit PTA-7840) to generate a plasmid that complements the *pyrF* deletion to restore prototropy. In particular embodiments, a dual *pyrF-proC* dual auxotrophic selection marker system in a *P. fluorescens* host cell is used. A *pyrF* deleted production host strain as described is often used as the background for introducing other desired genomic changes, including those described herein as useful in practicing the methods herein.

In embodiments, the host cell is of the order *Psezidomonadales.* Where the host cell is of the order *Pseudomonadales,* it may be a member of the family *Pseudomonadaceae,* including the genus *Pseudomonas.* Gamma Proteobacterial hosts include members of the species Escherichia coli and members of the species *Pseudomonas fluorescens.* Host cells of the order *Pseudomonadales,* of the family *Pseudomonadaceae,* or of the genus *Psudomonas* are identifiable by one of skill in the art and are described in the literature (e.g., Bergey's Manual of Systematics of Archaea and Bacteria (online publication, 2015)). Additionally, in such strains proteases can be inactivated, and folding modulator overexpression constructs introduced, using methods well known in the art.

It would be understood by one of skill in the art that a production host strain useful in the methods of the present invention can be generated using a publicly available host cell, for example, *P. fluorescens* MB101, e.g., by inactivating the *pyrF* gene, using any of many appropriate methods known in the art and described in the literature. It is also understood that a prototrophy restoring plasmid can be transformed into the strain, e.g., a plasmid carrying the *pyrF* gene from strain MB214 using any of many appropriate methods known in the art and described in the literature. Additionally, in such strains proteases can be inactivated, and folding modulator overexpression constructs introduced, using methods well known in the art.

Other *Pseudomonas* organisms may also be useful. *Pseudomonads* and closely related species include Gram-negative Proteobacteria Subgroup 1, which include the group of Proteobacteria belonging to the families and/or genera described in Bergey's Manual of Systematics of Archaea and Bacteria (online publication, 2015). **Table 5** presents these families and genera of organisms.

| **Table 5.** Families and Genera Listed in the Part, "Gram-Negative Aerobic Rods and Cocci" (in Bergey's Manual of Systematics of Archaea and Bacteria (online publication, 2015)) | |
| --- | --- |
| Family I. Pseudomonaceae | Gluconobacter<br>*Pseudomonas*<br>Xanthomonas<br>Zoogloea |
| Family II. Azotobacteraceae | Azomonas<br>Azotobacter<br>Beijerinckia<br>Derxia |
| Family III. Rhizobiaceae | Agrobacterium<br>Rhizobium |
| Family IV. Methylomonadaceae | Methylococcus<br>Methylomonas |
| Family V. Halobacteriaceae | Halobacterium<br>Halococcus |
| Other Genera | Acetobacter<br>Alcaligenes<br>Bordetella<br>Brucella<br>Francisella<br>Thermus |

*Pseudomonas* and closely related bacteria are generally part of the group defined as "Gram(-) Proteobacteria Subgroup 1" or "Gram-Negative Aerobic Rods and Cocci" (Bergey's Manual of Systematics of Archaea and Bacteria (online publication, 2015)). *Pseudomonas* host strains are described in the literature, e.g., in U.S. Pat. App. Pub. No. 2006/0040352, cited above.

"Gram-negative Proteobacteria Subgroup 1" also includes Proteobacteria that would be classified in this heading according to the criteria used in the classification. The heading also includes groups that were previously classified in this section but are no longer, such as the genera *Acidovorax, Brevundimonas, Burkholderia, Hydrogenophaga, Oceanimonas, Ralstonia,* and *Stenotrophomonas,* the genus *Sphingomonas* (and the genus *Blastomonas,* derived therefrom), which was created by regrouping organisms belonging to (and previously called species of) the genus *Xanthomonas,* the genus *Acidomonas,* which was created by regrouping organisms belonging to the genus *Acetobacter* as defined in Bergey's Manual of Systematics of Archaea and Bacteria (online publication, 2015). In addition hosts include cells from the genus *Pseudomonas, Pseudomonas enalia* (ATCC 14393), *Pseudomonas nigrifaciensi* (ATCC 19375), and *Pseudomonas putrefaciens* (ATCC 8071), which have been reclassified respectively as *Alteromonas haloplanktis, Alteromonas nigrifaciens,* and *Alteromonas putrefaciens.* Similarly, e.g., *Pseudomonas acidovorans* (ATCC 15668) and *Pseudomonas testosteroni* (ATCC 11996) have since been reclassified as *Comamonas acidovorans* and *Comamonas testosteroni,* respectively; and *Pseudomonas nigrifaciens* (ATCC 19375) and *Pseudomonas piscicida* (ATCC 15057) have been reclassified respectively as *Pseudoalteromonas nigrifaciens* and *Pseudoalteromonas piscicida.* "Gram-negative Proteobacteria Subgroup 1" also includes Proteobacteria classified as belonging to any of the families: *Pseudomonadaceae, Azotobacteraceae* (now often called by the synonym, the "Azotobacter group" of *Pseudomonadaceae), Rhizobiaceae,* and *Methylomonadaceae* (now often called by the synonym, "Methylococcaceae"). Consequently, in addition to those genera otherwise described herein, further Proteobacterial genera falling within "Gram-negative Proteobacteria Subgroup 1" include: 1) *Azotobacter* group bacteria of the genus *Azorhizophilus; 2) Pseudomonadaceae* family bacteria of the genera *Cellvibrio, Oligella,* and *Teredinibacter*; 3) *Rhizobiaceae* family bacteria of the genera *Chelatobacter, Ensifer,*

*Liberibacter* (also called "Candidatus Liberibacter"), and *Sinorhizobium*; and 4) *Methylococcaceae* family bacteria of the genera *Methylobacter, Methylocaldum, Methylomicrobium, Methylosarcina,* and *Methylosphaera.*

The host cell, in some cases, is selected from "Gram-negative Proteobacteria Subgroup 16." "Gram-negative Proteobacteria Subgroup 16" is defined as the group of Proteobacteria of the following *Pseudomonas* species (with the ATCC or other deposit numbers of exemplary strain(s) shown in parenthesis): *Pseudomonas abietaniphila* (ATCC 700689); *Pseudomonas aeruginosa* (ATCC 10145); *Pseudomonas alcaligenes* (ATCC 14909); *Pseudomonas anguilliseptica* (ATCC 33660); *Pseudomonas citronellolis* (ATCC 13674); *Pseudomonas flavescens* (ATCC 51555); *Pseudomonas mendocina* (ATCC *25411); Pseudomonas nitroreducens* (ATCC 33634); *Pseudomonas oleovorans* (ATCC 8062); *Pseudomonas pseudoalcaligenes* (ATCC 17440); *Pseudomonas resinovorans* (ATCC 14235); *Pseudomonas straminea* (ATCC 33636); *Pseudomonas agarici* (ATCC 25941); *Pseudomonas alcaliphila; Pseudomonas alginovora; Pseudomonas andersonii; Pseudomonas asplenii* (ATCC 23835); *Pseudomonas azelaica* (ATCC 27162); *Pseudomonas beyerinckii* (ATCC 19372); *Pseudomonas borealis; Pseudomonas boreopolis* (ATCC 33662); *Pseudomonas brassicacearum; Pseudomonas butanovora* (ATCC 43655); *Pseudomonas cellitlosa* (ATCC 55703); *Pseudomonas aurantiaca* (ATCC *33663); Pseudomonas chlororaphis* (ATCC 9446, ATCC 13985, ATCC 17418, ATCC 17461); *Pseudomonas fragi* (ATCC 4973); *Pseudomonas lundensis* (ATCC 49968); *Pseudomonas taetrolens* (ATCC 4683); *Pseudomonas cissicola* (ATCC 33616); *Pseudomonas coronafaciens; Pseudomonas diterpeniphila; Pseudomonas elongata* (ATCC 10144); *Pseudomonasflectens* (ATCC 12775); *Pseudomonas azotoformans; Pseudomonas brenneri; Pseudomonas cedrella; Pseudomonas corrugata* (ATCC 29736); *Pseudomonas extremorientalis; Pseudomonas fluorescens* (ATCC 35858); *Pseudomonas gessardii; Pseudomonas libanensis; Pseudomonas mandelii* (ATCC 700871); *Pseudomonas marginalis* (ATCC 10844); *Pseudomonas migulae; Pseudomonas mucidolens* (ATCC 4685); *Pseudomonas orientalis; Pseudomonas rhodesiae; Pseudomonas synxantha* (ATCC 9890); *Pseudomonas tolaasii* (ATCC 33618); *Pseudomonas veronii* (ATCC 700474); *Pseudomonas frederiksbergensis; Pseudomonas geniculata* (ATCC 19374); *Pseudomonas gingeri; Pseudomonas graminis; Pseudomonas grimontii; Pseudomonas halodenitrificans; Pseudomonas halophila*; *Pseudomonas hibiscicola* (ATCC 19867); *Pseudomonas* huttiensis (ATCC 14670); *Pseudomonas hydrogenovora; Pseudomonas jessenii* (ATCC 700870); *Pseudomonas kilonensis; Pseudomonas lanceolata* (ATCC 14669); *Pseudomonas lini; Pseudomonas marginata* (ATCC 25417); *Pseudomonas mephitica* (ATCC 33665); *Pseudomonas* denitrificans (ATCC 19244); *Pseudomonas pertucinogena* (ATCC 190); *Pseudomonas pictorum* (ATCC *23328); Pseudomonas psychrophila*; *Pseudomonas filva* (ATCC 31418); *Pseudomonas monteilii* (ATCC *700476); Pseudomonas mosselii; Pseudomonas oryzihabitans* (ATCC 43272); *Pseudomonas* plecoglossicida (ATCC 700383); *Pseudomonas putida* (ATCC 12633); *Pseudomonas* reactans; *Pseudomonas spinosa* (ATCC 14606); *Pseudomonas balearica; Pseudomonas luteola* (ATCC 43273);. *Pseudomonas stutzeri* (ATCC 17588); *Pseudomonas amygdali* (ATCC 33614); *Pseudomonas avellanae* (ATCC 700331); *Pseudomonas* caricapapayae (ATCC 33615); *Pseudomonas* cichorii (ATCC 10857); *Pseudomonas ficuserectae* (ATCC 35104); *Pseudomonas fuscovaginae, Pseudomonas meliae* (ATCC *33050); Pseudomonas syringae* (ATCC 19310); *Pseudomonas* viridiflava (ATCC 13223); *Pseudomonas thermocarboxydovorans* (ATCC 35961); *Pseudomonas thermotolerans; Pseudomonas thivervalensis; Pseudomonas vancouverensis* (ATCC 700688); *Pseudomonas wisconsinensis;* and *Pseudomonas xiamenensis.* In one embodiment, the host cell for expression of the polypeptide or protein of interest is *Pseudomonas fluorescens.*

The host cell, in some cases, is selected from "Gram-negative Proteobacteria Subgroup 17." "Gram-negative Proteobacteria Subgroup 17" is defined as the group of Proteobacteria known in the art as the "fluorescent Pseudomonads" including those belonging, e.g., to the following *Pseudomonas* species: *Pseudomonas azotoformans*; *Pseudomonas brenneri; Pseudomonas cedrella*; *Pseudomonas cedrina; Pseudomonas cornigata; Pseudomonas* extremorientalis; *Pseudomonas fluorescens; Pseudomonas gessardii; Pseudomonas* libanensis; *Pseudomonas mandelii; Pseudomonas marginalis; Pseudomonas migulae; Pseudomonas* mucidolens; *Pseudomonas orientalis; Pseudomonas rhodesiae; Pseudomonas synxantha; Pseudomonas tolaasii;* and *Pseudomonas veronii.*

Proteases

In one embodiment, the methods provided herein comprise using a *Pseudomonas* host cell, comprising one or more mutations (e.g., a partial or complete deletion) in one or more protease genes, to produce polypeptides or proteins of interest. In some embodiments, a mutation in a protease gene facilitates generation of soluble polypeptides or proteins of interest.

Exemplary target protease genes include those proteases classified as Aminopeptidases; Dipeptidases; Dipeptidyl-peptidases and tripeptidyl peptidases; Peptidyl-dipeptidases; Serine-type carboxypeptidases; Metallocarboxypeptidases; Cysteine-type carboxypeptidases; Omegapeptidases; Serine proteinases; Cysteine proteinases; Aspartic proteinases; Metallo proteinases; or Proteinases of unknown mechanism.

Aminopeptidases include cytosol aminopeptidase (leucyl aminopeptidase), membrane alanyl aminopeptidase, cystinyl aminopeptidase, tripeptide aminopeptidase, prolyl aminopeptidase, arginyl aminopeptidase, glutamyl aminopeptidase, x-pro aminopeptidase, bacterial leucyl aminopeptidase, thermophilic aminopeptidase, clostridial aminopeptidase, cytosol alanyl aminopeptidase, lysyl aminopeptidase, x-trp aminopeptidase, tryptophanyl aminopeptidase, methionyl aminopeptidas, d-stereospecific aminopeptidase, aminopeptidase ey. Dipeptidases include x-his dipeptidase, x-arg dipeptidase, x-methyl-his dipeptidase, cys-gly dipeptidase, glu-glu dipeptidase, pro-x dipeptidase, x-pro dipeptidase, met-x dipeptidase, non-stereospecific dipeptidase, cytosol non-specific dipeptidase, membrane dipeptidase, beta-ala-his dipeptidase. Dipeptidyl-peptidases and tripeptidyl peptidases include dipeptidyl-peptidase i, dipeptidyl-peptidase ii, dipeptidyl peptidase iii, dipeptidyl-peptidase iv, dipeptidyl-dipeptidase, tripeptidyl-peptidase I, tripeptidyl-peptidase II. Peptidyl-dipeptidases include peptidyl-dipeptidase a and peptidyl-dipeptidase b. Serine-type carboxypeptidases include lysosomal pro-x carboxypeptidase, serine-type D-ala-D-ala carboxypeptidase, carboxypeptidase C, carboxypeptidase D. Metallocarboxypeptidases include carboxypeptidase a, carboxypeptidase B, lysine(arginine) carboxypeptidase, gly-X carboxypeptidase, alanine carboxypeptidase, muramoylpentapeptide carboxypeptidase, carboxypeptidase h, glutamate carboxypeptidase, carboxypeptidase M, muramoyltetrapeptide carboxypeptidase, zinc d-ala-d-ala carboxypeptidase, carboxypeptidase A2, membrane pro-x carboxypeptidase, tubulinyl-tyr carboxypeptidase, carboxypeptidase t. Omegapeptidases include acylaminoacyl-peptidase, peptidyl-glycinamidase, pyroglutamyl-peptidase I, beta-aspartyl-peptidase, pyroglutamyl-peptidase II, n-fonnylmethionyl-peptidase, pteroylpoly-[gamma]-glutamate carboxypeptidase, gamma-glu-X carboxypeptidase, acylmuramoyl-ala peptidase. Serine proteinases include chymotrypsin, chymotrypsin c, metridin, trypsin, thrombin, coagulation factor Xa, plasmin, enteropeptidase, acrosin, alpha-lytic protease, glutamyl, endopeptidase, cathepsin G, coagulation factor viia, coagulation factor ixa, cucumisi, prolyl oligopeptidase, coagulation factor xia, brachyurin, plasma kallikrein, tissue kallikrein, pancreatic elastase, leukocyte elastase, coagulation factor xiia, chymase, complement component c1r55, complement component cls55, classical-complement pathway c3/c5 convertase, complement factor I, complement factor D, alternative-complement pathway c3/c5 convertase, cerevisin, hypodermin C, lysyl endopeptidase, endopeptidase 1a, gamma-reni, venombin ab, leucyl endopeptidase, tryptase, scutelarin, kexin, subtilisin, oryzin, endopeptidase k, thermomycolin, thermitase, endopeptidase SO, T-plasminogen activator, protein C, pancreatic endopeptidase E, pancreatic elastase ii, IGA-specific serine endopeptidase, U-plasminogen, activator, venombin A, furin, myeloblastin, semenogelase, granzyme A or cytotoxic T-lymphocyte proteinase 1, granzyme B or cytotoxic T-lymphocyte proteinase 2, streptogrisin A, treptogrisin B, glutamyl endopeptidase II, oligopeptidase B, limulus clotting factor c, limulus clotting factor, limulus clotting enzyme, omptin, repressor lexa, bacterial leader peptidase I, togavirin, flavirin. Cysteine proteinases include cathepsin B, papain, ficin, chymopapain, asclepain, clostripain, streptopain, actinide, cathepsin 1, cathepsin H, calpain, cathepsin t, glycyl, endopeptidase, cancer procoagulant, cathepsin S, picornain 3C, picomain 2A, caricain, ananain, stem bromelain, fruit bromelain, legumain, histolysain, interleukin 1-beta converting enzyme. Aspartic proteinases include pepsin A, pepsin B, gastricsin, chymosin, cathepsin D, neopenthesin, renin, retropepsin, pro-opiomelanocortin converting enzyme, aspergillopepsin I, aspergillopepsin II, penicillopepsin, rhizopuspepsin, endothiapepsin, mucoropepsin, candidapepsin, saccharopepsin, rhodotorulapepsin, physaropepsin, acrocylindropepsin, polyporopepsin, pycnoporopepsin, scytalidopepsin a, scytalidopepsin b, xanthomonapepsin, cathepsin e, barrierpepsin, bacterial leader peptidase I, pseudomonapepsin, plasmepsin. Metallo proteinases include atrolysin a, microbial collagenase, leucolysin, interstitial collagenase, neprilysin, envelysin, iga-specific metalloendopeptidase, procollagen N-endopeptidase, thimet oligopeptidase, neurolysin, stromelysin 1, meprin A, procollagen C-endopeptidase, peptidyl-lys metalloendopeptidase, astacin, stromelysin, 2, matrilysin gelatinase, aeromonolysin, pseudolysin, thermolysin, bacillolysin, aureolysin, coccolysin, mycolysin, beta-lytic metalloendopeptidase, peptidyl-asp metalloendopeptidase, neutrophil collagenase, gelatinase B, leishmanolysin, saccharolysin, autolysin, deuterolysin, serralysin, atrolysin B, atrolysin C, atroxase, atrolysin E, atrolysin F, adamalysin, horrilysin, ruberlysin, bothropasin, bothrolysin, ophiolysin, trimerelysin I, trimerelysin II, mucrolysin, pitrilysin, insulysin, O-syaloglycoprotein endopeptidase, russellysin, mitochondrial, intermediate, peptidase, dactylysin, nardilysin, magnolysin, meprin B, mitochondrial processing peptidase, macrophage elastase, choriolysin, toxilysin. Proteinases of unknown mechanism include thermopsin and multicatalytic endopeptidase complex.

Certain proteases have both protease and chaperone-like activity. When these proteases are negatively affecting protein yield and/or quality it is often useful to specifically delete their protease activity, and they are overexpressed when their chaperone activity may positively affect protein yield and/or quality. These proteases include, but are not limited to: Hsp100(Clp/Hsl) family members RXF04587.1 (clpA), RXF08347.1, RXF04654.2 (clpX), RXF04663.1, RXF01957.2 (hslU), RXF01961.2 (hslV); Peptidyl-prolyl cis-trans isomerase family member RXF05345.2 (ppiB); Metallopeptidase M20 family member RXF04892.1 (aminohydrolase); Metallopeptidase M24 family members RXF04693.1 (methionine aminopeptidase) and RXF03364.1 (methionine aminopeptidase); and Serine Peptidase S26 signal peptidase I family member RXF01181.1 (signal peptidase).

These and other proteases and folding modulators are known in the art and described in the literature, e.g., in U.S. Pat. No. 8,603,824. For example, Table D of the patent describes Tig (tig, Trigger factor, FKBP type ppiase (ec 5.2.1.8) RXF04655, UniProtKB - P0A850 (TIG_ECOLI)). WO 2008/134461 and U.S. Pat. No. 9,394,571, titled "Method for Rapidly Screening Microbial Hosts to Identify Certain Strains with Improved Yield and/or Quality in the Expression of Heterologous Proteins," and incorporated by reference in its entirety herein, describe Tig (RXF04655.2, SEQ ID NO: 34 therein), LepB (RXF01181.1, SEQ ID NO: 56 therein), DegP1 (RXF01250, SEQ ID NO: 57 therein), AprA (RXF04304.1, SEQ ID NO: 86 therein), Prc1 (RXF06586.1, SEQ ID NO: 120 therein), DegP2, (RXF07210.1, SEQ ID NO: 124 therein), Lon (RXF04653, SEQ ID NO: 92 therein); DsbA (RXF01002.1, SEQ ID NO: 25 therein), and DsbC (RXF03307.1, SEQ ID NO: 26 therein). These sequences and those for other proteases and folding modulators also are set forth in U.S. Pat. No. 9,580,719 (Table of SEQ ID NOS in columns 93-98 therein). For example, U.S. Pat. No. 9,580,719 provides the sequence encoding HslU (RXF01957.2) and HslV (RXF01961.2) as SEQ ID NOS 18 and 19, respectively.

## High Throughput Screens

In embodiments, a high throughput screen is conducted to determine optimal conditions for expressing a recombinant protein or polypeptide of interest. Conditions that can be varied in the screen include, for example, the host cell, genetic background of the host cell (e.g., deletions of different proteases), type of promoter in an expression construct, type of secretion leader fused to the encoded polypeptide or protein of interest, temperature of growth, OD of induction when an inducible promoter is used, amount of inducer added (e.g. amount of IPTG used for induction when a lacZ promoter or derivative thereof is used), duration of protein induction, temperature of growth following addition of an inducing agent to a culture, rate of agitation of culture, method of selection for plasmid maintenance, volume of culture in a vessel, and method of cell lysing.

In some embodiments, a library (or "array") of host strains is provided, wherein each strain (or "population of host cells") in the library has been genetically modified to modulate the expression of one or more target genes in the host cell. An "optimal host strain" or "optimal expression system" is often identified or selected based on the quantity, quality, and/or location of the expressed protein of interest compared to other populations of phenotypically distinct host cells in the array. Thus, an optimal host strain is the strain that produces the polypeptide of interest according to a desired specification. While the desired specification will vary depending on the polypeptide being produced, the specification includes the quality and/or quantity of protein, whether the protein is sequestered or secreted, protein folding, and the like. For example, the optimal host strain or optimal expression system produces a yield, characterized by the amount of soluble heterologous protein, the amount of recoverable heterologous protein, the amount of properly processed heterologous protein, the amount of properly folded heterologous protein, the amount of active heterologous protein, and/or the total amount of heterologous protein, of a certain absolute level or a certain level relative to that produced by an indicator strain, i.e., a strain used for comparison.

Methods of screening microbial hosts to identify strains with improved yield and/or quality in the expression of heterologous proteins are described, for example, in U.S. Patent Application Publication No. 20080269070.

## Bacterial growth conditions

Growth conditions useful in the methods herein often comprise a temperature of about 4 °C to about 42 °C and a pH of about 5.7 to about 8.8. When an expression construct with a lacZ promoter or derivative thereof is used, expression is often induced by adding IPTG to a culture at a final concentration of about 0.01 mM to about 1.0 mM.

The pH of the culture is sometimes maintained using pH buffers and methods known to those of skill in the art. Control of pH during culturing also is often achieved using aqueous ammonia. In embodiments, the pH of the culture is about 5.7 to about 8.8. In certain embodiments, the pH is about 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 83, 8.4, 8.5, 8.6, 8.7, or 8.8 In other embodiments, the pH is about 5.7 to 5.9, 5.8 to 6.0, 5.9 to 6.1, 6.0 to 6.2, 6.1 to 6.3, 6.2 to 6.5, 6.4 to 6.7, 6.5 to 6.8, 6.6 to 6.9, 6.7 to 7.0, 6.8 to 7.1, 6.9 to 7.2, 7.0 to 7.3, 7.1 to 7.4, 7.2 to 7.5, 7.3 to 7.6, 7.4 to 7.7, 7.5 to 7.8, 7.6 to 7.9, 7.7 to 8.0, 7.8 to 8.1, 7.9 to 8.2, 8.0 to 8.3, 8.1 to 8.4, 8.2 to 8.5, 8.3 to 8.6, 8.4 to 8.7, or 8.5 to 8.8. In yet other embodiments, the pH is about 5.7 to 6.0, 5.8 to 6.1, 5.9 to 6.2, 6.0 to 6.3, 6.1 to 6.4, or 6.2 to 6.5. In certain embodiments, the pH is about 5.7 to about 6.25.

In embodiments, the growth temperature is maintained at about 4 °C to about 42 °C. In certain embodiments, the growth temperature is about 4 °C, about 5 °C, about 6 °C, about 7 °C, about 8 °C, about 9 °C, about 10 °C, about 11 °C, about 12 °C, about 13 °C, about 14 °C, about 15 °C, about 16 °C, about 17 °C, about 18 °C, about 19 °C, about 20 °C, about 21 °C, about 22 °C, about 23 °C, about 24 °C, about 25 °C, about 26 °C, about 27 °C, about 28 °C, about 29 °C, about 30 °C, about 31 °C, about 32 °C, about 33 °C, about 34 °C, about 35 °C, about 36°C, about 37 °C, about 38 °C, about 39 °C, about 40 °C, about 41 °C, or about 42 °C. In other embodiments, the growth temperature is maintained at about 25 °C to about 27 °C, about 25 °C to about 28 °C, about 25 °C to about 29 °C, about 25 °C to about 30 °C, about 25 °C to about 31 °C, about 25 °C to about 32 °C, about 25 °C to about 33 °C, about 26 °C to about 28 °C, about 26 °C to about 29 °C, about 26 °C to about 30 °C, about 26 °C to about 31 °C, about 26 °C to about 32 °C, about 27 °C to about 29 °C, about 27 °C to about 30 °C, about 27 °C to about 31 °C, about 27 °C to about 32 °C, about 26 °C to about 33 °C, about 28 °C to about 30 °C, about 28 °C to about 31 °C, about 28 °C to about 32 °C, about 29 °C to about 31 °C, about 29 °C to about 32 °C, about 29 °C to about 33 °C, about 30 °C to about 32 °C, about 30 °C to about 33 °C, about 31 °C to about 33 °C, about 31 °C to about 32 °C, about 30 °C to about 33 °C, or about 32 °C to about 33 °C. In other embodiments, the temperature is changed during culturing. In certain embodiments, the temperature is maintained at about 30 °C to about 32 °C before an agent to induce expression from the construct encoding the polypeptide or protein of interest is added to the culture, and the temperature is dropped to about 25 °C to about 27 °C after adding an agent to induce expression, e.g., IPTG is added to the culture. In one embodiment, the temperature is maintained at about 30 °C before an agent to induce expression from the construct encoding the polypeptide or protein of interest is added to the culture, and the temperature is dropped to about 25 °C after adding an agent to induce expression is added to the culture.

Induction

As described elsewhere herein, inducible promoters are often used in the expression construct to control expression of the polypeptide or protein of interest, e.g., a lac promoter. In the case of the lac promoter derivatives or family members, e.g., the tac promoter, the effector compound is an inducer, such as a gratuitous inducer like IPTG (isopropyl-$\beta$-D-1-thiogalactopyranoside, also called "isopropylthiogalactoside"). In embodiments, a lac promoter derivative is used, and the polypeptide or protein of interest expression is induced by the addition of IPTG to a final concentration of about 0.01 mM to about 1.0 mM, when the cell density has reached a level identified by an OD575 of about 25 to about 160. In embodiments, the OD575 at the time of culture induction for the polypeptide or protein of interest is about 25, about 50, about 55, about 60, about 65, about 70, about 80, about 90, about 100, about 110, about 120, about 130, about 140, about 150, about 160, about 170 about 180. In other embodiments, the OD575 is about 80 to about 100, about 100 to about 120, about 120 to about 140, about 140 to about 160. In other embodiments, the OD575 is about 80 to about 120, about 100 to about 140, or about 120 to about 160. In other embodiments, the OD575 is about 80 to about 140, or about 100 to 160. The cell density is often measured by other methods and expressed in other units, e.g., in cells per unit volume. For example, an OD575 of about 25 to about 160 of a *Pseudomonas fluorescens* culture is equivalent to approximately $4 \times 10^{10}$ to about $1.6 \times 10^{11}$ colony forming units per mL or 11 to 70 g/L dry cell weight. In embodiments, expression of the polypeptide or protein of interest is induced by the addition of IPTG to a final concentration of about 0.01 mM to about 1.0 mM, when the cell density has reached a wet weight of about 0.05 g/g to about 0.4 g/g. In embodiments the wet weight is about 0.05 g/g, about 0.1 g/g, about 0.15 g/g, about 0.2 g/g, about 0.25 g/g, about 0.30 g/g, about 0.35 g/g, about 0.40 g/g, about 0.05 g/g to about 0.1 g/g, about 0.05 g/g to about 0.15 g/g, about 0.05 g/g to about 0.20 g/g, about 0.05 g/g to about 0.25 g/g, about 0.05 g/g to about 0.30 g/g, about 0.05 g/g to about 0.35 g/g, about 0.1 g/g to about 0.40 g/g, about 0.15 g/g to about 0.40 g/g, about 0.20 g/g to about 0.40 g/g, about 0.25 g/g to about 0.40 g/g, about 0.30 g/g to about 0.40 g/g, or about 0.35 g/g to about 0.40 g/g. In embodiments, the cell density at the time of culture induction is equivalent to the cell density as specified herein by the absorbance at OD575, regardless of the method used for determining cell density or the units of measurement. One of skill in the art will know how to make the appropriate conversion for any cell culture.

In embodiments, the final IPTG concentration of the culture is about 0.01 mM, about 0.02 mM, about 0.03 mM, about 0.04 mM, about 0.05 mM, about 0.06 mM, about 0.07 mM, about 0.08 mM, about 0.09 mM, about 0.1 mM, about 0.2 mM, about 0.3 mM, about 0.4 mM, about 0.5 mM, about 0.6 mM, about 0.7 mM, about 0.8 mM, about 0.9 mM, or about 1 mM. In other embodiments, the final IPTG concentration of the culture is about 0.08 mM to about 0.1 mM, about 0.1 mM to about 0.2 mM, about 0.2 mM to about 0.3 mM, about 0.3 mM to about 0.4 mM, about 0.2 mM to about 0.4 mM, about 0.08 to about 0.2mM, or about 0.1 to 1 mM.

In embodiments wherein a non-lac type promoter is used, as described herein and in the literature, other inducers or effectors are often used. In one embodiment, the promoter is a constitutive promoter.

After adding and inducing agent, cultures are often grown for a period of time, for example about 24 hours, during which time the polypeptide or protein of interest is expressed. After adding an inducing agent, a culture is often grown for about 1 hr, about 2 hr, about 3 hr, about 4 hr, about 5 hr, about 6 hr, about 7 hr, about 8 hr, about 9 hr, about 10 hr, about 11 hr, about 12 hr, about 13 hr, about 14 hr, about 15 hr, about 16 hr, about 17 hr, about 18 hr, about 19 hr, about 20 hr, about 21 hr, about 22 hr, about 23 hr, about 24 hr, about 36 hr, or about 48 hr. After an inducing agent is added to a culture, the culture is grown for about 1 to 48 hrs, about 1 to 24 hrs, about 10 to 24 hrs, about 15 to 24 hrs, or about 20 to 24 hrs. Cell cultures are often concentrated by centrifugation, and the culture pellet resuspended in a buffer or solution appropriate for the subsequent lysis procedure.

In embodiments, cells are disrupted using equipment for high pressure mechanical cell disruption (which are available commercially, e.g., Microfluidics Microfluidizer, Constant Cell Disruptor, Niro-Soavi homogenizer or APV-Gaulin homogenizer). Cells expressing polypeptides or proteins of interest are often disrupted, for example, using sonication. Any appropriate method known in the art for lysing cells are often used to release the soluble fraction. For example, in embodiments, chemical and/or enzymatic cell lysis reagents, such as cell-wall lytic enzyme and EDTA, are often used. Use of frozen or previously stored cultures is also contemplated in the methods herein. Cultures are sometimes OD-normalized prior to lysis. For example, cells are often normalized to an OD600 of about 10, about 11, about 12, about 13, about 14, about 15, about 16, about 17, about 18, about 19, or about 20.

Centrifugation is performed using any appropriate equipment and method. Centrifugation of cell culture or lysate for the purposes of separating a soluble fraction from an insoluble fraction is well-known in the art. For example, lysed cells are sometimes centrifuged at 20,800 $\times$ g for 20 minutes (at 4° C), and the supernatants removed using manual or automated liquid handling. The pellet (insoluble) fraction is resuspended in a buffered solution, e.g., phosphate buffered saline (PBS), pH 7.4. Resuspension is often carried out using, e.g., equipment such as impellers connected to an overhead mixer, magnetic stir-bars, rocking shakers, etc.

A "soluble fraction," i.e., the soluble supernatant obtained after centrifugation of a lysate, and an "insoluble fraction," i.e., the pellet obtained after centrifugation of a lysate, result from lysing and centrifuging the cultures.

Fermentation Format

In one embodiment, fermentation is used in the methods of producing polypeptides and proteins of interest herein. The expression system according to the present disclosure is cultured in any fermentation format. For example, batch, fed-batch, semi-continuous, and continuous fermentation modes may be employed herein.

In embodiments, the fermentation medium may be selected from among rich media, minimal media, and mineral salts media. In other embodiments either a minimal medium or a mineral salts medium is selected. In certain embodiments, a mineral salts medium is selected.

Mineral salts media consists of mineral salts and a carbon source such as, e.g., glucose, sucrose, or glycerol. Examples of mineral salts media include, e.g., M9 medium, *Pseudomonas* medium (ATCC 179), and Davis and Mingioli medium (see, B D Davis & E S Mingioli (1950) J. Bact. 60:17-28). The mineral salts used to make mineral salts media include those selected from among, e.g., potassium phosphates, ammonium sulfate or chloride, magnesium sulfate or chloride, and trace minerals such as calcium chloride, borate, and sulfates of iron, copper, manganese, and zinc. Typically, no organic nitrogen source, such as peptone, tryptone, amino acids, or a yeast extract, is included in a mineral salts medium. Instead, an inorganic nitrogen source is used and this may be selected from among, e.g., ammonium salts, aqueous ammonia, and gaseous ammonia. A mineral salts medium will typically contain glucose or glycerol as the carbon source. In comparison to mineral salts media, minimal media often contains mineral salts and a carbon source, but is often supplemented with, e.g., low levels of amino acids, vitamins, peptones, or other ingredients, though these are added at very minimal levels. Media is often prepared using the methods described in the art, e.g., in U.S. Pat. App. Pub. No. 2006/0040352, referenced and incorporated by reference above. Details of cultivation procedures and mineral salts media useful in the methods herein are described by Riesenberg, D. et al., 1991, "High cell density cultivation of Escherichia coli at controlled specific growth rate," J. Biotechnol. 20 (1):17-27.

Fermentation may be performed at any scale. The expression systems according to the present disclosure are useful for recombinant protein expression at any scale. Thus, e.g., microliter-scale, milliliter scale, centiliter scale, and deciliter scale fermentation volumes may be used, and 1 Liter scale and larger fermentation volumes are often used.

In embodiments, the fermentation volume is at or above about 1 Liter. In embodiments, the fermentation volume is about 0.5 liters to about 100 liters. In embodiments, the fermentation volume is about 1 liter, about 2 liters, about 3 liters, about 4 liters, about 5 liters, about 6 liters, about 7 liters, about 8 liters, about 9 liters, or about 10 liters. In embodiments, the fermentation volume is about 0.5 liters to about 2 liters, about 0.5 liters to about 5 liters, about 0.5 liters to about 10 liters, about 0.5 liters to about 25 liters, about 0.5 liters to about 50 liters, about 0.5 liters to about 75 liters, about 10 liters to about 25 liters, about 25 liters to about 50 liters, or about 50 liters to about 100 liters In other embodiments, the fermentation volume is at or above 5 Liters, 10 Liters, 15 Liters, 20 Liters, 25 Liters, 50 Liters, 75 Liters, 100 Liters, 200 Liters, 500 Liters, 1,000 Liters, 2,000 Liters, 5,000 Liters, 10,000 Liters, or 50,000 Liters.

**Protein Analysis**

In embodiments, polypeptides and proteins of interest produced by the methods provided herein are analyzed. Polypeptides and proteins of interest can be analyzed by any appropriate method known to those of skill in the art, for example, by biolayer interferometry, SDS-PAGE, Western blot, Far Western blot, ELISA, absorbance, or mass spectrometry (e.g., tandem mass spectrometry).

In some embodiments, the concentration and/or amounts of polypeptides or proteins of interest generated are determined, for example, by Bradford assay, absorbance, Coomassie staining, mass spectrometry, etc.

Protein yield in the insoluble and soluble fractions as described herein are often determined by methods known to those of skill in the art, for example, by capillary gel electrophoresis (CGE), SDS-PAGE, and Western blot analysis. Soluble fractions are often evaluated, for example, using biolayer interferometry.

Useful measures of protein yield include, e.g., the amount of recombinant protein per culture volume (e.g., grams or milligrams of protein/liter of culture), percent or fraction of recombinant protein measured in the insoluble pellet obtained after lysis (e.g., amount of recombinant protein in extract supernatant/amount of protein in insoluble fraction), percent or fraction of active protein (e.g., amount of active protein/amount protein used in the assay), percent or fraction of total cell protein (tcp), amount of protein/cell, and percent dry biomass.

In embodiments, the methods herein are used to obtain a yield of soluble polypeptide or protein of interest of about 20% to about 90% total cell protein. In certain embodiments, the yield of soluble polypeptide or protein of interest is about 20% total cell protein, about 25% total cell protein, about 30% total cell protein, about 31% total cell protein, about 32% total cell protein, about 33% total cell protein, about 34% total cell protein, about 35% total cell protein, about 36% total cell protein, about 37% total cell protein, about 38% total cell protein, about 39% total cell protein, about 40% total cell protein, about 41% total cell protein, about 42% total cell protein, about 43% total cell protein, about 44% total cell protein, about 45% total cell protein, about 46% total cell protein, about 47% total cell protein, about 48% total cell protein, about 49% total cell protein, about 50% total cell protein, about 51% total cell protein, about 52% total cell protein, about 53% total cell protein, about 54% total cell protein, about 55% total cell protein, about 56% total cell protein, about 57% total cell protein, about 58% total cell protein, about 59% total cell protein, about 60% total cell protein, about 65% total cell protein, about 70% total cell protein, about 75% total cell protein, about 80% total cell protein, about 85% total cell protein, or about 90% total cell protein. In some embodiments, the yield of soluble polypeptide or protein of interest is about 20% to about 25% total cell protein, about 20% to about 30% total cell protein, about 20% to about 35% total cell protein, about 20% to about 40% total cell protein, about 20% to about 45% total cell protein, about 20% to about 50% total cell protein, about 20% to about 55% total cell protein, about 20% to about 60% total cell protein, about 20% to about 65% total cell protein, about 20% to about 70% total cell protein, about 20% to about 75% total cell protein, about 20% to about 80% total cell protein, about 20% to about 85% total cell protein, about 20% to about 90% total cell protein, about 25% to about 90% total cell protein, about 30% to about 90% total cell protein, about 35% to about 90% total cell protein, about 40% to about 90% total cell protein, about 45% to about 90% total cell protein, about 50% to about 90% total cell protein, about 55% to about 90% total cell protein, about 60% to about 90% total cell protein, about 65% to about 90% total cell protein, about 70% to about 90% total cell protein, about 75% to about 90% total cell protein, about 80% to about 90% total cell protein, about 85% to about 90% total cell protein, about 31% to about 60% total cell protein, about 35% to about 60% total cell protein, about 40% to about 60% total cell protein, about 45% to about 60% total cell protein, about 50% to about 60% total cell protein, about 55% to about 60% total cell protein, about 31% to about 55% total cell protein, about 31% to about 50% total cell protein, about 31% to about 45% total cell protein, about 31% to about 40% total cell protein, about 31% to about 35% total cell protein, about 35% to about 55% total cell protein, or about 40% to about 50% total cell protein.

In embodiments, the methods herein are used to obtain a yield of soluble polypeptide or protein of interest of about 1 gram per liter to about 50 grams per liter. In certain embodiments, the yield of soluble polypeptide or protein of interest is about 1 gram per liter, about 2 grams per liter, about 3 grams per liter, about 4 grams per liter, about 5 grams per liter, about 6 grams per liter, about 7 grams per liter, about 8 grams per liter, about 9 grams per liter, about 10 gram per liter, about 11 grams per liter, about 12 grams per liter, about 13 grams per liter, about 14 grams per liter, about 15 grams per liter, about 16 grams per liter, about 17 grams per liter, about 18 grams per liter, about 19 grams per liter, about 20 grams per liter, about 21 grams per liter, about 22 grams per liter, about 23 grams per liter about 24 grams per liter, about 25 grams per liter, about 26 grams per liter, about 27 grams per liter, about 28 grams per liter, about 30 grams per liter, about 35 grams per liter, about 40 grams per liter, about 45 grams per liter, about 50 grams per liter, about 1 gram per liter to about 5 grams per liter, about 1 gram to about 10 grams per liter, about 10 gram per liter to about 12 grams per liter, about 10 grams per liter to about 13 grams per liter, about 10 grams per liter to about 14 grams per liter, about 10 grams per liter to about 15 grams per liter, about 10 grams per liter to about 16 grams per liter, about 10 grams per liter to about 17 grams per liter, about 10 grams per liter to about 18 grams per liter, about 10 grams per liter to about 19 grams per liter, about 10 grams per liter to about 20 grams per liter, about 10 grams per liter to about 21 grams per liter, about 10 grams per liter to about 22 grams per liter, about 10 grams per liter to about 23 grams per liter, about 10 grams per liter to about 24 grams per liter, about 10 grams per liter to about 25 grams per liter, about 10 grams per liter to about 30 grams per liter, about 10 grams per liter to about 40 grams per liter, about 10 grams per liter to about 50 grams per liter, about 10 gram per liter to about 12 grams per liter, about 12 grams per liter to about 14 grams per liter, about 14 grams per liter to about 16 grams per liter, about 16 grams per liter to about 18 grams per liter, about 18 grams per liter to about 20 grams per liter, about 20 grams per liter to about 22 grams per liter, about 22 grams per liter to about 24 grams per liter, about 23 grams per liter to about 25 grams per liter, about 10 grams per liter to about 25 grams per liter, about 11 grams per liter to about 25 grams per liter, about 12 grams per liter to about 25 grams per liter, about 13 grams per liter to about 25 grams per liter, about 14 grams per liter to about 25 grams per liter, about 15 grams per liter to about 25 grams per liter, about 16 grams per liter to about 25 grams per liter, about 17 grams per liter to about 25 grams per liter, about 18 grams per liter to about 25 grams per liter, about 19 grams per liter to about 25 grams per liter, about 20 grams per liter to about 25 grams per liter, about 21 grams per liter to about 25 grams per liter, about 22 grams per liter to about 25 grams per liter, about 23 grams per liter to about 25 grams per liter, or about 24 grams per liter to about 25 grams per liter. In embodiments, the soluble recombinant protein yield is about 10 gram per liter to about 13 grams per liter, about 12 grams per liter to about 14 grams per liter, about 13 grams per liter to about 15 grams per liter, about 14 grams per liter to about 16 grams per liter, about 15 grams per liter to about 17 grams per liter, about 16 grams per liter to about 18 grams per liter, about 17 grams per liter to about 19 grams per liter, about 18 grams per liter to about 20 grams per liter, about 20 grams per liter to about 22 grams per liter, about 22 grams per liter to about 24 grams per liter, or about 23 grams per liter to about 25 grams per liter. In embodiments, the soluble recombinant protein yield is about 10 grams per liter to about 25 grams per liter, about 12 gram per liter to about 24 grams per liter, about 14 grams per liter to about 22 grams per liter, about 16 grams per liter to about 20 grams per liter, or about 18 grams per liter to about 20 grams per liter. In embodiments, the extracted protein yield is about 5 grams per liter to about 15 grams per liter, about 5 gram per liter to about 25 grams per liter, about 10 grams per liter to about 15 grams per liter, about 10 grams per liter to about 25 grams per liter, about 15 grams per liter to about 20 grams per liter, about 15 grams per liter to about 25 grams per liter, or about 18 grams per liter to about 25 grams per liter.

In embodiments, the amount of soluble polypeptide or protein of interest detected in the soluble fraction is about 10% to about 100% of the amount of the total soluble polypeptide or protein of interest produced. In embodiments, this amount is about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 99%, or about 100% of the amount of the soluble polypeptide or protein of interest produced. In embodiments, this amount is about 10% to about 20%, 20% to about 50%, about 25% to about 50%, about 25% to about 50%, about 25% to about 95%, about 30% to about 50%, about 30% to about 40%, about 30% to about 60%, about 30% to about 70%, about 35% to about 50%, about 35% to about 70%, about 35% to about 75%, about 35% to about 95%, about 40% to about 50%, about 40% to about 95%, about 50% to about 75%, about 50% to about 95%, about 70% to about 95%, or about 80 to about 100% of the amount of the soluble polypeptide or protein of interest produced.

In some embodiments, the amount of soluble polypeptide or protein of interest is expressed as a percentage of the total soluble protein produced in a culture. Data expressed in terms of soluble polypeptide or protein of interest weight/volume of cell culture at a given cell density can be converted to data expressed as percent recombinant protein of total cell protein. It is within the capabilities of a skilled artisan to convert volumetric protein yield to % total cell protein, for example, knowing the amount of total cell protein per volume of cell culture at the given cell density. This number can be determined if one knows 1) the cell weight/volume of culture at the given cell density, and 2) the percent of cell weight comprised by total protein. For example, at an OD550 of 1.0, the dry cell weight of *E. coli* is reported to be 0.5 grams/liter ("Production of Heterologous Proteins from Recombinant DNA Escherichia coli in Bench Fermentors," Lin, N.S., and Swartz, J.R., 1992, METHODS: A Companion to Methods in Enzymology 4: 159-168). A bacterial cell is comprised of polysaccharides, lipids, and nucleic acids, as well as proteins. An *E. coli* cell is reported to be about 52.4 to 55% protein by references including, but not limited to, Da Silva, N.A., et al., 1986, "Theoretical Growth Yield Estimates for Recombinant Cells," Biotechnology and Bioengineering, Vol. XXVIII: 741-746 , estimating protein to make up 52.4% by weight of *E. coli* cells, and "Escherichia coli and Salmonella typhimurium Cellular and Molecular Biology," 1987, Ed. in Chief Frederick C. Neidhardt, Vol. 1, pp. 3-6, reporting protein content in *E. coli* as 55% dry cell weight. Using the measurements above (i.e., a dry cell weight of 0.5 grams/liter, and protein as 55% cell weight), the amount of total cell protein per volume of cell culture at an A550 of 1.0 for *E. coli* is calculated as 275 $\mu$g total cell protein/ml/A550. A calculation of total cell protein per volume of cell culture based on wet cell weight can use, e.g.,the determination by Glazyrina, et al. (Microbial Cell Factories 2010, 9:42, incorporated herein by reference) that an A600 of 1.0 for *E. coli* resulted in a wet cell weight of 1.7 grams/liter and a dry cell weight of 0.39 grams/liter. For example, using this wet cell weight to dry cell weight comparison, and protein as 55% dry cell weight as described above, the amount of total cell protein per volume of cell culture at an A600 of 1.0 for *E. coli* can be calculated as 215 $\mu$g total cell protein/ml/A600. For *Pseudomonas fluorescens,* the amount of total cell protein per volume of cell culture at a given cell density is similar to that found for *E. coli. P. fluorescens,* like *E. coli,* is a gram-negative, rod-shaped bacterium. The dry cell weight of *P. fluorescens* ATCC 11150 as reported by Edwards, et al., 1972, "Continuous Culture of Pseudomonas fluorescens with Sodium Maleate as a Carbon Source," Biotechnology and Bioengineering, Vol. XIV, pages 123-147, is 0.5 grams/liter/A500. This is the same weight reported by Lin, et al., for *E. coli* at an A550 of 1.0. Light scattering measurements made at 500nm and at 550nm are expected to be very similar. The percent of cell weight comprised by total cell protein for *P. fluorescens* HK44 is described as 55% by, e.g., Yarwood, et al., July 2002, "Noninvasive Quantitative Measurement of Bacterial Growth in Porous Media under Unsaturated-Flow Conditions," Applied and Environmental Microbiology 68(7):3597-3605. This percentage is similar to or the same as those given for *E. coli* by the references described above.

In embodiments, the amount of soluble polypeptide or protein of interest produced is about 0.1% to about 95% of the total soluble protein produced in a culture. In embodiments, this amount is more than about 0.1%, 0.5%, 1%, 5%, 10%, 15%, 20%, 25%, 30%, 40%, 45%, 50%, 55%, 60%, 65%. 70%, 75%, 80%, 85%, 90%, or 95% of the total soluble protein produced in a culture. In embodiments, this amount is about 0.1%, 0.5%, 1%, 5%, 10%, 15%, 20%, 25%, 30%, 40%, 45%, 50%, 55%, 60%, 65%, 70%, 75%, 80%, 85%, 90%, or 95% of the total soluble protein produced in a culture. In embodiments, this amount is about 5% to about 95%, about 10% to about 85%, about 20% to about 75%, about 30% to about 65%, about 40% to about 55%, about 1% to about 95%, about 5% to about 30%, about 1% to about 10%, about 10% to about 20%, about 20% to about 30%, about 30% to about 40%, about 40% to about 50%, about 50 to about 60%, about 60% to about 70%, or about 80% to about 90% of the total soluble protein produced in a culture.

In embodiments, the amount of soluble polypeptide or protein of interest produced is about 0.1% to about 50% of the dry cell weight (DCW). In embodiments, this amount is more than about 0.1%, 0.5%, 1%, 5%, 10%, 15%, 20%, 25%, 30%, 40%, 45%, or 50% of DCW. In embodiments, this amount is about 0.1%, 0.5%, 1%, 5%, 10%, 15%, 20%, 25%, 30%, 40%, 45%, or 50% of DCW. In embodiments, this amount is about 5% to about 50%, about 10% to about 40%, about 20% to about 30%, about 1% to about 20%, about 5% to about 25%, about 1% to about 10%, about 10% to about 20%, about 20% to about 30%, about 30% to about 40%, or about 40% to about 50% of the total soluble protein produced in a culture.

In embodiments, the amount of a soluble polypeptide or protein of interest heterologous to the secretion leader produced using the methods of the invention is greater than the amount of the protein native to the secretion leader, when produced under substantially similar conditions, e.g., growth conditions, including host cell. In embodiments, a soluble heterologous polypeptide or protein of interest operably linked to the AnsB, 8484, or 5193 secretion leader according to the present methods, is produced in an amount greater than the amount of the protein native to the respective leader. In embodiments, the soluble heterologous polypeptide or protein of interest operably linked to the AnsB, 8484, or 5193 secretion leader is produced at a yield that is about 1.2-fold to about 5-fold greater, about 1.2-fold to about 2.5-fold, about 1.2-fold to about 2-fold, about 1.2-fold to about 1.5-fold, about 1.4-fold to about 3-fold greater, about 1.4-fold to about 2.5-fold, about 1.4-fold to about 2-fold, about 1.5-fold to about 3-fold, about 1.5-fold to about 2.5-fold, about 1.5-fold to about 2-fold, about 2-fold to about 3-fold, about 1.2-fold, about 1.3-fold, about 1.4-fold, about 1.5-fold, about 1.6-fold, about 1.7-fold, about 1.8-fold, about 1.9-fold, about 2-fold, about 2.5-fold, about 3-fold, about 4-fold, or about 5-fold greater, than the amount of the protein native to the leader produced under substantially similar conditions.

In embodiments, the amount of a soluble polypeptide or protein of interest operably linked to the secretion leader produced using the methods of the invention is greater than the amount of the polypeptide or protein of interest produced without a secretion leader or with a different secretion leader, when produced under substantially similar conditions, e.g., growth conditions. Growth conditions can include, e.g., the culture medium and host cell used. In embodiments, a soluble heterologous polypeptide or protein of interest operably linked to the AnsB, 8484, or 5193 secretion leader according to the present methods, is produced in an amount greater than the amount of the protein of interest produced without a secretion leader or with a different secretion leader. In embodiments, the soluble heterologous polypeptide or protein of interest operably linked to the AnsB, 8484, or 5193 secretion leader is produced at a yield that is about 1.2-fold to about 20-fold greater, about 1.2-fold to about 2.5-fold, about 1.2-fold to about 2-fold, about 1.2-fold to about 1.5-fold, about 1.4-fold to about 3-fold greater, about 1.4-fold to about 2.5-fold, about 1.4-fold to about 2-fold, about 1.5-fold to about 3-fold, about 1.5-fold to about 2.5-fold, about 1.5-fold to about 2-fold, about 2-fold to about 5-fold, about 2-fold to about 10-fold, about 5-fold to about 15-fold, about 10-fold to about 20-fold, about 1.2-fold, about 1.3-fold, about 1.4-fold, about 1.5-fold, about 1.6-fold, about 1.7-fold, about 1.8-fold, about 1.9-fold, about 2-fold, about 2.5-fold, about 3-fold, about 4-fold, about 5-fold greater, about 10-fold greater, about 15-fold greater, or about 20-fold greater than the amount of the protein produced without a secretion leader or with a different secretion leader under substantially similar conditions.

*Solubility and Activity*

The "solubility" and "activity" of a protein, though related qualities, are generally determined by different means. Solubility of a protein, particularly a hydrophobic protein, indicates that hydrophobic amino acid residues are improperly located on the outside of the folded protein. Protein activity, which is often evaluated using different methods, e.g., as described below, is another indicator of proper protein conformation. "Soluble, active, or both" as used herein, refers to protein that is determined to be soluble, active, or both soluble and active, by methods known to those of skill in the art.

*Activity assay*

Assays for evaluating the activity of peptides, polypeptides or proteins of interest are known in the art and include but are not limited to fluorometric, colorometric, chemiluminescent, spectrophotometric, and other enzyme assays available to one of skill in the art. These assays are used to compare activity of a preparation of a peptide, polypeptide or protein of interest to a commercial or other preparation of a peptide, polypeptide or protein.

In embodiments, activity is represented by the percent active protein in the extract supernatant as compared with the total amount assayed. This is based on the amount of protein determined to be active by the assay relative to the total amount of protein used in assay. In other embodiments, activity is represented by the % activity level of the protein compared to a standard, e.g., native protein. This is based on the amount of active protein in supernatant extract sample relative to the amount of active protein in a standard sample (where the same amount of protein from each sample is used in assay).

**In** embodiments, about 40% to about 100% of the peptide, polypeptide or protein of interest, is determined to be active. In embodiments, about 40%, about 50%, about 60%, about 70%, about 80%, about 90%, or about 100% of the peptide, polypeptide or protein of interest is determined to be active. In embodiments, about 40% to about 50%, about 50% to about 60%, about 60% to about 70%, about 70% to about 80%, about 80% to about 90%, about 90% to about 100%, about 50% to about 100%, about 60% to about 100%, about 70% to about 100%, about 80% to about 100%, about 40% to about 90%, about 40% to about 95%, about 50% to about 90%, about 50% to about 95%, about 50% to about 100%, about 60% to about 90%, about 60% to about 95%, about 60% to about 100%, about 70% to about 90%, about 70% to about 95%, about 70% to about 100%, or about 70% to about 100% of the peptide, polypeptide or protein of interest is determined to be active.

In other embodiments, about 75% to about 100% of the peptide, polypeptide or protein of interest is determined to be active. In embodiments, about 75% to about 80%, about 75% to about 85%, about 75% to about 90%, about 75% to about 95%, about 80% to about 85%, about 80% to about 90%, about 80% to about 95%, about 80% to about 100%, about 85% to about 90%, about 85% to about 95%, about 85% to about 100%, about 90% to about 95%, about 90% to about 100%, or about 95% to about 100% of the peptide, polypeptide or protein of interest is determined to be active.

Proteins of Interest

The methods and compositions herein are useful for producing high levels of properly processed recombinant protein or polypeptide of interest in a cell expression system. In embodiments, the protein or polypeptide of interest (also referred to herein as "target protein" or "target polypeptide") is of any species and of any size. In certain embodiments, the protein or polypeptide of interest is a therapeutically useful protein or polypeptide. In some embodiments, the protein is a mammalian protein, for example a human protein, for example, a growth factor, a cytokine, a chemokine, a growth factor, or a blood protein. In embodiments, the protein or polypeptide of interest is processed in a similar manner to the reference protein or polypeptide. In certain embodiments, the protein or polypeptide does not include a secretion signal in the coding sequence. In embodiments, the protein or polypeptide of interest is less than 100 kD, less than 50 kD, or less than 30 kD in size. In embodiments, the protein or polypeptide of interest is of at least about 5, 10, 15, 20, 30, 40, 50 or 100 amino acids.

Extensive sequence information required for molecular genetics and genetic engineering techniques is widely publicly available. Access to complete nucleotide sequences of mammalian, as well as human, genes, cDNA sequences, amino acid sequences and genomes are often obtained from GenBank at the website www.ncbi.nlm.nih.gov/Entrez. Additional information is also sometimes obtained from GeneCards, an electronic encyclopedia integrating information about genes and their products and biomedical applications from the Weizmann Institute of Science Genome and Bioinformatics, nucleotide sequence information is also sometimes obtained from the EMBL Nucleotide Sequence Database or the DNA Databank or Japan (DDBJ); additional sites for information on amino acid sequences include Georgetown's protein information resource website and Swiss-Prot.

Examples of proteins that are often expressed in compositions and methods herein include, but are not limited to, molecules such as, e.g., renin, a growth hormone, including human growth hormone; bovine growth hormone; growth hormone releasing factor; parathyroid hormone; thyroid stimulating hormone; lipoproteins; $\alpha$-1-antitrypsin; insulin A-chain; insulin B-chain; proinsulin; thrombopoietin; follicle stimulating hormone; calcitonin; luteinizing hormone; glucagon; clotting factors such as factor VIIIC, factor IX, tissue factor, and von Willebrands factor; anti-clotting factors such as Protein C; atrial naturietic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; a serum albumin such as human serum albumin; mullerian-inhibiting substance; relaxin A-chain; relaxin B-chain; prorelaxin; mouse gonadotropin-associated polypeptide; a microbial protein, such as beta-lactamase; Dnase; inhibin; activin; vascular endothelial growth factor (VEGF); receptors for hormones or growth factors; integrin; protein A or D; rheumatoid factors; a neurotrophic factor such as brain-derived neurotrophic factor (BDNF), neurotrophin-3, -4, -5, or -6 (NT-3, NT-4, NT-5, or NT-6), or a nerve growth factor such as NGF-$\beta$; cardiotrophins (cardiac hypertrophy factor) such as cardiotrophin-1 (CT-1); platelet-derived growth factor (PDGF); fibroblast growth factor such as aFGF and bFGF; epidermal growth factor (EGF); transforming growth factor (TGF) such as TGF-alpha and TGF-$\beta$, including TGF-$\beta$1, TGF-$\beta$2, TGF-$\beta$3, TGF-$\beta$4, or TGF-$\beta$5; insulin-like growth factor-I and -II (IGF-I and IGF-II); des(1-3)-IGF-I (brain IGF-I), insulin-like growth factor binding proteins; CD proteins such as CD-3, CD-4, CD-8, and CD-19; erythropoietin; osteoinductive factors; immunotoxins; a bone morphogenetic protein (BMP); an interferon such as interferon-alpha, -beta, and -gamma; colony stimulating factors (CSFs), e.g., M-CSF, GM-CSF, and G-CSF; interleukins (ILs), e.g., IL-1 to IL-10; anti-HER-2 antibody; superoxide dismutase; T-cell receptors; surface membrane proteins; decay accelerating factor; viral antigen such as, for example, a portion of the AIDS envelope; transport proteins; homing receptors; addressins; regulatory proteins; antibodies; and fragments of any of the above-listed polypeptides.

In certain embodiments, the protein or polypeptide is selected from IL-1, IL-1a, IL-1b, IL-2, IL-3, IL-4, IL-5, IL-6, IL-7, IL-8, IL-9, IL-10, IL-11, IL-12, IL-12elasti, IL-13, IL-15, IL-16, IL-18, IL-18BPa, IL-23, IL-24, VIP, erythropoietin, GM-CSF, G-CSF, M-CSF, platelet derived growth factor (PDGF), MSF, FLT-3 ligand, EGF, fibroblast growth factor (FGF; e.g., $\alpha$-FGF (FGF-1), $\beta$-FGF (FGF-2), FGF-3, FGF-4, FGF-5, FGF-6, or FGF-7), insulin-like growth factors (e.g., IGF-1, IGF-2); tumor necrosis factors (e.g., TNF, Lymphotoxin), nerve growth factors (e.g., NGF), vascular endothelial growth factor (VEGF); interferons (e.g., IFN-$\alpha$, IFN-$\beta$, IFN-y); leukemia inhibitory factor (LIF); ciliary neurotrophic factor (CNTF); oncostatin M; stem cell factor (SCF); transforming growth factors (e.g., TGF-$\alpha$, TGF-$\beta$1, TGF-$\beta$2, TGF-$\beta$3); TNF superfamily (e.g., LIGHT/TNFSF14, STALL-1/TNFSF13B (BLy5, BAFF, THANK), TNFalpha/TNFSF2 and TWEAK/TNFSF12); or chemokines (BCA-1/BLC-1, BRAK/Kec, CXCL16, CXCR3, ENA-78/LIX, Eotaxin-1, Eotaxin-2/MPIF-2, Exodus-2/SLC, Fractalkine/Neurotactin, GROalpha/MGSA, HCC-1, I-TAC, Lymphotactin/ATAC/SCM, MCP-1/MCAF, MCP-3, MCP-4, MDC/STCP-1/ABCD-1, MIP-1.quadrature., MIP-1.quadrature., MIP-2.quadrature./GRO.quadrature., MIP-3.quadrature./Exodus/LARC, MIP-3/Exodus-3/ELC, MIP-4/PARC/DC-CK1, PF-4, RANTES, SDF1, TARC, TECK, or toxins (e.g, ω-agatoxin, μ-agatoxin, aitoxin, allopumilotoxin 267A, ω-atracotoxin-HV1, δ-Atracotoxin-Hv1b, Batrachotoxin, Botocetin, Arenobufagin, Bufotalin, Bufotenin · Cinobufagin, Marinobufagin, Bungarotoxin, Calcicludine, Calcisptine, Cardiotoxin III, Catrocollastatin C, Charybdotoxin, Cobra venom cytotoxin, Conotoxin, Echinoidin, Eledoisin, Epibatidine, Fibrolase, Hefutoxin, Histrionicotoxin, Huwentoxin I, Huwentoxin II, J-ACTX-Hv1c Kunitz Type Toxins, Dendrotoxin K, Dendrotoxin 1, Latrotoxin, Margatoxin, Maurotoxin, Onchidal, PhTx3, Pumilotoxin 251D, Rattlesnake lectin, Robustoxin, Saxitoxin, Scyllatoxin, Slotoxin, Stromatoxin, Taicatoxin, Tarichatoxin, Tetrodotoxin, Ricin, Gelonin, Aflatoxin, Amatoxin, Citrinin, Cytochalasin, Ergotamine, Fumagillin, Fumonisin, Gliotoxin, Helvolic Acid, Ibotenic Acid, Muscimol, Ochratoxin, Patulin, Sterigmatocytstin, Trichothecene, Vomitoxin, Zeranol, Zearalenone, Anthrax Toxin, Adenylate Protective antigen cyclase, rPA, Cry toxin, *Bordetella pertussis* toxins, *Clostridium botulinum* toxins, *Clostridium difficile* toxins, *Clostridium perfringens* toxins, Tetanus toxin, Diptheria toxins, Verotoxin/Shiga-like toxin, Heat stable enterotoxin, Heat labile enterotoxin, Enterotoxins, Listeriolysin O, *Mycobacterium tuberculosis* cord factor, *Pseudomonas* exotoxin, *Salmonella* endotoxin, *Salmonella* exotoxin, Shiga toxin, *Staphylococcus aureus* alpha toxin, *Staphylococcus aureus* beta toxin, *Staphylococcus aureus* delta toxin, Exfoliatin toxin, Toxic shock syndrome toxin, Enterotoxin, Leukocidin, Streptolysin S, or Cholera toxin).

In one embodiment herein, the protein of interest is a multi-subunit protein or polypeptide. Multisubunit proteins that are expressed include homomeric and heteromeric proteins. The multisubunit proteins may include two or more subunits, that may be the same or different. For example, the protein may be a homomeric protein comprising 2, 3, 4, 5, 6, 7, 8, 9, 10, 11, 12 or more subunits. The protein also may be a heteromeric protein including 2, 3, 4, 5, 6, 7, 8, 9, 10, 11, 12, or more subunits. Exemplary multisubunit proteins include: receptors including ion channel receptors; extracellular matrix proteins including chondroitin; collagen; immunomodulators including MHC proteins, full chain antibodies, antibody derivatives, and antibody fragments; enzymes including RNA polymerases, and DNA polymerases; and membrane proteins.

In another embodiment, the protein of interest is sometimes a blood protein. The blood proteins expressed in this embodiment include but are not limited to carrier proteins, such as albumin, including human and bovine albumin, transferrin, recombinant transferrin half-molecules, haptoglobin, fibrinogen and other coagulation factors, complement components, immunoglobulins, enzyme inhibitors, precursors of substances such as angiotensin and bradykinin, insulin, endothelin, and globulin, including alpha, beta, and gamma-globulin, and other types of proteins, polypeptides, and fragments thereof found primarily in the blood of mammals. The amino acid sequences for numerous blood proteins have been reported (see, S. S. Baldwin (1993) Comp. Biochem Physiol. 106b:203-218), including the amino acid sequence for human serum albumin (Lawn, L. M., et al., 1981, Nucleic Acids Research, 6103-6114) and human serum transferrin (Yang, F. et al., 1984, Proc. Natl. Acad. Sci. USA, 2752-2756).

In another embodiment, the protein of interest is sometimes a recombinant enzyme or co-factor. The enzymes and co-factors expressed in this embodiment include but are not limited to aldolases, amine oxidases, amino acid oxidases, aspartases, B12 dependent enzymes, carboxypeptidases, carboxyesterases, carboxylyases, chemotrypsin, CoA requiring enzymes, cyanohydrin synthetases, cystathione synthases, decarboxylases, dehydrogenases, alcohol dehydrogenases, dehydratases, diaphorases, dioxygenases, enoate reductases, epoxide hydrases, fumerases, galactose oxidases, glucose isomerases, glucose oxidases, glycosyltrasferases, methyltransferases, nitrile hydrases, nucleoside phosphorylases, oxidoreductases, oxynitilases, peptidases, glycosyltrasferases, peroxidases, enzymes fused to a therapeutically active polypeptide, tissue plasminogen activator; urokinase, reptilase, streptokinase; catalase, superoxide dismutase; Dnase, amino acid hydrolases (e.g., asparaginase, amidohydrolases); carboxypeptidases; proteases, trypsin, pepsin, chymotrypsin, papain, bromelain, collagenase; neuramimidase; lactase, maltase, sucrase, and arabinofuranosidases.

In another embodiment, the protein of interest sometimes is an antibody or antibody derivative. In embodiments, the antibody or antibody derivative is a humanized antibody, modified antibody, single-domain antibody (e.g., a nanobody, shark single domain IgNAR or VNAR antibody, camelid single domain antibody), heterospecific antibody, trivalent antibody, bispecific antibody (e.g., a BiTE molecule), single-chain antibody, Fab, Fab fragment, linear antibody, diabody, full chain antibody or a fragment or portion thereof. A single-chain antibody often includes the antigen-binding regions of antibodies on a single stably-folded polypeptide chain. Fab fragments are often a piece of a particular antibody. The Fab fragment usually contains the antigen binding site. The Fab fragment often contains 2 chains: a light chain and a heavy chain fragment. These fragments are usually linked via a linker or a disulfide bond. Antibodies and antibody derivatives useful in the present methods are known in the art and described in the literature, e.g., in U.S. 9,580,719 at Table 9, referenced elsewhere herein and incorporated by reference.

The coding sequence for the protein or polypeptide of interest is often a native coding sequence for the target polypeptide, if available, but will more often be a coding sequence that has been selected, improved, or optimized for use in the selected expression host cell: for example, by synthesizing the gene to reflect the codon use bias of a *Pseudomonas* species such as *P. fluorescens* or other suitable organism. The gene(s) that result will have been constructed within or will be inserted into one or more vectors, which will then be transformed into the expression host cell. Nucleic acid or a polynucleotide said to be provided in an "expressible form" means nucleic acid or a polynucleotide that contains at least one gene that is expressed by the selected expression host cell.

In certain embodiments, the protein of interest is, or is substantially homologous to, a native protein, such as a native mammalian or human protein. In these embodiments, the protein is not found in a concatameric form, but is linked only to a secretion signal and optionally a tag sequence for purification and/or recognition.

In other embodiments, the protein of interest is a protein that is active at a temperature from about 20 to about 42 °C. In one embodiment, the protein is active at physiological temperatures and is inactivated when heated to high or extreme temperatures, such as temperatures over 65 °C.

In one embodiment, the protein of interest is a protein that is active at a temperature from about 20 to about 42 °C. and/or is inactivated when heated to high or extreme temperatures, such as temperatures over 65 °C; is, or is substantially homologous to, a native protein, such as a native mammalian or human protein and not expressed from nucleic acids in concatameric form; and the promoter is not a native promoter in *P. fluorescens* but is derived from another organism, such as *E. coli.*

In other embodiments, the protein when produced also includes an additional targeting sequence, for example a sequence that targets the protein to the extracellular medium. In one embodiment, the additional targeting sequence is operably linked to the carboxy-terminus of the protein. In another embodiment, the protein includes a secretion signal for an autotransporter, a two partner secretion system, a main terminal branch system or a fimbrial usher porin.

The following examples are offered by way of illustration and not by way of limitation.

## EXAMPLES

The following examples are given for the purpose of illustrating various embodiments of the disclosure and are not meant to limit the present disclosure in any fashion. The present examples, along with the methods described herein are presently representative embodiments, are exemplary, and are not intended as limitations on the scope. Changes therein and other uses which are encompassed within the spirit of the disclosure as defined by the scope of the claims will occur to those skilled in the art.

### Example 1: Protein Expression Analysis

The efficiency of each of the three *P. fluorescens* secretion leaders, AnsB, 8484, and 5193, to direct soluble expression of three different recombinant proteins was assessed at the 0.5 mL culture scale.

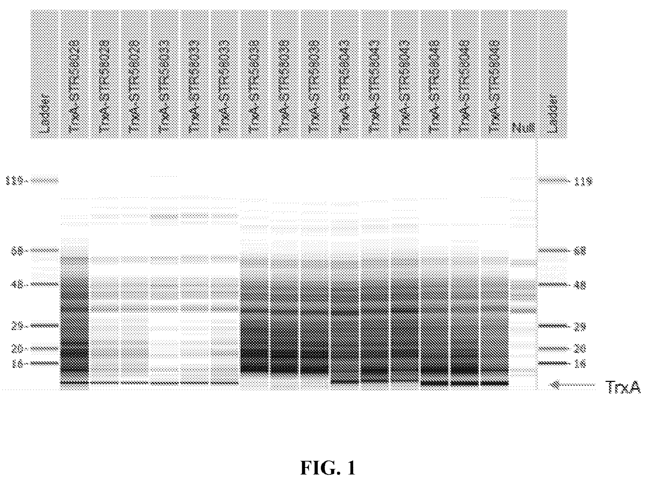

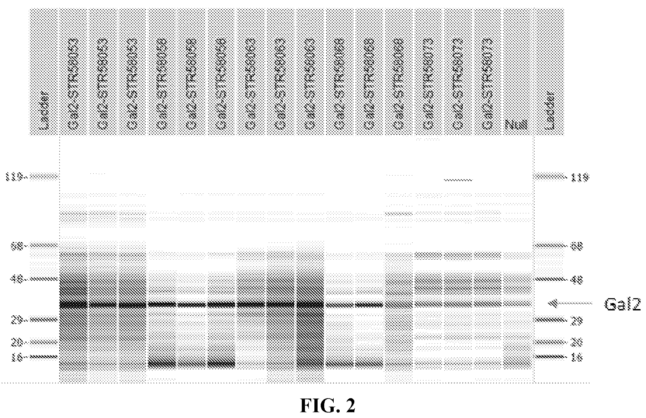

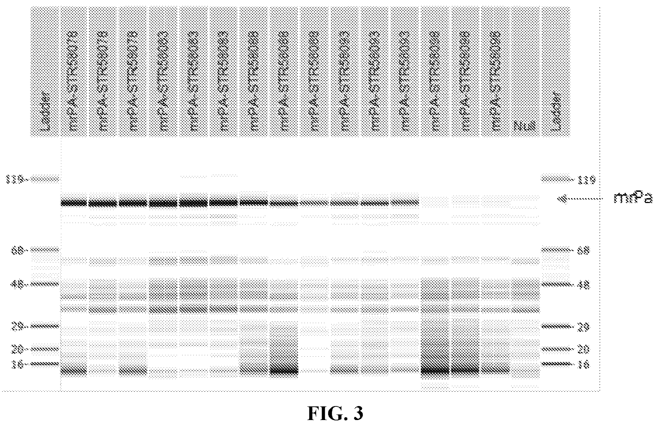

Each secretion leader coding sequence was fused in frame with genes encoding *E. coli* K-12 thioredoxin 1 (Trx-1 or TrxA, 11.9 kDa), the Gal2 single chain antibody (gal2 scFv, 29.6 kDa) and a *Bacillus anthracis* mutant recombinant protective antigen (mrPA, 83.7 kDa). Protein expression from these constructs was compared to expression from constructs having the Pbp secretion leader coding sequence fused to each of the same recombinant proteins, as well as to strains constructed for cytoplasmic expression of each protein. The cytoplasmic expression strain constructs encoded the initiator methionine followed by an alanine, fused to the TrxA, Gal2 or mrPA coding sequence. Plasmid constructs were transformed into the *P. fluarescens* strain DC454 and grown in selective medium (M9 glucose) to isolate positive clones. The results of this test are shown in **Table 6** below. Exemplary SDS-CGE images are shown in **FIG. 1** (TrxA), **FIG. 2** (Gal2 scFv), and **FIG. 3** (mrPA).

| Table 6: Protein Expression | | | |
|---|---|---|---|
| **Strain ID** | **Secretion Leader** | **Protein** | **Average Soluble Titer (mg/L)** |
| STR58028 | AnsB | TrxA | 98 |
| STR58033 | 8484 | TrxA | 96 |
| STR58038 | 5193 | TrxA | none detected |
| STR58043 | Pbp | TrxA | 112 |
| STR58048 | None (Met-Ala) | TrxA | 472 |
| STR58053 | AnsB | Gal2_ScFv | 234 |
| STR58058 | 8484 | Gal2_ScFv | 352 |
| STR58063 | 5193 | Gal2_ScFv | 296 |
| STR58068 | Pbp | Gal2_ScFv | 101 |
| STR58073 | None (Met-Ala) | Gal2_ScFv | 59 |
| STR58078 | AnsB | mrPA | 1131 |

(continued)

| Table 6: Protein Expression | | | |
|---|---|---|---|
| Strain ID | Secretion Leader | Protein | Average Soluble Titer (mg/L) |
| STR58083 | 8484 | mrPA | 1564 |
| STR58088 | 5193 | mrPA | 609 |
| STR58093 | Pbp | mrPA | 487 |
| STR58098 | None (Met-Ala) | mrPA | none detected |

Each strain was grown in triplicate for 24 hours in mineral salts medium, followed by induction with IPTG and an additional 24 hours of incubation. The broth was collected, diluted three-fold with PBS, sonicated and centrifuged. The supernatant was collected as the soluble protein fraction and analyzed by SDS-CGE (using the Caliper LabChip® GXII system). The induced bands were quantified in comparison to the system internal ladder. The Gal2 scFv and mrPA proteins were expressed at higher levels than TrxA when fused to the AnsB, 8484 and 5193 leaders, however, no expression of Gal2 or mrPA was detected when the proteins were targeted to the cytoplasm.

## Example 2: Crisantaspase Tier 1 Expression Plasmid Screening in 96 Well Format

In addition, the protein expression from *Erwinia* type II L-asparaginase (crisantaspase)-leader fusion constructs was evaluated.

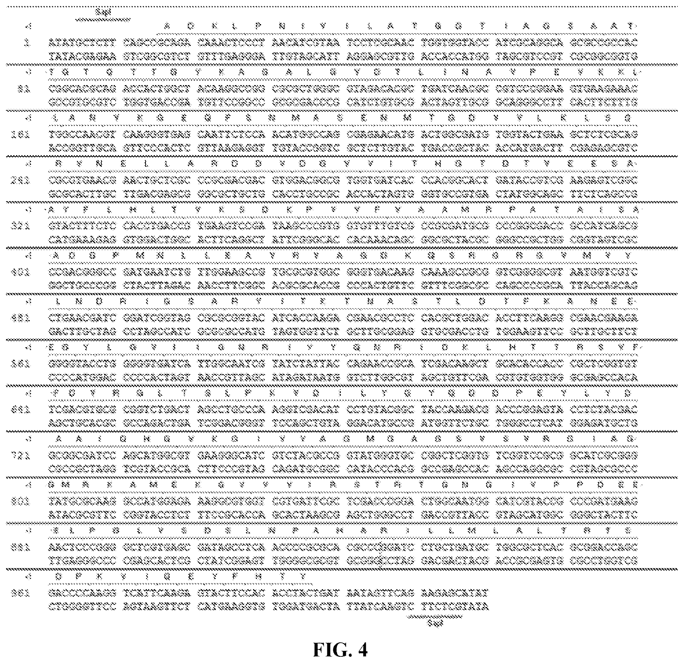

For the expression plasmid screening, an optimized crisantaspase protein coding sequence was designed and synthesized for expression in *P. fluorescens.* The DNA coding for the crisantaspase peptide sequence **(FIG. 4,** amino acid sequence set forth as SEQ ID NO: 7 and nucleic acid sequence set forth as SEQ ID NO: 8) was designed to reflect appropriate codon usage for *P. fluorescens.* A DNA region containing a unique restriction enzyme site (SapI or LguI) was added upstream of the crisantaspase coding sequence designed for direct fusion in frame with the secretion leader coding sequence present in the expression vector. A DNA region containing 3 stop codons and a unique restriction enzyme site (SapI) was added downstream of the coding sequence. Plasmids were constructed carrying the optimized crisantaspase gene fused to 37 different *P. fluorescens* secretion leaders and two ribosome binding-site (RBS) affinities **(Table 7).** An additional plasmid was constructed to express crisantaspase protein without a periplasmic leader in order to localize crisantaspase protein within the cell cytoplasm. Expression of the target was driven from the Ptac promoter, and translation initiated from a high activity ribosome binding site (RBS). The resulting 40 plasmids were transformed into two *P. fluorescens* host strains, DC454 (WT) and DC441 (PD), to produce 80 expression strains for the Tier 1 (expression strategy) screening. The ranking of the expression strategies was based on SDS-CGE estimated titers of crisantaspase monomer. The primary goal of the expression strategy screen was to evaluate a large subset of expression plasmids and their incorporated genetic elements, and to eliminate those expression strategies that yielded low or poor quality target expression.

The resulting cultures from the 80 transformations (40 expression strategies x 2 host strains) were grown in 96-well plates as described in Example 2. Sonicate fraction samples from whole broth culture harvested 24 hours after induction were analyzed by SDS-CGE. Expression of induced protein consistent with the expected molecular weight for crisantaspase monomer (35 kDa), which also co-migrated with *E. coli* L-Asp (Sigma Product #A3809), was quantified. SDS-CGE quantitation of reduced samples was completed by comparing the induced bands to an *E. coli* L-Asp standard curve. The 20 highest yielding samples from both the DC454 and DC441 host strains were ranked **(Table 9)** based on estimated soluble crisantaspase monomer titers. **FIG. 5** shows SDS-CGE gel-like figures generated from the analysis of the 96-well culture soluble sonicate samples. **Table 9** shows 24 hours post-induction (124) titers as estimated by SDS-CGE analysis of reduced soluble sonicates and quantified by Labchip® internal ladder for plasmids expressed in the DC454 (table on left) and DC441 (table on right) host strains. Also shown is the secretion leader fusion produced from each p743 expression plasmid.

The six plasmids and incorporated secretion leaders (p743-013 (FlgI), p743-038 (8484), p743-020 (LoIA), p743-018 (DsbC), p743-009 (Ibp-S31A) and p743-034 (5193)) observed in the top 10 highest yielding expression strains derived from both the DC454 and DC441 host strains are marked with ** in **Table 9.** Additionally, the p743-001 expression plasmid, designed for cytoplasmic expression of crisantaspase protein, ranked in the top two highest soluble yields for both hosts. From both host strains combined, the top ten highest soluble titers ranged from 525 to 1,523 $\mu$g/mL. Insoluble yield was low for all the expressions observed with the highest insoluble yield achieving 230 $\mu$g/mL using the p743-013 plasmid. Observation of the SDS-CGE banding patterns **(FIG. 5)** showed that the most complete secretion leader processing (removal upon export to the periplasm) occurred using the p743-013, p743-033 (leader O), p743-038, p743-009 and p743-018 expression plasmids while the p743-016 and p743-017 plasmids were observed to produce a prominent lower molecular weight truncation product. The 10 expression plasmids shown in **Table 8,** were chosen for the subsequent host strain screening at 96-well HTP scale based on SDS-CGE estimated high soluble titer. The ten selected expression strategies were then combined with 24 unique host strains which could further influence crisantaspase protein titer and quality.

| Table 7: Expression Plasmids | | | |
|---|---|---|---|
| **Plasmid ID** | **RBS Strength** | **Secretion Leader** | **RpC Vector** |
| p743-001 | High | None: (Met-Ala) | pDOW5277 |
| p743-002 | High | DsbD | pDOW3949 |
| p743-003 | High | Leader A | pFNX3952 |
| p743-004 | High | DsbA | pDOW5206 |
| p743-005 | Med | DsbA | pDOW5207 |
| p743-006 | High | Azu | pDOW5209 |
| p743-007 | Med | Azu | pDOW5210 |
| p743-008 | High | Lao | pDOW5217 |
| p743-009 | High | Ibp-S31A | pDOW5220 |
| p743-010 | High | TolB | pDOW5223 |
| p743-011 | High | Tpr | pDOW5226 |
| p743-012 | High | Ttg2C | pDOW5232 |
| p743-013 | High | FlgI | pDOW5235 |
| p743-014 | High | CupC2 | pDOW5238 |
| p743-015 | High | CupB2 | pDOW5241 |
| p743-016 | High | Pbp | pDOW5201 |
| p743-017 | High | PbpA20V | pDOW5259 |
| p743-018 | High | DsbC | pDOW5262 |
| p743-019 | High | Leader B | pFNX3941 |
| p743-020 | High | Leader C | pFNX3942 |
| p743-021 | High | Leader D | pFNX3943 |
| p743-022 | High | Leader E | pFNX3944 |
| p743-023 | High | Leader F | pFNX3947 |
| p743-024 | High | Leader G | pFNX3948 |
| p743-025 | High | Leader H | pFNX3950 |
| p743-026 | High | PorE | pDOW5256 |
| p743-027 | High | Leader I | pFNX3959 |
| p743-028 | High | Leader J | pFNX3957 |
| p743-029 | High | Leader K | pFNX3958 |
| p743-030 | High | Leader L | pFNX4202 |
| p743-031 | High | Leader M | pFNX4203 |
| p743-032 | High | Leader N | pFNX4204 |
| p743-033 | High | Leader O | pFNX4205 |
| p743-034 | High | 5193 | pFNX4206 |
| p743-035 | High | Leader P | pFNX4207 |

(continued)

| Table 7: Expression Plasmids | | | |
|---|---|---|---|
| Plasmid ID | RBS Strength | Secretion Leader | RpC Vector |
| p743-036 | High | Leader Q | pFNX4208 |
| p743-037 | High | Leader R | pFNX4209 |
| p743-038 | High | 8484 | pFNX4210 |
| p743-039 | High | Leader S | pFNX4211 |
| p743-040 | High | Leader T | pFNX4212 |
| p743-041 | High | AnsB | pFNX3968 |
| p743-042 | High | None | pDOW5271 |

| Table 8: Expression Plasmids Selected for Strain Screening | | |
|---|---|---|
| Plasmid ID | RBS Strength | Secretion Leader |
| p743-042 | High | None |
| p743-009 | High | Ibp-S31A |
| p743-017 | High | Pbp-A20V |
| p743-013 | High | FlgI |
| p743-018 | High | DsbC |
| p743-020 | High | Leader C |
| p743-033 | High | Leader O |
| p743-034 | High | 5193 |
| p743-038 | High | 8484 |
| p743-041 | High | AnsB |

**Table 9** shows 24 hours post-induction (I24) titers as estimated by SDS-CGE analysis of reduced soluble sonicates and quantified by Labchip® internal ladder for plasmids expressed in the DC454 (table on left) and DC441 (table on right) host strains. Also shown is the secretion leader fusion produced from each p743 expression plasmid.

| Table 9: Top 20 Expression Strains from Tier 1 Screening (**Six secretion leaders observed in the top 10 highest yielding plasmids from both host strains.) | | | | | | | |
|---|---|---|---|---|---|---|---|
| Strain ID (DC454 Host) | Plasmid | Secretion Leader | I24 Soluble (ug/mL) | Strain ID (DC441 Host) | Plasmid | Secretion Leader | I24 Soluble (ug/mL) |
| STR55337 | p743-001 | None | 1482 | STR55429 | p743-013 | FlgI** | 1523 |
| STR55349 | p743-013 | FlgI** | 1314 | STR55417 | p743-001 | None | 1369 |
| STR55369 | p743-033 | Leader O | 969 | STR55424 | p743-008 | Lao | 1329 |
| STR55374 | p743-038 | 8484** | 966 | STR55442 | p743-026 | PorE | 1136 |
| STR55356 | p743-020 | Leader C** | 852 | STR55434 | p743-018 | DsbC** | 946 |
| STR55354 | p743-018 | DsbC** | 832 | STR55436 | p743-020 | Leader C** | 727 |
| STR55345 | p743-009 | Ibp-S31A** | 810 | STR55454 | p743-038 | 8484** | 639 |
| STR55370 | p743-034 | 5193** | 674 | STR55425 | p743-009 | Ibp-S31A** | 600 |
| STR55347 | p743-011 | Tpr | 657 | STR55450 | p743-034 | 5193** | 532 |
| STR55348 | p743-012 | Ttg2C | 567 | STR55432 | p743-016 | Pbp | 525 |

(continued)

| Table 9: Top 20 Expression Strains from Tier 1 Screening (**Six secretion leaders observed in the top 10 highest yielding plasmids from both host strains.) | | | | | | | | |
|---|---|---|---|---|---|---|---|---|
| Strain ID (DC454 Host) | Plasmid | Secretion Leader | I24 Soluble (ug/mL) | | Strain ID (DC441 Host) | Plasmid | Secretion Leader | I24 Soluble (ug/mL) |
| STR55371 | p743-035 | Leader P | 439 | | STR55427 | p743-011 | Tpr | 492 |
| STR55351 | p743-015 | CupB2 | 433 | | STR55449 | p743-033 | Leader O | 477 |
| STR55346 | p743-010 | TolB | 431 | | STR55428 | p743-012 | Ttg2C | 384 |
| STR55360 | p743-024 | Leader G | 279 | | STR55426 | p743-010 | TolB | 363 |
| STR55359 | p743-023 | Leader F | 273 | | STR55435 | p743-019 | Leader B | 342 |
| STR55340 | p743-004 | DsbA | 270 | | STR55431 | p743-015 | CupB2 | 248 |
| STR55355 | p743-019 | Leader B | 249 | | STR55433 | p743-017 | PbpA20V | 225 |
| STR55353 | p743-017 | PbpA20V | 239 | | STR55451 | p743-035 | Leader P | 223 |
| STR55373 | p743-037 | Leader R | 223 | | STR55420 | p743-004 | DsbA | 222 |
| STR55350 | p743-014 | CupC2 | 220 | | STR55452 | p743-036 | Leader Q | 190 |

## Example 3: Preparation of *E. coli* Asparaginase Expression Constructs

Protein expression from *E. coli* asparaginase-leader fusion constructs was evaluated.

The *E. coli* A-1-3 L-asparaginase II gene was optimized for expression in *P. fluorescens* and cloned into a set of expression vectors for cytoplasmic and periplasmic expression. The amino acid sequence used is disclosed herein as SEQ ID NO: 9. The nucleic acid sequence used is disclosed herein as SEQ ID NO: 10.

Expression was evaluated using a series of the secretion leader sequences, some with a high RBS sequence and some with a medium RBS sequence. In addition, cytoplasmic expression was evaluated, using no leader.

Each construct was transformed into *P. fluorescens* host strains DC454 (*pyrF* deficient, no PD or FMO) and DC441 (*pyrF* deficient PD), and the resulting expression strains were evaluated for *E. coli* A-1-3 L-asparaginase II production in 0.5 mL cultures. The whole broth was sonicated, centrifuged, and the soluble fractions analyzed by SDS-CGE.

### Growth and Expression in 96 Well Format

For the expression plasmid screening, ligation mixtures for each of the *E. coli* A-1-3 L-asparaginase II expression plasmids were transformed into *P. fluorescens* host strains DC454 and DC441 cells as follows. Twenty-five microliters of competent cells were thawed and transferred into a 96-multiwell Nucleovette® plate (Lonza), and ligation mixture was added to each well. Cells were electroporated using the Nucleofector™ 96-well Shuttle™ system (Lonza AG). Cells were then transferred to 96-well deep well plates with 400 µl M9 salts 1% glucose medium and trace elements. The 96-well plates (seed plates) were incubated at 30 °C with shaking for 48 hours. Ten microliters of seed culture were transferred in duplicate into 96-well deep well plates, each well containing 500 µl of HTP medium, supplemented with trace elements and 5% glycerol, and incubated as before, for 24 hours. Isopropyl-β-D-1-thiogalactopyranoside (IPTG) was added at the 24-hour time point to each well for a final concentration of 0.3 mM, to induce the expression of target proteins. Mannitol (Sigma) was added to each well for a final concentration of 1% to induce the expression of folding modulators in folding modulator overexpressing strains. Cell density was measured by optical density at 600 nm (OD600) at 24 hours after induction to monitor growth. Twenty-four hours after induction, cells were harvested, diluted 1:3 in 1X PBS for a final volume of 400µl, then frozen. Samples were prepared and analyzed as described below.

The expression results for the top samples identified in the expression plasmid screening are shown in Tables 10 and 11.

| Table 10: Expression Plasmid Screening in DC454 | | |
|---|---|---|
| Sample_Name | Leader | Result (mg/L) |
| STR55304-2 | Lao | 2052 |

(continued)

| Table 10: Expression Plasmid Screening in DC454 | | |
|---|---|---|
| Sample_Name | Leader | Result (mg/L) |
| STR55312-2 | Pbp | 2016 |
| STR55305-2 | Ibp-S3 1A | 1413 |
| STR55334-2 | 8484 | 1322 |
| STR55333-2 | Leader R | 1073 |
| STR55313-2 | PbpA20V | 1068 |
| STR55302-2 | Azu | 884 |
| STR55317-2 | Leader D | 819 |
| STR55315-2 | Leader B | 764 |
| STR55310-2 | CupC2 | 751 |

| Table 11: Expression Plasmid Screening in DC441 Host | | |
|---|---|---|
| Sample_Name | Leader | Result-Bkg (10) ug/mL |
| STR55382-2 | Azu | 993 |
| STR55384-2 | Lao | 1422 |
| STR55385-2 | Ibp-S31A | 1048 |
| STR55392-2 | Pbp | 1305 |
| STR55393-2 | PbpA20V | 997 |
| STR55397-2 | Leader D | 698 |
| STR55413-2 | Leader R | 1005 |
| STR55414-2 | 8484 | 1199 |

For the host strain screening, expression plasmids selected based on the expression plasmid screening results each were transformed into each of 24 *P. fluorescens* host strains in an array, including the wild-type (WT) or parent DC454 strain, protease deletion (PD) strains, folding modulator overexpressing (FMO) strains and protease deletion plus folding modulator overexpressor (PD/FMO) strains. *E. coli* asparaginase fused to the *P. fluorescens* aparaginase secretion leader (AnsB) was included in the array (amino acid sequence set forth as SEQ ID NO: 1; coding sequence set forth as SEQ ID NO: 4). Folding modulators, when present, were encoded on a second plasmid and expression was driven by a *P. fluorescens*-native mannitol inducible promoter. The host strain screen transformations were performed as follows: twenty-five microliters of *P. fluorescens* host strain competent cells were thawed and transferred into a 96-multi-well Nucleovette® plate, and 10 μl plasmid DNA (10 ng) was added to each well. The cells were electroporated, cultured, induced in HTP format and harvested as described for the plasmid expression screening above. Samples were prepared and analyzed as described below.

Preparation of Samples for Analysis

Soluble fractions were prepared by sonication followed by centrifugation. Culture broth samples (400 μL) were sonicated with the Cell Lysis Automated Sonication System (CLASS, Scinomix) with a 24 probe tip horn under the following settings: 20 pulses per well at 10 seconds per pulse, and 60% power with 10 seconds between each pulse (Sonics Ultra-Cell). The lysates were centrifuged at 5,500 x g for 15 minutes (4 °C) and the supernatants collected (soluble fraction).

SDS-CGE Analysis

Protein samples were analyzed by microchip SDS capillary gel electrophoresis using a LabChip GXII instrument (PerkinElmer) with a HT Protein Express chip and corresponding reagents (PerkinElmer). Samples were prepared following the manufacturer's protocol (Protein User Guide Document No. 450589, Rev. 3). Briefly, in a 96-well polypropylene conical well PCR plate, 4 μL of sample were mixed with 14 μL of sample buffer, with 70 mM DTT reducing agent, heated at

Whole broth sampled 24 hours post induction was processed as described above and soluble fractions were analyzed by SDS-CGE.

A commercially available L-asparaginase activity assay kit (Sigma) detected significant L-asparaginase activity in HTP culture lysate samples from top yielding strain STR55382 (Lao leader) when compared to a Null sample.

The plasmids and corresponding secretion leaders screened in the expression plasmid screening experiment included:

p742-006 (Azu)
p742-008 (LAO)
p742-009 (Ibp-S31A)
p742-016 (Pbp)
p742-017 (PbpA20V)
p742-021 (Leader D)
p742-037 (Leader R)
p742-038 (8484)
p742-041 (*P. fluorescens* AnsB).

The expression strains were cultured and induced as described above. The SDS-CGE analysis of the soluble and insoluble fractions showed high level expression of asparaginase **(FIG. 6).** High titers were observed in the expression strains including those set forth in **Table 12.**

| Table 12: Host Strain Screening | | | | | |
|---|---|---|---|---|---|
| **Expression Strain ID** | **Plasmid (Leader)** | **Host Strain** | **Soluble I24 (ug/ml)** | **Insoluble I24 (ug/ml)** | **Whole Cell I24 (ug/ml)** |
| STR55467 | p742-041 (AnsB) | DC454 (pyrF, no PD, no FMO) | 3603 | 268 | 3871 |
| STR55689 | p742-009 (Ibp-S31A) | DC542 (pyrF proC, FMO lepB (RXF01181.1) | 2503 | 152 | 2655 |
| STR55559 | p742-009 (Ibp-S31A) | PF1201 (pyrF proC, deficient in proteases Lon (RXF04653.1), DegP1(RXF01250.2) DegP2 S219A (RXF07210.1 with S219A substitution), Prc1(RXF06586.1), and AprA (RXF04304.1)) | 2491 | 174 | 2665 |
| STR55561 | p742-016 (Pbp) | PF1201 | 2070 | 484 | 2554 |
| STR55555 | p742-038 (8484) | DC549 (pyrF proC, FMO tig-RXF04655) | 1772 | 94 | 1865 |

## Example 4: Construction of Strains

The following *P. fluorescens* asparaginase KO host strains were generated.

PF1433 (PyrF, AspG1, and AspG2 deficient), was constructed by sequential deletion of the aspG2 and aspG1 genes in the host strain DC454 (PyrF deficient).

PF1434 (PyrF, ProC, AspG1, and AspG2 deficient), was constructed by sequential deletion of the *aspG1* and *aspG2* genes in the host strain DC455 (*pyrF proC*). Strain DC455 is the parent strain of both DC542 and DC549.

PF1442 (PyrF, ProC, AspG1, AspG2, Lon, DegP1, DegP2 S219A, Prc1, and AprA deficient), was constructed by sequential deletion of aspG2 and aspG1 in the host strain PF1201 (PyrF, ProC, proteases Lon, DegP1, DegP2 S219A, Prc1, and AprA deficient).

PF1443 (PyrF, ProC, AspG1, and AspG2 deficient; FMO LepB expressed from pDOW3700), was constructed by transformation of the LepB-encoding FMO plasmid pDOW3700 into PF1434.

PF1444 (PyrF, ProC, AspG1, and AspG2 deficient; FMO Tig expressed from pDOW3707), was constructed by transformation of the Tig-encoding FMO plasmid pDOW3703 into PF1434.

PF1445 (PyrF, ProC, AspG1, AspG2, Lon, DegP1, DegP2, S219A, Prc1, and AprA deficient; FMO DsbAC-Skp expressed from pFNX4142), was constructed by the transformation of PF1442 with the DsbAC-Skp-encoding plasmid pFNX4142.

Strains used are described in **Table 13.**

\* with regard to protease deficiency/deletion and folding modulator overexpression.

| Table 13: Host Backgrounds of Asparaginase Deficient Expression Strains | | | | |
|---|---|---|---|---|
| Expression Strain ID | Host Strain | Expression Plasmid | Background Phenotype* | Secretion Leader |
| STR57867 | PF1433 | p742-041 | Wild-type | AnsB |
| STR57864 | PF1445 | p742-009 | PD/FMO | Ibp-S31A |
| STR57865 | PF1445 | p742-016 | PD/FMO | Pbp |
| STR57866 | PF1445 | p742-041 | PD/FMO | AnsB |
| STR57860 | PF1443 | p742-041 | FMO | AnsB |
| STR57861 | PF1444 | p742-041 | FMO | AnsB |
| STR57862 | PF1443 | p742-009 | FMO | Ibp-S31A |
| STR57863 | PF1444 | p742-038 | FMO | 8484 |

## Example 5: 2 L Fermentation and Calculation of Soluble % TCP of Selected Expression Strains

Strains STR57863 and STR57860 described in Example 3 were scaled to 2 L fermentation and each screened under up to eight different fermentation conditions. The 2 L scale fermentations (approximately 1 L final fermentation volume) were generated by inoculating a shake flask containing 600 mL of a chemically defined medium supplemented with yeast extract and glycerol with a frozen culture stock of the selected strain. After 16 to 24 h incubation with shaking at 30 °C, equal portions of each shake flask culture were then aseptically transferred to each of the 8-unit multiplex fermentation system containing a chemically defined medium designed to support a high biomass. In the 2 L fermentors, cultures were operated under controlled conditions for pH, temperature, and dissolved oxygen in a glycerol fed-batch mode. The fed-batch high cell density fermentation process consisted of a growth phase followed by an induction phase, initiated by the addition of IPTG and 5 g/L mannitol once the culture reached the target biomass (wet cell weight). The conditions during the induction phase were varied according to the experimental design. The induction phase of the fermentation was allowed to proceed for approximately 24 hours. Analytical samples were withdrawn from the fermentor to determine cell density (optical density at 575 nm) and were then frozen for subsequent analyses to determine the level of target gene expression. At the final time point of 24 hours post-induction, the whole fermentation broth of each vessel was harvested by centrifugation at 15,900 x g for 60 to 90 minutes. The cell paste and supernatant were separated and the paste retained and frozen at -80 °C.

**Table 14** shows expression results with strains STR57863 and STR57860 under several fermentation conditions. As shown, several of the initial strain/fermentation condition combinations resulted in >30% TCP asparaginase expression. Total cell protein was calculated as follows:

0.55 DCW total cell protein x 500 $\mu$g/mL DCW at A550 = 275 $\mu$g total cell protein/ml (or mg/L) at A550 =1

$$\text{TCP at the final timepoint (I24)} = \text{OD575} * 275\text{mg/L TCP}$$

$$\text{Soluble \% TCP} = 100* (\text{soluble titer / TCP})$$

| Table 14: 2 L Fermentation Expression Results | | | | | | | | | | |
|---|---|---|---|---|---|---|---|---|---|---|
| Strain Name | Induction Setpoints | | | Final OD575 | TCP at I24 (mg/L) | Soluble Titer (mg/L) | Insol. Titer (mg/L) | Total Titer (mg/L) | % soluble Titer | % soluble TCP |
| | wcw g/g | pH | IPTG (mM) | | | | | | | |
| STR57863 | 0.4 | 6.5 | 0.2 | 197 | 54175 | 4652 | 745 | 5397 | 86.2 | 8.59 |
| | 0.4 | 7.2 | 0.08 | 218 | 59950 | 3879 | 756 | 4635 | 83.7 | 6.47 |
| | 0.2 | 7.2 | 0.2 | 217 | 59675 | 8518 | 1565 | 10083 | 84.5 | 14.27 |
| | 0.2 | 6.5 | 0.2 | 189 | 51975 | 24658 | 2329 | 26987 | 91.4 | 47.44 |
| | 0.2 | 6.5 | 0.08 | 221 | 60775 | 2863 | 837 | 3700 | 77.4 | 4.71 |
| | 0.4 | 7.2 | 0.2 | 221 | 60775 | 14245 | 1206 | 15451 | 92.2 | 23.44 |
| | 0.4 | 6.5 | 0.08 | 201 | 55275 | 8226 | 1137 | 9363 | 87.9 | 14.88 |
| STR57860 | 0.4 | 6.5 | 0.2 | 233 | 64075 | 18508 | 2166 | 20674 | 89.5 | 28.88 |
| | 0.4 | 7.2 | 0.08 | 218 | 59950 | 15241 | 1404 | 16645 | 91.6 | 25.42 |
| | 0.2 | 7.2 | 0.2 | 245 | 67375 | 21445 | 4700 | 26145 | 82.0 | 31.83 |
| | 0.2 | 6.5 | 0.08 | 168 | 46200 | 15478 | 3357 | 18835 | 82.2 | 33.50 |
| | 0.4 | 7.2 | 0.2 | 206 | 56650 | 34283 | 2226 | 36509 | 93.9 | 60.52 |
| | 0.2 | 7.2 | 0.08 | 228 | 62700 | 35301 | 2387 | 37688 | 93.7 | 56.30 |
| | 0.4 | 6.5 | 0.08 | 198 | 54450 | 30284 | 1903 | 32187 | 94.1 | 55.62 |

### Example 6: Asparaginase Shake Flask Expression

Shake flask expression (200 mL) was performed to evaluate protein production from crisantaspase, *E. coli* type II L-asparaginase, and *P. fluorescens* type II L-asparaginase expression strains. Asparaginase expression plasmids were transformed into asparaginase deficient host strain PF1433 to produce the expression strains, as shown in **Table 15.** Lysate generated from shake flask samples were used for initial activity analysis and confirmation of intact mass by LC-MS analysis.

| Table 15. Shake Flask Expression Results | | | | | |
|---|---|---|---|---|---|
| Strain | Plasmid | Avg Soluble Reduced (µg/ml) | %CV | Avg Insoluble Reduced (µg/ml) | %CV |
| STR55976 (*E. coli* A-1-3 asparaginase type II) | p742-041 (AnsB) | 1011 | 5 | 129 | 41 |
| STR55977 (*E. coli* A-1-3 asparaginase type II) | p742-009 (Ibp-S31A) | 1514 | 9 | 253 | 56 |
| STR55978 (Crisantaspase) | p743-042 (cytoplasmic; no leader) | 1464 | 7 | 181 | 30 |
| STR55979 (Crisantaspase) | p743-033 (Leader O) | 743 | 13 | 195 | 42 |
| STR55980 (Crisantaspase) | p743-083 (8484 leader) | 908 | 2 | 180 | 48 |
| STR55981 (*P. fluorescens* MB 101 native L-Asp2) | p744-001 (AnsB) | 677 | 12 | 200 | 49 |

**Table 15** shows the average estimated titers as determined by SDS-CGE analysis of reduced soluble and insoluble sonicate fractions (ten different repetitions) of the three crisantaspase expression strains constructed using the PF1433 host strain (native asparaginase deficient, wild-type strain) analyzed at 200 mL working volume shake flask scale. SDS-CGE titers were estimated based on comparison to an *E. coli* L-Asp (Sigma) standard curve. The SDS-CGE gel-like images taken from both the soluble and insoluble sonicate analysis of each strain are shown in **FIG. 7.** It is notable that the titer of *P. fluorescens* native L-Asp2 protein, produced from a construct comprising the AnsB leader (STR55981, the native secretion leader-native asparaginase protein fusion) was substantially lower than the titer of the *E. coli* asparaginase protein produced from a construct comprising the AnsB leader (STR55976), i.e., wherein the AnsB leader was fused to the heterologous asparaginase protein (677 μg/ml compared with 1011 μg/ml, about a 1.5-fold difference).

Included in the analysis were shake flask growth from two null strains: STR55982 and DC432. The DC432 strain harbors plasmid pDOW1169, which does not contain the crisantaspase coding region, in a wild-type *P. fluorescens* host strain. STR55982 harbors plasmid pDOW1169 in host strain PF1433 which contains chromosomal deletions of both the native asparaginase coding sequences. All three of the crisantaspase expression strains produced predominantly soluble crisantaspase protein expression with strain STR55978 achieving the highest soluble titers of up to 14 g/L. Furthermore, no growth penalty was observed as all three crisantaspase expression strains achieved a similar cell density (OD600 = 23.0, 27.0 and 27.8) at 24 hrs. post induction when compared to the STR55982 and DC432 null strains, which gave an OD600 of 21.7 and 23.7, respectively, at 24 hours post induction.

Soluble sonicate samples generated from each of the five shake flask expression strains were analyzed for asparaginase activity using a commercial kit purchased from Sigma (Asparaginase Activity Assay Kit) according to the manufacturer's instructions. This kit measures activity using a coupled enzyme reaction which produces a colorimetric end product proportional to the aspartate generated. *E. coli* asparaginase type II from Sigma (A3809) was spiked into STR55982 null lysate as a positive control (last row).

The activity results are shown in **Table 16.**

| Table 16. Asparaginase Activity Assay of Shake Flask Culture Sonicate Samples | | | | | | |
|---|---|---|---|---|---|---|
| **Sample Description** | **Plasmid ID** | **Sample ID** | **Asparaginase Titer (mg/ml)*** | **Sample Dilution Factor** | **Aspartate Generated (nmol)** | **Δ A570 (TF-T0) 20 min** |
| AnsB/*E. coli* Asp2 | p742-041 | STR55976 | 0.20 | 25,000 | 0.36 | 0.03 |
| Ibp-S31A/*E. coli* Asp2 | p742-009 | STR55977 | 0.30 | 25,000 | 0.74 | 0.04 |
| Cytoplasmically-expressed Crisantaspase | p743-042 | STR55978 | 0.29 | 25,000 | 0.89 | 0.04 |
| Leader O/Crisantaspase | p743-033 | STR55979 | 0.15 | 25,000 | 0.86 | 0.04 |
| 8484 leader/Crisantaspase | p743-038 | STR55980 | 0.18 | 25,000 | 0.88 | 0.04 |
| AnsB/*P. fluorescens* native L-Asp2 | p744-001 | STR55981 | 0.14 | 25,000 | 0.10 | 0.02 |
| L-Asp-null | empty plasmid | STR55982 | 0.00 | 25,000 | 0.00 | 0.00 |
| L-asp+null | empty plasmid | DC432 | 0.00 | 25,000 | 0.00 | 0.00 |
| Null spike to 250 μg/ml** | N/A | L-Asp2 Sigma | 0.25 | 25,000 | 0.53 | 0.03 |
| *Determined by SDS-CGE | | | | | | |
| **Sigma A3809 *E. coli* AspG2 spiked into STR55982 (AspG deficient Null) lysate | | | | | | |

While both of the null samples showed no measurable activity at the 1:25,000 dilution factor, soluble sonicate samples from strains STR55976, STR55977, STR55978, STR55979 and STR55980 diluted 1:25,000 showed activity comparable to similarly diluted STR55982 null strain sample spiked with 250 μg/mL *E. coli* L-asparaginase from Sigma (A3809). These initial activity results using a commercially available kit would seem to indicate that crisantaspase protein and *E. coli* asparaginase protein expressed in *P. fluorescens* can readily form active, tetrameric asparaginase enzyme within the generated sonicates.

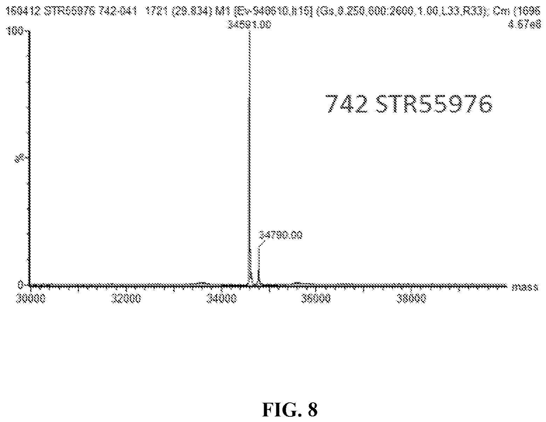

**Table 17** shows the LC-MS intact mass results from the analysis of crisantaspase protein from soluble sonicates produced by strains STR55978, STR55979 and STR55980 in shake flasks, and of *E. coli* asparaginase from soluble sonicates produced by strains STR55976 and STR55977. The observed molecular weight (35,053 Da) of the crisantaspase protein from each strain is consistent with the theoretical molecular weight (35054.2 Da) indicating that all three strains are generating the expected amino acid sequence and complete processing, or removal, of secretion leader if present. The observed molecular weight (34591) of *E. coli* asparaginase from each strain is consistent with the theoretical molecular weight (34591.96), indicating that both strains generate the expected amino acid sequence and that complete processing, or removal, of the secretion leader occurred. Sigma *E. coli* L-Asp was analyzed as a control. **FIG. 8** shows a mass spectrometry readout for STR55978, indicating proper cleavage of crisantaspase from the AnsB leader. The *E. coli* Asp2 expressed in STR55976 and STR55977 was shown by LC-MS to be properly cleaved from the AnsB and Ibp-S31A leaders, respectively.

| Table 17. LC-MS Analysis of Shake Flask Culture Sonicate Samples | | | |
|---|---|---|---|
| Sample Name | Theor. MW (Da) - signal | Observed MW (Da) | Obs. - Theor. MW (Da) |
| STR55976 AnsB/*E. coli* Asp2 | 34591.96 | 34591 | -0.96 |
| STR55977 Ibp-S31A/*E. coli* Asp2 | | 34591 | -0.96 |
| STR55978 Cytoplasmically-expressed Crisantaspase | 35054.2 | 35053 | -1.2 |
| STR55979 Leader O/Crisantaspase | | 35053 | -1.2 |
| STR55980 8484 leader/Crisantaspase | | 35053 | -1.2 |
| STR55981 AnsB/*P. fluorescens* native L-Asp2 | 36178.4 | 36177 | -1.4 |
| Sigma *E. coli* L-Asp A3809 | 34591.96 | 34591 | -0.96 |

| Table 18: Table of Sequences Listed | | |
|---|---|---|
| **Protein or Nucleic Acid** | **Sequence** | **SEQ ID NO:** |
| AnsB amino acid | MKSALKNVIPGALALLLLFPVAAQA | 1 |
| 8484 amino acid | MRQLFFCLMLMVSLTAHA | 2 |
| 5193 amino acid | MQSLPFSALRLLGVLAVMVCVLLTTPARA | 3 |
| AnsB nucleic acid | ATGAAATCTGCATTGAAGAACGTTATTCCGGGCGCCCTGGCCCTTCTGCTGC TATTCCCCGTCGCCGCCCAGGCC | 4 |
| 8484 nucleic acid | ATGCGACAACTATTTTTCTGTTTGATGCTGATGGTGTCGCTCACGGCGCACG CC | 5 |
| 5193 nucleic acid | ATGCAAAGCCTGCCGTTCTCTGCGTTACGCCTGCTCGGTGTGCTGGCAGTCA TGGTCTGCGTGCTGTTGACGACGCCAGCCCGTGCC | 6 |
| *Erwinia* Crisantaspase amino acid | ADKLPNIVILATGGTIAGSAATGTQTTGYKAGALGVDTLINAVPEVKKLANV KGEQFSNMASENMTGDVVLKLSQRVNELLARDDVDGVVITHGTDTVEESAYF LHLTVKSDKPVVFVAAMRPATAISADGPMNLLEAVRVAGDKQSRGRGVMVVL NDRIGSARYITKTNASTLDTFKANEEGYLGVIIGNRIYYQNRIDKLHTTRSV FDVRGLTSLPKVDILYGYQDDPEYLYDAAIQHGVKGIVYAGMGAGSVSVRGI AGMRKAMEKGVVVIRSTRTGNGIVPPDEELPGLVSDSLNPAHARILLMLALT RTSDPKVIQEYFHTY | 7 |
| A nucleic acid sequence optimized for *P. fluorescens,* encoding the *Erwinia* Crisantaspase of SEQ ID NO: 7 | GCAGACAAACTCCCTAACATCGTAATCCTCGCAACTGGTGGTACCATCGCAG GCAGCGCCGCCACCGGCACGCAGACCACTGGCTACAAGGCCGGCGCGCTGGG CGTAGACACGCTGATCAACGCCGTCCCGGAAGTGAAGAAACTGGCCAACGTC AAGGGTGAGCAATTCTCCAACATGGCCAGCGAGAACATGACTGGCGATGTGG TACTGAAGCTCTCGCAGCGCGTGAACGAACTGCTCGCCCGCGACGACGTGGA CGGCGTGGTGATCACCCACGGCACTGATACCGTCGAAGAGTCGGCGTACTTT CTCCACCTGACCGTGAAGTCCGATAAGCCCGTGGTGTTTGTCGCCGCGATGC GCCCGGCGACCGCCATCAGCGCCGACGGGCCGATGAATCTGTTGGAAGCCGT GCGCGTGGCGGGGTGACAAGCAAAGCCGCGGTCGGGGCGTAATGGTCGTCCTG | 8 |

(continued)

| Protein or Nucleic Acid | Sequence | SEQ ID NO: |
|---|---|---|
| **Table 18: Table of Sequences Listed** | | |
| | AACGATCGGATCGGTAGCGCGCGGTACATCACCAAGACGAACGCCTCCACGC TGGACACCTTCAAGGCGAACGAAGAGGGGTACCTGGGGGTGATCATTGGCAA TCGTATCTATTACCAGAACCGCATCGACAAGCTGCACACCACCCGCTCGGTG TTCGACGTGCGCGGTCTGACTAGCCTGCCCAAGGTCGACATCCTGTACGGCT ACCAAGACGACCCGGAGTACCTCTACGACGCGGCGATCCAGCATGGCGTGAA GGGCATCGTCTACGCCGGTATGGGTGCCGGCTCGGTGTCGGTCCGCGGCATC GCGGGTATGCGCAAGGCCATGGAGAAAGGCGTGGTCGTGATTCGCTCGACCC GGACTGGCAATGGCATCGTACCGCCCGATGAAGAACTCCCGGGGCTCGTGAG CGATAGCCTCAACCCCGCGCACGCCCGGATCCTGCTGATGCTGGCGCTCACG CGGACCAGCGACCCCAAGGTCATTCAAGAGTACTTCCACACCTAC | |
| Mature *E. coli* A-1-3 L-Asparaginase Type II amino acid sequence (without secretion leader sequence) | LPNITILATGGTIAGGGDSATKSNYTAGKVGVENLVNAVPQLKDIANVKGEQ VVNIGSQDMNDDVWLTLAKKINTDCDKTDGFVITHGTDTMEETAYFLDLTVK CDKPVVMVGAMRPSTSMSADGPFNLYNAVVTAADKASANRGVLVVMNDTVLD GRDVTKTNTTDVATFKSVNYGPLGYIHNGKIDYQRTPARKHTSDTPFDVSKL NELPKVGIVYNYANASDLPAKALVDAGYDGIVSAGVGNGNLYKTVFDTLATA AKNGTAVVRSSRVPTGATTQDAEVDDAKYGFVASGTLNPQKARVLLQLALTQ TKDPQQIQQIFNQY | 9 |
| Optimized nucleic acid sequence encoding mature *E. coli* A-1-3 L-Asparaginase Type II amino acid sequence of SEQ ID NO: 9 | CTCCCTAACATTACTATTCTGGCCACTGGCGGTACGATTGCAGGCGGCGGTG ACTCAGCCACCAAGTCGAATTACACCGCCGGTAAGGTCGGTGTCGAAAACCT CGTCAACGCCGTGCCGCAGCTGAAAGATATCGCCAACGTCAAGGGCGAGCAA GTGGTGAACATCGGCTCCCAAGATATGAACGATGACGTGTGGCTGACGCTGG CCAAGAAAATCAACACCGATTGCGACAAGACGGACGGGTTTGTCATCACCCA CGGCACCGACACTATGGAAGAGACTGCCTACTTCCTCGACCTCACGGTGAAG TGCGATAAACCGGTAGTGATGGTGGGCGCCATGCGCCCGAGCACCTCGATGA GCGCGGACGGCCCGTTCAATCTGTACAACGCCGTGGTAACCGCAGCGGACAA GGCGTCCGCGAACCGCGGTGTATTGGTAGTGATGAACGATACGGTGCTCGAT GGGCGCGATGTGACCAAGACCAATACCACTGATGTGGCCACCTTCAAGAGCG TGAACTATGGCCCGCTGGGCTACATCCATAACGGCAAGATCGATTACCAGCG TACTCCCGCCCGGAAGCACACCTCGGACACCCCCTTCGACGTGTCGAAACTG AACGAACTGCCCAAGGTCGGCATCGTCTACAACTACGCCAATGCGAGCGATC TGCCCGCGAAGGCCCTGGTGGACGCCGGCTACGACGGGATCGTATCGGCGGG TGTGGGCAATGGTAACCTGTACAAGACCGTGTTTGACACCCTGGCGACGGCG GCGAAGAACGGCACCGCCGTGGTCCGCAGCAGCCGCGTGCCCACTGGGGCGA CCACCCAAGACGCCGAGGTCGACGACGCGAAGTACGGCTTCGTAGCCAGCGG CACCCTGAACCCGCAAAAGGCCCGGGTCCTGCTGCAGCTGGCGCTCACGCAG ACGAAGGACCCGCAGCAAATCCAACAGATCTTCAACCAGTAC | 10 |
| Shine-Dalgarno RBS (consensus) | AGGAGG | 11 |
| RBS2 | GGAGCG | 12 |
| RBS34 | GGAGCG | 13 |
| RBS41 | AGGAGT | 14 |
| RBS43 | GGAGTG | 15 |
| RBS48 | GAGTAA | 16 |
| RBS1 | AGAGAG | 17 |

(continued)

| Table 18: Table of Sequences Listed | | |
|---|---|---|
| **Protein or Nucleic Acid** | **Sequence** | **SEQ ID NO:** |
| RBS35 | AAGGCA | 18 |
| RBS49 | CCGAAC | 19 |
| A nucleic acid sequence optimized for *P. fluorescens,* encoding the *Erwinia* Crisantaspase of SEQ ID NO: 7, including restriction sites as shown in Fig. 4 | ATATGCTCTTCAGCCGCAGACAAACTCCCTAACATCGTAATCCTCGCAACTG GTGGTACCATCGCAGGCAGCGCCGCCACCGGCACGCAGACCACTGGCTACAA GGCCGGCGCGCTGGGCGTAGACACGCTGATCAACGCCGTCCCGGAAGTGAAG AAACTGGCCAACGTCAAGGGTGAGCAATTCTCCAACATGGCCAGCGAGAACA TGACTGGCGATGTGGTACTGAAGCTCTCGCAGCGCGTGAACGAACTGCTCGC CCGCGACGACGTGGACGGCGTGGTGATCACCCACGGCACTGATACCGTCGAA GAGTCGGCGTACTTTCTCCACCTGACCGTGAAGTCCGATAAGCCCGTGGTGT TTGTCGCCGCGATGCGCCCGGCGACCGCCATCAGCGCCGACGGGCCGATGAA<br><br>TCTGTTGGAAGCCGTGCGCGTGGCGGGTGACAAGCAAAGCCGCGGTCGGGGC GTAATGGTCGTCCTGAACGATCGGATCGGTAGCGCGCGGTACATCACCAAGA CGAACGCCTCCACGCTGGACACCTTCAAGGCGAACGAAGAGGGGTACCTGGG GGTGATCATTGGCAATCGTATCTATTACCAGAACCGCATCGACAAGCTGCAC ACCACCCGCTCGGTGTTCGACGTGCGCGGTCTGACTAGCCTGCCCAAGGTCG ACATCCTGTACGGCTACCAAGACGACCCGGAGTACCTCTACGACGCGGCGAT CCAGCATGGCGTGAAGGGCATCGTCTACGCCGGTATGGGTGCCGGCTCGGTG TCGGTCCGCGGCATCGCGGGTATGCGCAAGGCCATGGAGAAAGGCGTGGTCG TGATTCGCTCGACCCGGACTGGCAATGGCATCGTACCGCCCGATGAAGAACT CCCGGGGCTCGTGAGCGATAGCCTCAACCCCGCGCACGCCCGGATCCTGCTG ATGCTGGCGCTCACGCGGACCAGCGACCCCAAGGTCATTCAAGAGTACTTCC ACACCTACTGATAATAGTTCAGAAGAGCATAT | 20 |

While preferred embodiments of the present disclosure have been shown and described herein, such embodiments are provided by way of example only. Numerous variations, changes, and substitutions will now occur to those skilled in the art without departing from the disclosure. It should be understood that various alternatives to the embodiments of the disclosure described herein may be employed in practicing the methods herein. It is intended that the following claims define the scope of the invention and that methods and structures within the scope of these claims and their equivalents be covered thereby.

The invention also refers to the following embodiments, wherein the term "claim" means "embodiment":

1. A polypeptide comprising a leader peptide operably linked to a protein or polypeptide of interest, wherein the leader peptide has an amino acid sequence as set forth in SEQ ID NO: 1, 2 or 3.

2. The polypeptide of claim 1, wherein the leader peptide is not native to the protein or polypeptide of interest.

3. The polypeptide of claim 1, wherein the protein or polypeptide of interest is selected from an antibody or antibody derivative, an enzyme, a cytokine, a chemokine, a growth factor, and a vaccine antigen.

4. The polypeptide of claim 2, wherein the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody.

5. The polypeptide of claim 1, further comprising a linker.

6. The polypeptide of claim 1, further comprising a cleavage domain.

7. The polypeptide of claim 1, wherein the first portion is a leader peptide that directs expression of the protein or polypeptide of interest to the periplasm of a prokaryotic host cell.

8. The polypeptide of claim 7, wherein the prokaryotic host cell is selected from a *Pseudomonad* cell or an *E. coli* cell.

9. A method of producing a protein or polypeptide of interest in a prokaryotic cell culture, the method comprising: (a) culturing prokaryotic cells in a cell culture growth medium, wherein the prokaryotic cells comprise a nucleic acid encoding the protein or polypeptide of interest operably linked to a leader peptide; and (b) isolating the protein or polypeptide of interest from the periplasm of the prokaryotic cells, wherein the leader peptide comprises an amino acid sequence selected from SEQ ID NOS: 1, 2 and 3.

10. The method of claim 9, wherein the protein or polypeptide of interest is selected from an antibody or antibody derivative, an enzyme, a cytokine, a chemokine, a growth factor, and a vaccine antigen.

11. The method of claim 10, wherein the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody.

12. The method of claim 9, wherein the nucleic acid encodes a linker.

13. The method of claim 9, wherein the nucleic acid encodes a cleavage domain.

14. The method of claim 9, wherein the prokaryotic cells are selected from a *Pseudomonad* cell or an *E. coli* cell.

15. A method of expressing a protein or polypeptide of interest in the periplasm of a prokaryotic cell, the method comprising culturing the prokaryotic cell comprising a nucleic acid encoding the protein or polypeptide of interest operably linked to a leader peptide in a cell culture growth medium, wherein the leader peptide comprises an amino acid sequence selected from SEQ ID NOS: 1, 2 and 3.

16. The method of claim 15, wherein the protein or polypeptide of interest is selected from an antibody or antibody derivative, an enzyme, a cytokine, a chemokine, a growth factor, and a vaccine antigen.

17. The method of claim 16, wherein the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody.

18. The method of claim 15, wherein the nucleic acid encodes a linker.

19. The method of claim 15, wherein the nucleic acid encodes a cleavage domain.

20. The method of claim 15, wherein the prokaryotic cells are selected from a *Pseudomonad* cell or an *E. coli* cell.

**Claims**

1. A polypeptide comprising a leader peptide operably linked to a protein or polypeptide of interest, wherein the leader peptide has an amino acid sequence as set forth in SEQ ID NO: 3 or 2.

2. The polypeptide of claim 1, wherein the leader peptide is not native to the protein or polypeptide of interest.

3. The polypeptide of claim 1, wherein the protein or polypeptide of interest is selected from an antibody or antibody derivative, an enzyme, a cytokine, a chemokine, a growth factor, and a vaccine antigen.

4. The polypeptide of claim 2, wherein the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody.

5. The polypeptide of claim 1, further comprising a linker.

6. The polypeptide of claim 1, further comprising a cleavage domain.

7. The polypeptide of claim 6, wherein the leader peptide directs expression of the protein or polypeptide of interest to the periplasm of a *Pseudomonad* cell or an *E. coli* cell.

8. A method of producing a protein or polypeptide of interest in a prokaryotic cell culture, the method comprising: (a) culturing prokaryotic cells in a cell culture growth medium, wherein the prokaryotic cells comprise a nucleic acid encoding the protein or polypeptide of interest operably linked to a leader peptide; and (b) isolating the protein or polypeptide of interest from the periplasm of the prokaryotic cells, wherein the leader peptide comprises an amino acid sequence as set forth in SEQ ID NO: 3 or 2.

9. A method of expressing a protein or polypeptide of interest in the periplasm of a prokaryotic cell, the method comprising culturing the prokaryotic cell comprising a nucleic acid encoding the protein or polypeptide of interest operably linked to a leader peptide in a cell culture growth medium, wherein the leader peptide comprises an amino acid sequence as set forth in SEQ ID NO: 3 or 2.

10. The method of claim 8 or claim 9, wherein the protein or polypeptide of interest is selected from an antibody or antibody derivative, an enzyme, a cytokine, a chemokine, a growth factor, and a vaccine antigen.

11. The method of claim 10, wherein the antibody or antibody derivative is an scFv, a Fab, a humanized antibody, a modified antibody, a single-domain antibody, a heterospecific antibody, a trivalent antibody, a bispecific antibody, a single-chain antibody, a Fab fragment, a linear antibody, a diabody, or a full chain antibody.

12. The method of claim 8 or claim 9, wherein the nucleic acid encodes a linker.

13. The method of claim 8 or claim 9, wherein the nucleic acid encodes a cleavage domain.

14. The method of claim 8 or claim 9, wherein the prokaryotic cells are selected from a *Pseudomonad* cell or an *E. coli* cell.

**FIG. 1**

**FIG. 2**

**FIG. 3**

FIG. 4

743 soluble sonicates, reduced SDS-CGE

**FIG. 5**

**FIG. 6**

**FIG. 7**

163412 STR55976 742-041 1721 (29.834) M1 [Ev-940610,It15] (Gs,0.250,600:2600,1.00,L33,R33); Cm (1695

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62578304
- US 7985564 B
- US 4695462 A
- US 20070292918
- US 7618799 B
- US 9394571 B
- US 9580719 B
- US 9453251 B
- US 8603824 B
- US 8530171 B
- US 4551433 A, DeBoer
- US 20080269070
- US 610207
- EP 0207459 A
- US 5055294 A, Gilroy
- US 5128130 A, Gilroy
- US 5281532 A, Rammler
- US 4695455 A
- US 4861595 A, Barnes
- US 4755465 A, Gray
- US 5169760 A, Wilcox
- US 20090325230
- US 20060040352
- WO 2008134461 A

**Non-patent literature cited in the description**

- **DERMAN et al.** *Science*, 1993, 1744-7
- Pathways of Disulfide Bond Formation in Proteins In Vivo. **BARDWELL et al.** Phosphate in microorganisms : cellular and molecular biology. 1994, 270-5
- **MANOIL**. *Methods in Enzymol.*, 2000, vol. 326, 35-47
- **AGARRABERES** ; **DICE**. *Biochim Biophys Acta*, 2001, vol. 1513, 1-24
- **MULLER et al.** *Prog Nucleic Acid Res Mol. Biol.*, 2001, vol. 66, 107-157
- **SAMBROOK J.** ; **RUSSELL, D.W.** Molecular Cloning: A Laboratory Manual. Cold Spring Harbor Laboratory Press, 2001
- **INNIS et al.** PCR Protocols: A Guide to Methods and Applications. Academic Press, 1990
- **W. R. PEARSON** ; **D. J. LIPMAN**. *Proc. Natl. Acad. Sci. USA*, April 1988, vol. 85, 2444-48
- **T. F. SMITH** ; **M. S. WATERMAN**. *Adv. Appl. Math.*, 1981, vol. 2, 482-89
- *J. Molec. Biol.*, 1981, vol. 147, 195-97
- **S. B. NEEDLEMAN** ; **C. D. WUNSCH**. *J. Molec. Biol.*, March 1970, vol. 48 (3), 443-53
- **W. R. PEARSON**. *Genomics*, November 1991, vol. 11 (3), 635-50
- **W. R. PEARSON**. *Methods Molec. Biol.*, vol. 24, 307-31
- **D. G. HIGGINS** ; **P. M. SHARP**. *Comp. Appl'ns in Biosci.*, 1989, vol. 5, 151-53
- *Gene*, 15 December 1988, vol. 73 (1), 237-44
- **SAMBROOK et al.** Molecular Cloning: A Laboratory Manual. Cold Spring Harbor Laboratory Press, 1989
- **MEINKOTH** ; **WAHL**. *Anal. Biochem.*, 1984, vol. 138, 267-284
- **TIJSSEN**. Laboratory Techniques in Biochemistry and Molecular Biology-Hybridization with Nucleic Acid Probes. Elsevier, 1993
- Current Protocols in Molecular Biology. Greene Publishing and Wiley-Interscience, 1995
- **WELCH et al.** Design Parameters to Control Synthetic Gene Expression in Escherichia coli. *PLoS One*, 2009, vol. 4 (9), e7002
- **KRISHNA R. et al.** Optimization of the AT-content of Codons Immediately Downstream of the Initiation Codon and Evaluation of Culture Conditions for High-level Expression of Recombinant Human G-CSF in Escherichia coli. *Mol Biotechnology*, 2008, vol. 38, 221-232
- **J. SANCHEZ-ROMERO** ; **V. DE LORENZO**. Manual of Industrial Microbiology and Biotechnology. ASM Press, 1999, 460-74
- **H. SCHWEIZER**. Current Opinion in Biotechnology. 2001, vol. 12, 439-445
- **R. SLATER** ; **R. WILLIAMS**. Molecular Biology and Biotechnology. The Royal Society of Chemistry, 2000, 125-54
- **L.-M. GUZMAN et al.** *J. Bacteriol.*, 1995, vol. 177 (14), 4121-4130
- **MCCARTHY et al.** *Trends in Genetics*, 1990, vol. 6, 78-85
- **SHINE** ; **DALGARNO**. *Proc. Natl. Acad. Sci. USA*, 1974, vol. 71, 1342-1346
- **D. FRISHMAN et al.** *Gene*, 08 July 1999, vol. 234 (2), 257-65

- **B. E. SUZEK et al.** *Bioinformatics*, December 2001, vol. 17 (12), 1123-30
- **O. IKEHATA et al.** *Eur. J. Biochem.*, 1989, vol. 181 (3), 563-70
- **SCHNEIDER et al.** Auxotrophic markers pyrF and proC, in some cases, replace antibiotic markers on protein production plasmids in high-cell-density Pseudomonas fluorescens fermentation. *Biotechnol. Progress*, 2005, vol. 21 (2), 343-8
- Bergey's Manual of Systematics of Archaea and Bacteria. 2015
- Gram-Negative Aerobic Rods and Cocci. Bergey's Manual of Systematics of Archaea and Bacteria. 2015
- UniProtKB, P0A850
- **B D DAVIS** ; **E S MINGIOLI**. *J. Bact.*, 1950, vol. 60, 17-28
- **RIESENBERG, D. et al.** High cell density cultivation of Escherichia coli at controlled specific growth rate. *J. Biotechnol.*, 1991, vol. 20 (1), 17-27
- **LIN, N.S.** ; **SWARTZ, J.R.** Production of Heterologous Proteins from Recombinant DNA Escherichia coli in Bench Fermentors. *METHODS: A Companion to Methods in Enzymology*, 1992, vol. 4, 159-168
- **DA SILVA, N.A. et al.** Theoretical Growth Yield Estimates for Recombinant Cells. *Biotechnology and Bioengineering*, 1986, vol. XXVIII, 741-746
- Escherichia coli and Salmonella typhimurium Cellular and Molecular Biology. 1987, vol. 1, 3-6
- **GLAZYRINA et al.** *Microbial Cell Factories*, 2010, vol. 9, 42
- **EDWARDS et al.** Continuous Culture of Pseudomonas fluorescens with Sodium Maleate as a Carbon Source. *Biotechnology and Bioengineering*, 1972, vol. XIV, 123-147
- **YARWOOD et al.** Noninvasive Quantitative Measurement of Bacterial Growth in Porous Media under Unsaturated-Flow Conditions. *Applied and Environmental Microbiology*, July 2002, vol. 68 (7), 3597-3605
- **S. S. BALDWIN**. *Comp. Biochem Physiol.*, 1993, vol. 106b, 203-218
- **LAWN, L. M. et al.** *Nucleic Acids Research*, 1981, 6103-6114
- **YANG, F. et al.** *Proc. Natl. Acad. Sci. USA*, 1984, 2752-2756